# EUROPEAN PATENT APPLICATION

(11) **EP 1 661 995 A1**
(43) Date of publication of application: **31.05.2006**
(21) Application number: 05027722.7
(22) Date of filing: 29.05.2001
(51) Int. Cl.: C12N 15/12, C07K 14/47, C12Q 1/68, G01N 33/574, C07K 16/18, A61K 51/00, A61K 39/395, A61K 38/17, A61P 35/00

(54) **Method of diagnosing, monitoring, staging, imaging and treating colon cancer**

(30) Priority: 26.05.2000 US 207383 P
(62) Divisional of application: 01939755.3
(71) Applicant: Diadexus, Inc., South San Francisco, CA 94080 (US)
(72) Inventor: The designation of the inventor has not yet been filed
(74) Representative: Peebles, Katrina

(57) **Abstract**

The invention relates to CSG polypeptides, polynucleotides encoding the polypeptides, methods for producing the polypeptides, in particular by expressing the polynucleotides, and agonists and antagonists of the polypeptides. The invention further relates to methods for utilizing such polynucleotides, polypeptides, agonists and antagonists for applications, which relate, in part, to research, diagnostic and clinical arts.

SEQUENCE LISTING

<110> Macina, Roberto A
      Chen, Sei-Yu
      Pluta, Jason
      Sun, Yongming
      Recipon, Herve
      diaDexus, Inc.

<120> Method of Diagnosing, Monitoring, Staging, Imaging and
      Treating Colon Cancer

<130> DEX-0208

<140>
<141>

<150> 60/207,383
<151> 2000-05-26

<160> 25

<170> PatentIn Ver. 2.1

<210> 1
<211> 911
<212> DNA
<213> Homo sapiens

<400> 1
tttttttttt ttgcctgttt gttcataatg tttactgtac aaagaaacaa aacccaggaa 60
tagtacaagt attgaacagt agcgagagtg gttgtgaaat aaaggaccac tttggaagac 120
agttttattg gcttgctgtc ttcaccaaga aagacttgtg atttttgaaa acttctacct 180
gaaatgtatt ttttctgctt tcccgaggaa gcggcactta cagtgttcct aggctttcct 240
gtgacgtggg tgccagtctg gattcaaaat atccttgcat gcactgcagc tccttaggga 300
gtcttttcct gcccttgagg cctgggcaga ctctcccctg acaccctccc gccctctccc 360
acgacgcagc agaaataaag cacaacctca gaaagtctca ggcacgaaga actgtcctcg 420
ggtggagcat gggaccttta ttcgttaaga catcaggctc cagatatgaa ctttcagcag 480
aagcgcttgc cgggagcaaa gggacagaaa agctgagatg aacagtgcct ggcagcaatc 540
acagccgggc aagggtgctc cgagcctcgc atcccccggc cgggggcagc tggaggtgcc 600
tcagaaggtg cattctgctt cctgcagggg cttgaaacac caaggcactc cagggatcct 660
ggagtcaaag cagcagcccc ggttgttgca ctccttgggg gtgacatggg ggtagccgca 720
gtccaccctg tccttggctg gcacggcaca ctggtttgca gctgtcccag acaaagccct 780
gtcagctgcc agagcccttg ctgggacagg cccacgtact tcctcagcag agctggagga 840
cagcaaggcc aggaccagcc ccagcatgca gagcgctctg gcagccatga ccaccgtggg 900
ctccgggacg c                                                      911

<210> 2
<211> 322
<212> DNA
<213> Homo sapiens

<220>
<221> unsure
<222> (244)

<400> 2
gacaagcaac aaacccttga tgattattca tcacttggat gagtgcccac acagtcaagc 60
tttaaagaaa gtgtttgctg aaaataaaga aatccagaaa ttggcagagc agtttgtcct 120
cctcaatctg gtttatgaaa caactgacaa acacctttct cctgatggcc agtatgtccc 180
caggattatg tttgttgacc catctctgac agttagagcc gatatcactg gaagatattc 240
aaancgtctc tatgcttacg aacctgcaga tacagctctg ttgcttgaca acatgaagaa 300
agctctcaag ttgctgaaga ct 322

<210> 3
<211> 4569
<212> DNA
<213> Homo sapiens

<400> 3
atggataaat tcctcaacac atacactctc ccaagactaa accaggaaga agttgaatct 60
ctgaatagac caataacagg ctctgatatt gtggcaataa tcaagagctt accaaccaaa 120
aagagtccag gaccagatgg attcacagct gaattctacc agaggtacaa ggaggaactg 180
gtaccattcc ctctgaaagt attacaatca atagaaaaag aggcaatcct ccctaactcg 240
ttttatgagg ccaacatcat cctgatacca aagccgggca gagacacaac caaaaaagag 300
aattttagac caatatcttt gatgaacatt gatgcaaaaa tcctcaataa aatactggca 360
aaccgaatcc agcagcacat caaaaagctt atccaccatg atcaagtggg cttcatccct 420
gggataacca aagacaaaaa ccacatgatt atctcaatag atgcagaaaa ggcctttgac 480
aaaattcaac aacccttcat gctaaaaacc ctcaataaat tagatattga tgggacatat 540
ctcaaaataa taagagctat ctatggcaaa gccacagcca atatcatact gaatgggcaa 600
aaactggaag cattcccttt gaaaactggc acaagacagg gatgccctct ctcaccactc 660
ctattcaaca tagttttgga agttctggcc agggcaatta ggcaggagaa ggaaataaag 720
ggttttcaat taggaaaaga ggaagtcaaa ttgtccctgt ttgcaggtga catgattgta 780
tacctagaaa accccattct ctcagcccaa aatctcctta agctgataag caacttcagc 840
aaagtctcag gatacaaaat caatgtacaa aaatcacaag cattcctata caccaataac 900
agagaaacag agagccaaat catgaatgaa ctcccattca caattgcttc aaagagaata 960
aaatacctag gaatccaact tacaagggat gtgaaggacc tcttcaagga gaactacaaa 1020
ccactgctca atgaaataaa agaggataca aacaaatgga agaacattcc atgctcatgg 1080
ataggaagaa tcaatatcgt gaaaatggcc atactgccca agattatgct agatataaag 1140
ggtattcaat taggaaaaga ggaagtcaaa ttgtccctgt ttgcagatga catgattgta 1200
tatctagaaa accccattgt ctcagcccaa aatctcctta agctgataag caacttcagc 1260
aaagtctcag gatacaaaat caatgtacaa aaatcacaag cattcttata caccaacaac 1320
agacaaacag agagccaaat catgagtgaa ctcccattca caattgcttc aaagagaata 1380
aaatacctag gaatccaact tacaagggac gtgaaggacc tcttcaagga gaactacaaa 1440

ccactgctca aggaaataaa agaggataca aacaaatgga agaacatttc atgctcatgg 1500
ataggaagaa tcaatatcgt gaaaatggcc atactgccca agagagaaat cacagggaga 1560
tgtacagcaa tggggccatt taagagttct gtgttcatct tgattcttca ccttctagaa 1620
ggggccctga gtaattcact cattcagctg aacaacaatg gctatgaagg cattgtcgtt 1680
gcaatcgacc ccaatgtgcc agaagatgaa acactcattc aacaaataaa gggggagtac 1740
acgtcacaag atgaggaagg gagagtcaga gagaaactct ctcttccccc gtcaaatata 1800
catacacaca caccacacgc acaagctcgt gtgcacacac acacgcccat gcacacacgc 1860
agacatacac gcacacacgc acgtcagaag gacatggtga cccaggcatc tctgtatctg 1920
cttgaagcta caggaaagcg attttatttc aaaaatgttg ccattttgat tcctgaaaca 1980
tggaagacaa aggctgacta tgtgagacca aaacttgaga cctacaaaaa tgctgatgtt 2040
ctggttgctg agtctactcc tccaggtaat gatgaaccct acactgagca gatgggcaac 2100
tgtggagaga agggtgaaag gatccacctc actcctgatt tcattgcagg aaaaaagtta 2160
gctgaatatg gaccacaagg tagggcattt gtccatgagt gggctcatct acgatgggga 2220
gtatttgacg agtacaataa tgatgagaaa ttctacttat ccaatggaag aatacaagca 2280
gtaagatgtt cagcaggtat tactggtaca aatgtagtaa agaagtgtca gggaggcagc 2340
tgttacacca aaagatgcac attcaataaa gtaacaggac tctatgaaaa aggatgtgag 2400
tttgttctcc aatcccgcca gacggagaag gcttctataa tgtttgcaca acatgttgat 2460
tctatagttg aattctgtac agaacaaaac cacaacaaag aagctccaaa caagcaaaat 2520
caaaaatgca atctccgaag cacatgggaa gtgatccgtg attctgagga ctttaagaaa 2580
accactccta tgacaacaca gccaccaaat cccaccttct cattgctgca gattggacaa 2640
agaattgtgt gtttagtcct tgacaaatct ggaagcatgg cgactggtaa ccgcctcaat 2700
cgactgaatc aagcaggcca gcttttcctg ctgcagacag ttgagctggg gtcctgggtt 2760
gggatggtga catttgacag tgctgcccat gtacaaaatg aactcataca gataaacagt 2820
ggcagtgaca gggacacact cgccaaaaga ttacctgcag cagcttcagg agggacgtcc 2880
atctgcagcg ggcttcgatc ggcatttact gatatgtggc aacatttgcc tgttttccat 2940
gacacacagc agttatgggg agtgcgacaa gaaaatccaa attgggcctc tctggcctgc 3000
agcttagtga ttaggaagaa atatccaact gatggatctg aaattgtgct gctgacggat 3060
ggggaagaca acactataag tgggtgcttt aacgaggtca aacaaagtgg tgccatcatc 3120
cacacagtcg ctttggggcc ctctgcagct caagaactag aggagctgtc caaaatgaca 3180
ggaggtttac agacatatgc ttcagatcaa gttcagaaca atggcctcat tgatgctttt 3240
ggggcccttt catcaggaaa tggagctgtc tctcagcgct ccatccagct tgagagtaag 3300
ggattaaccc tccagaacag ccagtggatg aatggcacag tgatcgtgga cagcaccgtg 3360
ggaaaggaca ctttgtttct tatcacctgg acaatgcagc ctccccaaat ccttctctgg 3420
gatcccagtg gacagaagca aggtggcttt gtagtggaca aaaacaccaa aatggcctac 3480
ctccaaatcc caggcattgc taaggttggc acttggaaat acagtctgca agcaagctca 3540
caaaccttga ccctgactgt cacgtcccgt gcgtccaatg ctaccctgcc tccaattaca 3600
gtgacttcca aaacgaacaa ggacaccagc aaattcccca gccctctggt agtttatgca 3660
aatattcgcc aaggagcctc cccaattctc agggccagtg tcacagccct gattgaatca 3720
gtgaatggaa aaacagttac cttggaacta ctggataatg gagcaggtgc tgatgctact 3780
aaggatgacg gtgtctactc aaggtatttc acaacttatg acacgaatgg tagatacagt 3840
gtaaaagtgc gggctctggg aggagttaac gcagccagac ggagagtgat accccagcag 3900
agtggagcac tgtacatacc tggctggatt gagaatgatg aaatacaatg gaatccacca 3960
agacctgaaa ttaataagga tgatgttcaa cacaagcaag tgtgtttcag cagaacatcc 4020
tcgggaggct catttgtggc ttctgatgtc ccaaatgctc ccatacctga tctcttccca 4080
cctggccaaa tcaccgacct gaaggcggaa attcacgggg gcagtctcat taatctgact 4140
tggacagctc ctggggatga ttatgaccat ggaacagctc acaagtatat cattcgaata 4200
agtacaagta ttcttgatct cagagacaag ttcaatgaat ctcttcaagt gaatactact 4260
gctctcatcc caaaggaagc caactctgag gaagtctttt tgtttaaacc agaaaacatt 4320

acttttgaaa atggcacaga tcttttcatt gctattcagg ctgttgataa ggtcgatctg 4380
aaatcagaaa tatccaacat tgcacgagta tctttgttta ttcctccaca gactccgcca 4440
gagacaccta gtcctgatga aacgtctgct ccttgtccta atattcatat caacagcacc 4500
attcctggca ttcacatttt aaaaattatg tggaagtgga taggagaact gcagctgtca 4560
atagcctag 4569

<210> 4
<211> 3206
<212> DNA
<213> Homo sapiens

<400> 4
ttcggctcga gtgtaaaact gccaaggaaa gtaattacct gtaggagttt gctgagcttg 60
aagagtgaaa actgttgtga atgagcctga tcataaaacg gaccaggcca ttcattattc 120
ctcaagtgtt aatatactga cttatgcagt attcaaacaa aaacattgca ctagatggtg 180
caagaacagc gtaaaatgaa agccatcatt catcttactc ttcttgcgtc tcctttctgt 240
aaacacagcc accaaccaag gcaactcagc tgatgctgta acaaccacag aaactgcgac 300
tagtggtcct acagtagctg cagctgatac cactgaaact aatttgccct gaaactgcta 360
gcaccacagc aaatacacct tctttcccaa cagctacttc acctgctccc cccataatta 420
gtacacatag ttcctccaca attcctacac ctgctccccc cataattagt acacatagtt 480
cctccacaat tcctatacct actgctgcag acagtgagtc aaccacaaat gtaaattcag 540
ttagctacct ctgacataat caccgcttca tctccaaatg atggattaat tcacaatggt 600
tccttctgaa acacaaagta acaatgaaat gtcccccacc acagaagaca atcaatcctc 660
agtggcctcc cactgggcac cgctttattt ggatgaccat gcacgcctaa acagcacagt 720
gtcccagcaa tccttgccaa agatgatccc cctgtgcaga taattcgtta ttgtttgtta 780
agcttgctat aatacaagtt tttgcctgtg tttagaaggg tattactaca actcttctac 840
atgtaagaaa ggaaaggtat tccctggaga agatttcagt gacagtatca gaaacatttg 900
acccagaaga gaaacattcc atggcctatc aagacttgca tagtgaaatt actagcttgt 960
ttaaagatgt atttggcaca tctgtttatg gacagactgt aattcttact gtaaggcaca 1020
tctctgtcac caagattctg aaatgcgtgc ttgatgacaa gttttgttaa tgtaacaata 1080
gtaacaattt tggcagaaac cacaagtgac aatgagaaga ctgtgactgg agaaaattaa 1140
taaagcaatt tataagtagc tcaagcaact tttctaaact atgattggac cctgtcggtg 1200
tggattgatt gagggctggg aaccaagact ggctggatga ctgcctcaat gggtttagca 1260
tgcgatgtgc aaatgctgac ctgcaaaggc ctaacccaca gagccctttc tgcgttgctt 1320
ccagtctcag agtgtcctga tgcctgcaac gcacagcaca agcgaatgct taataaagaa 1380
gagtggtggg gtcccctgca gtgttgcgtt gcgtgcccgg tctaccagga agatgctaat 1440
gggaactgcc aaaagtgtgc atttgggcta cagtggactc gactgtaagg acaaatttca 1500
gctgatcctc acttatttgt gggcaccatc gctggcattg tcattctcag catgataatt 1560
gcattgattg tcactagcaa gatcaaataa caaaagcgaa gcatattgaa gaacgagaac 1620
ttgattgacg aagactttca aaatctaaaa ctgcggtcgc acaggcttca ccaatctatg 1680
gagcataacg gagcgtcttc cctcaggtca ggattacggc ctccaagaga ccgcctagat 1740
gcaaaaatcc cgtagtttca agacacagca gcatgccccc ggcctgacta ttagaatcca 1800
tcagaatgtg gaacccgcca tggcccccaa ccatatgtac atatctatta ttctagcagt 1860
gtttagacaa gactgcatgg agaagtgagc accacgtaaa gactctggcc tccgggagtt 1920
tcttcttcca tctagacata ctgccagtcc tcatctgcaa tggcaacgtt gtgcaatgtc 1980
ttgcaaacga catccacgct cacttgctaa aataagaatc tatgacatta acatgtagct 2040
cgatgctatt agcgctgtgc tcagagaggt gggttttctt caatcagtaa caaagtactg 2100

agacaatgct taggggttgg tttcttaatt cttttccctg gtagggcaac aagaccccat 2160
ttccaaatct agaggaaagc ctccccagca ttgctttgct ccctgggcca aaccatgctt 2220
cttgagttaa gttgacctaa cttcccctgg gacgacatac cgcatcaact gtggaggtcc 2280
gagggggatg agaaagggat acccaccatc tttcataggg tcacaagcta cactctcgtg 2340
acaagtcaga ataggggaca cctgcttcta tccctccaat ggaggagatt ctggccaaac 2400
cccccttttt ttgaaaacca ggcccccaga gcttggcaac ctagcctcaa cccaagaaga 2460
ctggaaagga gacatatctt ttcagctttt tcaggaggcg tgccttggga atccaggaac 2520
gtttttgatg ctaattagaa ggcctggact ataataatgt ccatctatgg ggttttaatc 2580
tacagttttt gaacatgcta ggaggcagaa cggggccaga gagtaaaaaa acatgacctg 2640
gtagaaggaa gagaggcaaa ggaaactggg tggggaggat caattagaga ggaggcacct 2700
gggatccacc ttcgttcctt aggtcccctc ctccatgcag caaaggagca cttctctaag 2760
tcatgccctc ccgaagactg gctgggagaa ggtttaaaaa acaaaaaatc caggagtaaa 2820
gagccttagg gtcagttttg aaaattggag acaaacttgt cttggcaaag ggtgccaaga 2880
gcggagcttg ttgctcagga gtcccagccg tccagcctcg gggtgtaagg tctctgaggt 2940
gtgccatggg ggcctcagcc ttctctggtg acccgaggct cagctgtggc caccaacaca 3000
caaccacaca cacacaacca cacacacaaa tgggggcaac ccacatccac gtaaccaagc 3060
tttaacacaa atgttattag tgtccctttt tatttctaat agccctgtcc tcttaaaagt 3120
tattttattt gttattatta tttgttcttg actgttaatt gtgaatggta atgcaataaa 3180
gtgcctttgt tagatggaaa aaaaaa 3206

<210> 5
<211> 2610
<212> DNA
<213> Homo sapiens

<400> 5
gatgtgggca cgcctcagag ccagaagttt atggctccca cctgctcaat ctgacaggaa 60
gcttctgctc cccagttctc cccagccact gtggtctaca gattccagga aacccatccc 120
cctgtgacct cagggtgtgc tctgttctcc accctaggga ccagaaggag ccaggagtaa 180
agaactggct tacttggccg ccactgggaa attctgggta attcgagacg ccctggaatt 240
tggacccact ccgctgatag gtggtgggca gggttctagg gaacacaaga ggcggagcca 300
ggtggcttcc ctgtgctggc attcttggct ctctctctct ctctttctct ctctctgtct 360
ctctctctct ctctgtctct cagccttgca gcccgtttcc cctccctgcg cttcagtgtg 420
agtgtgactc gatttcaggg aaagggaact cgcgtgggct gaggagaccg gagtggacgg 480
gctggggaag gcaccgtgat gcccgcaacc cccgtcccct ggaaggggtg gtccatgagc 540
tgcctgcctg taccctctgt gcggggccgc tggaggatgc ggtgaccatt ccctgtggac 600
acaccttctg ccggctctgc ctccccgcgc tctcccagat gggggcccaa tcctcgtggc 660
aagatcctgc tctgcccgct ctgccaagag gagtagcagg cagagactcc catggcccct 720
gtgcccctgg gcccgctggg agataactta ctgcgaggag cacggcgaga agatctactt 780
cttcttgcga gaacgatgcc gagttcctct gtgtgttctg cagggagggt cccacgcacc 840
aggcgcacac cgtggggttc ctggacgagg ccattcagcc ctaccgggat cgtctcagga 900
gtcgactgga agctctgagc acggagagag atgagattgt aggatgtaaa gtgtcaagaa 960
gaccagaagc ttcaagtgcg gctgactcag atcgaacaag caagaagccg tcagggtgca 1020
cacagctcct tgagaggctg caagcgggag ctgcagcagc agcgatgtct cctgctggcg 1080
caggactgag tggtacgctc ggagtcacag atttggaagg agaggatgaa atatatcaca 1140
aaggtctctg aggaagtcac ccggcttgga gccccagctc aaggagctcg gaggagaagt 1200
gtcagcagcc agcaagtgag cttctacaag atgtcagagt caagccagag caggtgtgag 1260

atgaagactt ttgtgagtcc tgaggccatt tctccctgac ctgttcaaga agatccgtga 1320
tttccacagg aaaatactca ccctcccaga gatgatgaga atgttctcaa gaaaacttgg 1380
cgcatcatct ggaaatagat tcaggggtca tcactctgga ccctcagacc gccagccgga 1440
gacctggttc tctcggaaga caggaagtca gtgaggtaca cccggcagaa gaagagcctg 1500
ccagacagcc ccctgcgctt cgacggcctc ccggcggttc tgggcttccc gggcttctcc 1560
tccgggcgcc accgctggca ggttgacctg cagctgggcg acggcggcgg ctgcacggtg 1620
ggggtggccg gggagggggt gaggaggaca gggagagatg ggactcagcg ccgaggacgg 1680
cgtctgggcc gtgatcatct ctgcaccaag cagtgctggg ccagcacctc cccgggcacc 1740
gacctgtccg ctgagcgaga tcccgcgcag gcgtgagagt cgccctggac tacgaggcgg 1800
ggcaggtgac cctccacaac gcccagagcc caggggccca tccttcacct tcactggctc 1860
ttttctccgg ccaaggtctt ccctgtcctt ggccgcctgg acacaaaggg tcctggcctt 1920
aggctgacac gggggaaatg gggcgcgcga agggcggcga agcggagacg gcggctctcc 1980
gggatccagc tccgcccctg gccagtgtgc ggcccggggg ctccctgtgc ccgcgtgagg 2040
cgagagaaac acggggactt gagtctcgaa cagcggttgt tttactttta tttatcttag 2100
gccctcagct ccctgacgtc ctgagcctcc ctgtgacgct ctggccttct ctgcacctca 2160
gagtgcagaa ccacagacgg cttcggctgt gcctagggca acagccaacc taggaacccg 2220
ccggcctttc ggggaaaaac taaagaagga gacatctaaa atgtaatgtt taaactgttt 2280
caagataatt atcttgggaa aaatcagggt tttgctggac ttgcactaat ttgtacagtt 2340
aacttcgtac tttgacacac acctgaagat gcctccacct ttgtagggct tagggccttt 2400
ttatcagccc tgggtggacc ccaggccccc ttcctttccc ttcccttctg gtcatttctc 2460
tggacttgta gagaatgtcc taagaaagtg tgactcacag acctctggat tccatgtgtc 2520
caattagcgc tgatgggact ggagaaaggc ttaaatccaa tgggatcttg cctgtgttgg 2580
caatttaggg ccgagatggc tcgagggagt 2610

<210> 6
<211> 1627
<212> DNA
<213> Homo sapiens

<400> 6
ttttattttc tagagtgata tatatttttt ggtctttttc tttttttttc ttccaaaaca 60
aacaattaga gctttaggcc cctcgccctc cccacaccca ccgcagaacc ctcccatata 120
atcgacaact gaaaacaagc gagacaatca cccccaaaga gatcacgaaa cacgagcaca 180
agtttcacag acagccaccg acaaagcaaa aaaacttgct actaggaatg tccgccttgc 240
atgatcatgt agaagcagga gcaagagtct acaaattgaa tggggacctg attaagtatg 300
gggtagcagg gggatggtac ggaatcagaa gagtaaagct tccatgctga tgcgttaggt 360
gccattttgc ccctttcctg ttgcacggcg ggtactgttt tcccagaagc gcgcgcacgc 420
acctggccac gcagatctgc agtcctaggc cctgtgtagt caggatgtcc atagcccggt 480
ccctggggcg ggtctccttt ggcgctgggg ctagagccgc caagcccggg gcttctctgc 540
gtgggtcgag aagccgacgg gattcggagg aacgctgcag agcgttgtcg cactggggcc 600
gttgcatcct ccctgtccca tgtaccactt gtacccggaa gggagtcatt gggaatcgag 660
tgcgcaaata aattctcatt cggactctcc tggcctggct ttcctgtcta cagtggggtt 720
gacactagcg gtggaacgga aggtggaggg atttttctac aaggggcggc ttgacttgcg 780
ggtgcaaggt ggatacgacc gaagagagtt gatttcagag ctagggaggg tgcggaagaa 840
tgcagtgccg gtcgaagagc aagagaagct acagtctgtc aagtggtgca cagatgaaca 900
ggaggacaac attgtcaagg ctcatacgac ccacagtgtg accttatttt gttggaagga 960
tgagggaaac atcatgctgg taaatataac atttcgtgca acaataatgt atataatggt 1020

gggaggtggg gagtagctcc acctaagata ccttcataaa accacgtgct gccttttctt 1080
gtactttcta gcccaccggc ttgggggcta ggtttgctcc atcttcccca tggcccttgg 1140
cctgagaata gttggccact ccatgggaat ggtatggcca tgctgcagcc tttgggctgc 1200
aactcctcac tcaggagtct gcctctagac atctccctgg tgggtatttg cattaggggt 1260
agaacccggg cttgcctgac agtctgaggg ctgttttgcc caatttggtg tgcgatggtc 1320
tgcaactggt agtgtcacct cacttgactg aatggtggtt gtgagctcac cccattactg 1380
tgtgtgaatg tctgctgagc tgtgtagagt tggagtgtcc ctgggtgact tttgggtggg 1440
tgtagagaag aaacaggcaa gctggaagtg aggggctagg acttcccaga aaaattacag 1500
ggcatactag gagcttgact ggggtctctc tttccttgtg gcccatcaca ttcttaggaa 1560
ccaactattt ctatcttcta aatcaacaaa actttctcct gacacctaga gacctgagca 1620
agccatg 1627

<210> 7
<211> 929
<212> DNA
<213> Homo sapiens

<400> 7
catgtatgca ataaaaaata aaagatacat acacaaaatt ctttaaatgt cccacacaca 60
agacaaatac gtgttcaaat acatcagtct ctgaagcctc tgcaccactc tacacgctgc 120
tccttctgac tagtaatgcc ctcctgcccc tcctgtccac gtgtcaaact cccaatcacc 180
ctttaaaacc agattgaatt attttgcttc tgtgaagctt tccctgacta tccccgggat 240
agaataatgt ttccactagt gttttgtcat ttactcgcta taataagaat acgaaagaac 300
atgtattttt gaaaagtatc tgtgatctct aatgagcttg taaacatctt gaggaataga 360
gactaagttt tgcttctttg ttcccccaaa gagaacttta ttaataacat ttaccatctc 420
tttagagaga gggtttttcc catctctgtg agaaagctcc agaatctaca accaggaata 480
agtgttaatg ggatagaacc aatgtagaga acagcatatg atatgtgaaa tgtactttat 540
tattaatacg aattcagtgg gctcacagaa tgaacctttt tgccaaactg gggggaaagc 600
attttctgta aaggtatctt tagaaaaata tgtataattt gaaaaatggt tatccaaatt 660
taacatttgt catataaaag gctcataaaa cgtgtgtggc tgtgtttctc aaaattgtgg 720
ggtcaattgg tcacattatg cctagacatt ctggttttgt tgcttggggt taataatggt 780
tgtggtctta tacagaaaag gaaatctgga catcttgccc ctgttattaa tacacctgtc 840
attactaata aaagtggttt gttgatatgc taaataggtt gaaaaagctg tcactttgca 900
tgaaattaac tagggaatac ttctttata 929

<210> 8
<211> 2303
<212> DNA
<213> Homo sapiens

<400> 8
gagaggaagc agcatcagga caccttacca ccactgccgc tgcctcagca tccaccccgc 60
agcccacgtg tggcaaaccg gggaaggggt ggagtgaacg gccggagacc acgtggagaa 120
aggggccgct ttggcccttc catctgggtg ccgggagccc ctaggccctc cggccatggc 180
cgacagcggc gatgctggca gctccggccc ctggtggaaa tcgctcacca acagcagaaa 240
gaaaagcaag gaagccgcag tgggggtgcc gcctcccgcc cagcccgctc ccggggagcc 300

cacgccacct gcgccgccca gcccggactg gaccagcagc tcccgggaga accagcaccc 360
ccaatctcct cgggggcgcc ggcgagcccc ccaaaccaga caagttatac ggggacaaat 420
ccggcagcag ccgccgcaat ttgaagatct cgcgctccgg ccgctttaag gagaagagga 480
aagtgcgcgc cacgctgctc ccggaggcgg gcaggtcctc ggaggaggca ggctttcctg 540
gtgaccccca cgaggacaag cagtagcccc aatagcctgc gcgctccagg actgcctacc 600
cagcactacc ccaaaccccc agttccaaac ccgagacttc aggcccgccc ccttacgcgt 660
tgtctcattc caccaaattc agaatattta cacaatgcct tcatgattaa atttttctgg 720
aacttgaagt gtcaattggg ttctcaagat ttcatgacgc caaggatgcc ttgaatattt 780
atttgtggta agagaagata cctgccgcgg agtagggtgg cataattatt ttttttctac 840
agtgcaaggg ttttaatagt ccacactaaa ataggctgta cacttttgta gtttaacatc 900
tcaaagcaat cctgccttat gtttaaaatg cttctactta agaatgcttc tgtcctcccc 960
gcactccgtt cacttacagg tataagtcta cccctagaag tgcatttctc acggcaatta 1020
aaaactagca ctgtgatttg ctttcctaca gagtcctgaa ataactagcc accttccttg 1080
catttgatga ggctactaga gttccaagct cgagctcgtg actaggagca cagggggcca 1140
gggcccacag aatacgcttt cttagaagaa aaaactaatt atgccaccct tcttccgcgg 1200
caggtatcta tctcttacca caaataaata tttacaatgc atccttggga gtcatgaaat 1260
attgagaacc caataagaca ctacaatttc cagaaaaata aaatcatgaa ggcattgctg 1320
taaatattct gcaatttggt ggaatgagaa caacgcgtaa gggggcggac ctgaagtctc 1380
ggttttggaa ctgggggttt agaggtagtg ctgggtaggc agtcctggag cgcgcaggct 1440
attggggcta ctgcttgtcc tcgtgggggt caccaggaaa gcctgcctcc tccgaggacc 1500
tgcccgcctc cgggagcagc gtggcgcgca ctttcctctt ctccttaaag cggccggagc 1560
gcgagatctt caacattgcg gcggctgctg ccggatgtgt ccccgtataa cttgtctggt 1620
ttggggggct cgccggcgcc cccgaggaga cttcggggtg ctggttctcc cgggagctgc 1680
tggtccagtc cgggctgggc ggcgcaggtg gcgtgggctc ccccggagcg ggctgggcgg 1740
gaggcggcac ccccactgcg gcttccttgc ttttctttct gctgttggtg agcgatttcc 1800
accaggggcc cgagctgcca gcatcgccgc tgtcggccat ggccggaggg cctaggggct 1860
cccggcaccc agatggaagg gccaaagcgg cccctttctc cacgtggtct ccggccgttc 1920
actccacccc ttccccggct tgccacacgt ggggctgcgg ggtggatgct gaggcagcgg 1980
cctgtgctgg gaggagggcc ctgggaacca agtgcatcct ctctacaggt gaacggtatt 2040
aattaagtcc atggtcaaac aagtcacgaa atttccctcc aaagatttgc ccccatcgac 2100
tttcgtccca ggaagccttt tcgatgagat acttaggaga attttatatc ccagttagga 2160
agagaaggac aagcttatga tatttggttt tgggttcctt ttaaaattct ggcttttgac 2220
caattctgcc ttgtgacttt caaagaagca tgtctagact taactttccc ttgaaaaacg 2280
gcatcctaaa tcttcccttt act 2303

<210> 9
<211> 1769
<212> DNA
<213> Homo sapiens

<220>
<221> unsure
<222> (878)..(948)

<400> 9
attctccagt cacttcctat agacttctgg cttcctgtca ggcatataac aagcttgaaa 60
tttgtcactg gtttctaacg ctaagtaaaa agctgaacaa actcaaaagt caacaacttg 120

ttaaaatccc tcagagatgg ctgggcactc catctctgag tggactcttg accccatcct 180
cactcatgac gccatcctca acctgctgtg gcgctcatat cctccagtgg atcctgggac 240
ctcccccagg tggagctggc caggcaggtg ctgtctgata ggtttgctgc ccattccaca 300
tacacctgtg tcctcatgat gatgccattg tcataaggtg gagtcccttg gactgagaag 360
tgaaccagcc actggcgtct cacttagact ctacccagtt acaaaaactt aaactctagt 420
tgtgttttct gaggttgata ggagaggaag aaaacctttc acatgcctgt tttgaggctt 480
ctcctctttt tgcctaactc tgcacaggaa ctaggggcag ggagcgcttt ctaaatttac 540
taacatcaca cacattgctt ctcctaactt ggcatcattt ctccctttat gtaactgaca 600
cacacctaag agttcctctc tgaccggttc tgtcctctta acaggtctca catccctctc 660
tctgttcagg gagtcactga tttcaaacca ctttcagcat cttgccttag agcataatgt 720
gatcactttg gaattcagag cagacctaaa ccttagcata atattaaaat gaaatactac 780
ttcctagcaa attagataat tagatcttta ggaccaatga taagaattgt ccaccttatg 840
gaaaagactt taaggtgttc ccccaaatgt ctttcacnnn nnnnnnnnnn nnnnnnnnnn 900
nnnnnnnnnn nnnnnnnnnn nnnnnnnnnn nnnnnnnnnn nnnnnnnnac tacagattga 960
gtatcccaaa tccgaaaatc caaaaatcca aaatgtacca aaaatctgaa atgctcccaa 1020
aatccaaaac ttttgagtgc caacataaca attaaaacaa aaatgctcac tggagcattt 1080
cggatttggg attggatttt ggattttcag attagggatg ctcagctggg tgtcagatgc 1140
ctgatacatt caattcatgg tttcttataa ccctactcca cgtctgggag atttatgtag 1200
ttggaatttg tgttggcatt gtaagtgtta acagatttgt agagactccc cttttcaaat 1260
tgtcatggag cactagtacc ttctcagtgc agaaattaat tttacaaaat ggaatggaac 1320
aaataaaatt ggaacatacc tatgatggag gctgtcctgt ggccctcatg ctccccccag 1380
aagggttagg cttcatagtg agggagtttg ggaaaccagg tggagatagc catgtacaca 1440
gccctggaaa agggatgtgt ctagtccgaa tgaagcagga aggccggagt gggaagtaca 1500
tgtgtcgtat catagttcat tttatgtggg aggatgttca gcagcgcggc agagtcatgg 1560
ggtgggttcg tggtctcgct gacttcaaga atgaagccgc agaccttcac agcaagtgtt 1620
accagctctt aaaggtggtg cggacccaaa gagtgagcag cagcaagatt tatggtgaag 1680
accgaaagaa caaagcttcc acagtgtgga agggggacct gagcgggttg ccactgctgg 1740
ctaggggcaa agttctccct gtggactga 1769

<210> 10
<211> 2159
<212> DNA
<213> Homo sapiens

<400> 10
cactagcaga gaagctgttg tccttccacc accagcaccg gaccacctgc tccaagacca 60
gcctcctggg gggaccaggc acccggcctt cactggcacc cagggagccg tcctcagcag 120
cgtcaacatg tcaaggccca gcagcagagc catttacttg caccggaagg agtactccca 180
gaacctcacc tcagagccca ccctcctgca gcacagggtg gagcacttga tgacatgcaa 240
gcaggggagt cagagagtcc aggggcccga ggatgccttg cagaagctgt tcgagatgga 300
tgcacagggc cgggtgtgga gccaagactt gatcctgcag gtcagggacg gctggctgca 360
gctgctggac attgagacca aggaggagct ggactcttac cgcctagaca gcatccaggc 420
catgaatgtg gcgctcaaca catgctccta caactccatc ctgtccatca ccgtgcagga 480
gccgggcctg ccaggcacta gcactctgct cttccagtgc caggaagtgg gggcagagcg 540
actgaagacc agcctgcaga aggctctgga ggaagagctg gagcaaagac ctcgacttgg 600
aggccttcag ccaggccagg acagatggag ggggcctgct atggaaaggc cgctccctat 660
ggagcaggca cgctatctgg agccggggat ccctccagaa cagccccacc agaggaccct 720

agagcacagc ctcccaccat ccccaaggcc cctgccacgc cacaccagtg cccgagaacc 780
aagtgccttt actctgcctc ctccaaggcg gtcctcttcc cccgaggacc cagagaggga 840
cgaggaagtg ctgaaccatg tcctaaggga cattgagctg ttcatgggaa agctggagaa 900
ggcccaggca aagaccagca ggaagaagaa atttgggaaa gaagagaaca aggaccaggg 960
aggtctcacc caggcacagt acagttgact gcttccagaa gatcaagcac agcttcaacc 1020
tcctgggaag gctggccacc tggctgaagg agacaagtgc ccctgagctc gtacacatcc 1080
tcttcaagtc cctgaacttc atcctggcca ggtgccctga ggctggccta gcagcccaag 1140
tgatctcacc cctcctcacc cctaaagcta tcaacctgct acagtcctgt ctaagctcac 1200
ctgagagtaa cctttggatg gggttgggcc cagcctggac cactagccgg gccgactgga 1260
caggcgatga gcccctgccc taccaaccca cattctcaga tgactggcaa cttccagagc 1320
cctccagcca agcaccctta ggataccagg accctgtttc ccttcgggcc tccagtcccc 1380
aaacctgccc agccagtccc tgaaaatgca agtcttgtac gagtttgaag ctaggaatcc 1440
cacgggaaac tgactgtggt ccaggtagag aagctggagg ttctggacca cagcaagcgg 1500
tggtggctgg tgaagaatga ggcgggacgg agcggctaca ttccaagcaa catcctggag 1560
cccctacagc cggggacccc tgggacccag ggccagtcac ccctctcggg ttccaatgct 1620
tcgacttagc tcgaggcctg aagaggtcac agactggctg caggcagaga acttctccac 1680
tgccacggtg aggacacttg ggtccctgac gggggagccc agctacttcg cattaagacc 1740
tggggagcta ccaggatgct atgtccacca ggaggccccc acgaaatcct gtcccggctg 1800
gaggctgtca gaaggatgct tggggataag cccttaggca ccagcttaga cacctccaag 1860
aaccaggccc cgctgatgca agatggcaga tctgataccc attagagccc cgagaattcc 1920
tcttctggat cccagtttgc agcaaacccc acacctccag cgtcacacag caaaaacaat 1980
ggacaggccc agaggctgaa gcaaacagtg tcccttctgg ctgtgttgga gcttccccag 2040
taaccaccta tttattttac ctctttccca aacctggagc atttatgcct aggcttgtca 2100
agaatctgtt cagtccctct ccttctcaat aaaagcatct tcaagcttga aaaaaaaaa 2159

<210> 11
<211> 3872
<212> DNA
<213> Homo sapiens

<220>
<221> unsure
<222> (2663)..(2664)

<400> 11
gaaaccgaca caaatacctg aaatacacag ccacagacag acacacacgg aagcactcta 60
tgcacaaaac actcacacag tacacaccat gctgcacata ccctgaccca aacagtctaa 120
caagccctga gggtctccag ggctgccctg gggctattgc ccacccctcc caccgtcccc 180
gctagggtga gatggtgttc cccagggaac agaagtctcc agtcccatct taagctctgc 240
cggatcccgc gtgacatcag ctagccccct cgcggctgcc gggagctgtg agctctgtgc 300
tggggccagg ccggcaccag gcacagacac ttaggccctt gttgggagaa cagagagagg 360
ctctcttgtc cactgcctgt cttcggttcc aactgctggt tctcctagag gcctctcctc 420
agactcgcag gtatgtggga ccagggaggc cgggtcctgg ccaaaggccc actggggtca 480
gcccaggaga gggtgtggca gtgttgtggg ccgtttgcag gagcacacac gtctggcatt 540
ggctaggggc aggctgcgct tccttagcag ttctgcagct tgctcttaag gcttggcagg 600
gctgggcctc tcagggaagc ctgggctggg ggatcctctc agttcccctt cactttctct 660
gttcccaaga aggccatgag gttggtgcct ccaggacccc cccttgtaaa gataggaaat 720

ctctactcag agaggctggg ctgcagccca ggccccacag tgggccaaga ctaaggtctt 780
gagatgcgcg gcaactgggc tttcaggtga gatctctgct cttcagcctt ttccaagcaa 840
ggatgagact ttggggcccc aagcaatctg tttgcagggc ctgggcaccc tggccccttc 900
tcccctgcag ggtggaagca aggaagacac tattcctggc cacatagatc agctggtcac 960
accttctgtt gtttggcccc gaatagatat tggccagtct tgggtctctc tgtggcccca 1020
gcccaaggct tccagggcag ctgcctttcc tgaggcattg ggcagaattc cttgtggcaa 1080
ggagatcgta gcacagagcc cagctgggac tgcgcacagt aattcagggt tgccattgtt 1140
cctctatggg agtccggaga gcccagcctg tgcttcacaa ggctatgtgg ccctaagaag 1200
gtcctttttt aggccacagg ccttccatct gtgaaatggg ggatgggttc agactttatg 1260
ccctgaaaag atccttccag ccctggccat cttggacttc tggagctacc ctggctcaca 1320
ggggtcttgt tgccctgggt gtccccagtt cttgaaaaga atcagcctgg gaggggccac 1380
accctgacca tcccccctta tcccttctga gatgtttgtt aggaagtctg ggtccagggg 1440
atatcatttc ttgttccatc catgcagggg ttgcttacct cgggtaggaa accctcaggc 1500
ggtggcaggt gcacaggtag gggaggatgg agagggcagt ggtgcctgaa gccctggatg 1560
ggcggagctg accccccaac accaactcta tcatgcctgc tcctccctgt ccccccagag 1620
ctgcctgatc attgctacag aatgaactct agcccagctg gtgaccccaa tgtccacagc 1680
ccgtccaggg gccaaatggg aacatcaacc tggtgtgcct tcagccaacc caaatgccca 1740
gcccacggac ttcgacttcc tcaaagtcat cggcagaagg gaactacgtg gaagtgtcct 1800
actgtgccaa gcgcaagtct gatggggcgt tctatgcagt gaatggtact acagaaagaa 1860
gtccatctta aatgaagaaa gagcagatgc cacatcatgg cagagcgcag tgtgcttctg 1920
aagaacgtgc ggcacccctt cctcgtgggc ctgcgctact ccttccagac acctgagaag 1980
ctctacttct gtgctcgact atgtcaacgg gggaggagct cttcttccac ctgcagcggt 2040
gagcgccggt tcctggagcc cctgggccat gttctacgct gctgaggtgg ccagccgcca 2100
ttggctacct gcactccctc aacatcattt acagggatct gaaaacagga gaaacattct 2160
cttggactgc cagcccatgc cctccgtcat tctcagggac acgtggtgct gacggatttt 2220
ggcctctgca aggaaggtgt agagcctgaa gacaccacat ccacattctg tggtacccct 2280
gagtattgtg ccccctgaag tgcttctgga aagagcctta tgatcgagca gtggactggt 2340
ggtgcttggg ggcagtcctc tacgagatgc tccatggcct gccgcccttc tacagccaag 2400
atgtatccca gatgtatgag aacattctgc accagccgct acagatcccc ggatgccgga 2460
cagtggccgc ctgtgacctc ctgcaaagcc ttctccacaa ggaccagagg cagcggctgg 2520
gctccaaagc agactttctt tgagattaag aaaccatgta ttcttcagcc ccataaactg 2580
ggatgacctg taccacaaga ggctaactcc acccttcaac ccaaatgtga caggacctgg 2640
ctgacttgga agcatttttt ganncccaga gttcacccag gaagctgtgt ccaagtccat 2700
tggctgtacc ccctgacact gtggccagca gctctggggc ctcaagctgc atttcctggg 2760
attttcttat gcgccagagg atgatgacat cttggattgc tagaagagaa ggacctgtga 2820
aactactgag gccagctggt attagtaagg aattaccttc agctgctagg aagagcgact 2880
caaactaaca atggcttcat ccgagttagt caggtttatt gttattgcca gcatcatata 2940
aagatgagaa tatatgtctc tacggaggtg ccatggatct ggcaggatca ggctcatcag 3000
actacctcca cgaggactgt atctctgccc tgccaacctt gacaaatggc ttccaaatgt 3060
ttaggtttgc ttacaaagat ggttactggg agctctaagc ctgccttatt ttggtgtttt 3120
tagggaaggg aaaatgggag gaaaggggag aagagcaaag ggcgcttttt aaagagcttt 3180
ccctaaaagc tccatccaat gagctttctg cttccatctc acttaaccac ccacccctac 3240
ctgggaatgg aggcctggga gatgtggctt atttgctggg tacgtgacta tccctaataa 3300
caaaggggtt ctgacactaa gacattaggg gagaatgttg ggtaggcagc cagcactctt 3360
ttaccagagg gcctcctggt gtttggattt tgatctcaat gtgtaaacat gacagagatg 3420
taacaagctc atagggtatc aatatctctt attgttctat gttgatgata tttgtctttg 3480
ttgtgggtaa tactggacat tttgtttatt gggtctgggt gccttggtta tctgaacccc 3540
cttcttgtct ccagagaacc ccctatttta tgagacttca tgggggggca ataactacct 3600

ccacttaaga gtacctgaaa atgctagaca ctgactttcc cagcctcccc ttagctaggg 3660
ccaggcatgg ggaccaggca taaacctgtg ccacattttg actcagggaa gggatcggga 3720
gagctctttt gtgtggtaac tgtgataaca gtacccgcaa aattgagttc ctggtgtaga 3780
agtgacaagg atgcaaactg tagcagttgg tgctcagtgg cagcaacgcc atcagaccag 3840
ccctgcaatg tcattcctgg aagcctcaag tg 3872

<210> 12
<211> 4728
<212> DNA
<213> Homo sapiens

<400> 12
atggccagcc agcgggtaag cttccagcac gaggtgtacc cagcggagcc agccacaggc 60
cctgcggccc ccagccagga gctggaggag cgaccgctgt cccgtcaggt gttcatcgtg 120
caggagctgg aggtccgaga ccggctcgcc tcctcccaga tcaacaagtt cctgtaccta 180
cacacgagtg agcggatgcc gcgacgtgcc cactctaaca tgctcaccat caaagcgctg 240
catgtggccc ccactaccaa cctgggtggg cctgagtgct gtctccgcgt ctcgctgatg 300
cccctgcggc tcaatgtgga ccaggatgcc ctcttcttcc tcaaggactt cttcactagt 360
ctggtggccg gcatcaaccc cgtggtccca ggggagacct ccgctgaggc tcgccccgag 420
actcgagccc agcccagcag ccccctggaa gggcaggccg aaggcgtaga gaccactggt 480
tcgcaggagg ccccaggagg tggacacagc ccctcccctc ctgaccagca gcccatctac 540
ttcagagagt tccgcttcac gtctgaggtc cccatctggc tggattacca tggcaagcac 600
gtcacgatgg accaggtggg cacttttgct ggcctcctca tcggcctggc ccaactcaac 660
tgctccgagc tgaagctaaa gcggctctgt tgcaggcacg ggctcctggg tgtggacaag 720
gtgctgggct atgccctcaa cgagtggctg caggacatcc gcaagaacca gctgcccggc 780
ctgctgggag gcgtgggccc catgcactcg gttgtccagc tcttccaagg gttccgggac 840
ctgctgtggc tgcccattga gcagtacagg aaggatggcc gcctcatgcg ggggctgcag 900
cgaggggctg cctcctttgg ctcatccaca gcctctgccg ccctggaact cagcaaccgg 960
ttggtacagg ctatccaggc cacagctgag accgtgtatg acatcctgtc cccggcagcc 1020
cccgtctccc gctccctgca ggataagcgc tctgcgcgga ggctgcgcag gggccagcag 1080
cctgccgacc tgcgggaggg tgtggccaag gcctacgaca cagtgcgaga gggcatcttg 1140
gatacagctc agaccatctg tgacgtggca tcgcggggcc atgagcagaa ggggctgacg 1200
ggcgccgtgg ggggcgtgat ccgccagctg cccccgactg tggtgaagcc gctcatcctg 1260
gccacggagg ccacgtccag cctgctcggg ggcatgcgca accagattgt ccccgacgcc 1320
cacaaggacc acgccctcaa gactggcacc tgtcaccgga acctgtctgg gagggacgag 1380
aacacgcttt gcaagaggaa gctctgcctc acagagccct gggctcactc agggaccctg 1440
gccagcagct gcttcctctc cccacagcgg agagagaccc aagggtccca gggcggatgc 1500
ttcccaccag gccagcccag cgtgcagggt ggcctccccc ccacacttct tcttagtctc 1560
atcttcagct tcccatacga ggccatcctc atgaaatcag gcactgggag gtccctgggg 1620
actgacaagt gccagctgtc ccttgctgtc tctctgcccc atggctgcag cagggaggga 1680
aggagtgctg gcagcacacg gggcgccagg tgtgggcccc ggatgataag aagcctcggt 1740
gaaaagacca tggacctggg gccacgaaga ctggggagcc cagcaactcc atgtggaagt 1800
gcccactggt tccagtgggg ctgctgttat ctggggcgag ggccagtacc cacgaagaag 1860
gagaggcagg taagcttcca gcacgaggtg tacccagcgg agccagccac aggccctgcg 1920
gcccccagcc aggagctgga ggagcgaccg ctgtcccgtc aggtgttcat cgtgcaggag 1980
ctggaggtcc gagaccggct cgcctcctcc cagatcaaca agttcctgta cctacacacg 2040
agtgagcgga tgccgcgacg tgcccactct aacatgctca ccatcaaagc gctgcatgtg 2100

gcccccacta ccaacctggg tgggcctgag tgctgtctcc gcgtctcgct gatgcccctg 2160
cggctcaatg tggaccagga tgccctcttc ttcctcaagg acttcttcac tagtctggtg 2220
gccggcatca accccgtggt cccaggggag acctccgctg aggctcgccc cgagactcga 2280
gcccagccca gcagccccct ggaagggcag gccgaaggcg tagagaccac tggttcgcag 2340
gaggccccag gaggtggaca cagcccctcc cctcctgacc agcagcccat ctacttcaga 2400
gagttccgct tcacgtctga ggtccccatc tggctggatt accatggcaa gcacgtcacg 2460
atggaccagg tgggcacttt tgctggcctc ctcatcggcc tggcccaact caactgctcc 2520
gagctgaagc taaagcggct ctgttgcagg cacgggctcc tgggtgtgga caaggtgctg 2580
ggctatgccc tcaacgagtg gctgcaggac atccgcaaga accagctgcc cggcctgctg 2640
ggaggcgtgg gccccatgca ctcggttgtc cagctcttcc aagggttccg ggacctgctg 2700
tggctgccca ttgagcagta caggaaggat ggccgcctca tgcgggggct gcagcgaggg 2760
gctgcctcct ttggctcatc cacagcctct gccgccctgg aactcagcaa ccggttggta 2820
caggctatcc aggccacagc tgagaccgtg tatgacatcc tgtccccggc agcccccgtc 2880
tcccgctccc tgcaggataa gcgctctgcg cggaggctgc gcaggggcca gcagcctgcc 2940
gacctgcggg agggtgtggc caaggcctac gacacagtgc gagagggcat cttggataca 3000
gctcagacca tctgtgacgt ggcatcgcgg ggccatgagc agaaggggct gacgggcgcc 3060
gtggggggcg tgatccgcca gctgcccccg actgtggtga agccgctcat cctggccacg 3120
gaggccacgt ccagcctgct cgggggcatg cgcaaccaga ttgtccccga cgcccacaag 3180
gaccacgccc tcaagactgg cacctgtcac cggaacctgt ctgggaggga cgagaacacg 3240
ctttgcaaga ggaagctctg cctcacagag ccctgggctc actcagggac cctggccagc 3300
agctgcttcc tctccccaca gcggagagag acccaagggt cccagggcgg atgcttccca 3360
ccaggccagc ccagcgtgca gggtggcctc ccccccacac ttcttcttag tctcatcttc 3420
agcttcccat acgaggccat cctcatgaaa tcaggcactg ggaggtccct ggggactgac 3480
aagtgccagc tgtcccttgc tgtctctctg ccccatggct gcagcaggga gggaaggagt 3540
gctggcagca cacggggcgc caggtgtggg ccccggatga taagaagcct cggtgaaaag 3600
accatggacc tggggccacg aagactgggg agcccagcaa ctccatgtgg aagtgcccac 3660
tggttccagt ggggctgctg ttatctgggg cgagggccag tacccacgaa gaaggagagg 3720
caggtgctgg ccagcagacc agccaggact accgtggcga cgctcccagg ccagatggtg 3780
gcgggtagtg gagggctgtc tggtgggctg ccgagaccga gtgcacaggg ctctgaccta 3840
tgaattgaca gccagtgctc tcgtctcccc tctggctgcc aattccatag gtcacaggta 3900
tgttcgcctc aatgccagcc accaggacct gcagggatag gggagggccg ggggtgtcca 3960
gcagtcagca gagatcctgc gaccccagtg cagcactcat ggtcccacct ccctctgtct 4020
cattccccgt gaatgagcct gaacagcttc agtcctgccc ctgccctgcc tgccctgtgg 4080
cacctctatg ctttgcccat gctgttccct tgggctgcaa tactcttcct agcttatttg 4140
ccaggctcac tcttactaac cctttcaagc tctgtccaag catttgctgc ctccagaagg 4200
ccttattgaa gcttctaagt ccccacctgg gcacccccac acagtgctgc cgcagagcac 4260
tgccctctcg gagccccggg tgctggtttc tgcttatgtc tcgactcctc ttccccatct 4320
gtgagctcag ttcccagccc aaggcgcgtg cccaaataaa tgtttgctga accaatcctg 4380
agcctctgtc ttgcaacctg aggaagcaac ccaccgaaca atgcagtgtg gccaaagggg 4440
ggctgagtgc tctaggccca gtgtttgtgc ttggagcccc cccacccagg atggggccct 4500
gagccagcct ccccatctgc ttcctactct cccctccttt gccagtctca tctccctgga 4560
gcacagccct gtggttggtg gagcagcttc tccagcccct aggattccta agaggcccca 4620
ggaccccagc tgctggtaga ggaagagcag ccaacccagg acaggacagc tgaccccacc 4680
cctgtcccgc ctcccacaac agcctcattt ccacctattt ctttgtgg 4728

<210> 13
<211> 6650

<212> DNA
<213> Homo sapiens

<220>
<221> unsure
<222> (4298)

<220>
<221> unsure
<222> (4307)

<220>
<221> unsure
<222> (4311)

<220>
<221> unsure
<222> (4313)

<220>
<221> unsure
<222> (4315)

<220>
<221> unsure
<222> (4327)

<400> 13
tcctccacat accggctcag ctcctccagg acgcagcccg ccagacacgc tgtggaagct 60
gaggacccgg ccttgttttg ttcatgaaca ttgggtttag tgcctggcaa cttgatgcat 120
atggaagagc aatgccaagt gatctgacat aatacaaatt cacgaagtga cattcaatca 180
caagcaaagt tggaaattcc aaagagaagt ggtgagatct ttactagtca cagtgaagat 240
gggagaaaat gacatacctg cagcagatgt gggctgaaaa tatcctcttc tctgcccaat 300
caggaatgct acctgttttt gggaataaac tttagagaaa ggaagggcca aaactacgac 360
ttggctttct gaaacggaag cataaatgtt cttttcctcc atttgtctgg atctgagaac 420
ctgcatttgg tattagctag tggaagcagt atgtatggtt gaagtgcatt gctgcagctg 480
gtagcatgag tggtggccac cagctgcagc tggctgccct ctggccctgg ctgctgatgg 540
ctaccctgca ggcaggcttt ggacgcacag gactggtact ggcagcagcg gtggagtctg 600
aaagatcagc agaacagaaa gctattatca gagtgatccc cttgaaaatg gaccccacag 660
gaaaactgaa tctcactttg gaaggtgtgt ttgctggtgt tgctgaaata actccagcag 720
aaggaaaatt aatgcagtcc cacccgctgt acctgtgcaa tgccagtgat gacgacaatc 780
tggagcctgg attcatcagc atcgtcaagc tggagagtcc tcgacgggcc ccccgcccct 840
gcctgtcact ggctagcaag gctcggatgg cgggtgagcg aggagccagt gctgtcctct 900
ttgacatcac tgaggatcga gctgctgctg agcagctgca gcagccgctg gggctgacct 960
ggccagtggt gttgatctgg ggtaatgacg ctgagaagct gatggagttt tgtgtacaat 1020
gaaccgaaaa ggcccatgtt gaggattgac gctgagagga gcccccggtc gtggccagca 1080
ttatgcatgt gtggatccta actgacatgt ggtgggcacc atctttgtga tcatcctggc 1140
ttcggtgctg cgcatccggt gccgcccccg ccacagcagg ccggatccgc ttcagcagag 1200

aacagcctgg gccatcagcc agctggccac caggaggtac caggccagct gcaggcaggc 1260
ccggggtgag tggccagact cagggagcag ctgcagctca gcccctgtgt gtgccatctg 1320
tctggaggag ttctctgagg ggcaggagct acgggtcatt tcctgcctcc atgagttcca 1380
tcgtaactgt gtggacccct ggttacatca gcatcggact tgccccctct gcgtgttcaa 1440
catcacagag ggagattcat tttcccagtc cctgggaccc tctcgatctt accaagaacc 1500
aggtcgaaga ctccacctca ttcgccagca tcccggccat gcccactacc acctccctgc 1560
tgcctacctg ttgggccctt cccggagtgc agtggctcgg cccccacgac ctggtccctt 1620
cctgccatcc caggagccag gcatgggccc tcggcatcac cgcttcccca gagctgcaca 1680
tccccgggct ccaggagagc agcagcgcct ggcaggagcc cagcacccct atgcacaagg 1740
ctggggaatg agccacctcc aatccacctc acagcaccct gctgcttgcc cagtgcccct 1800
acgccgggcc aggccccctg acagcagtgg atctggagaa agctattgca cagaacgcag 1860
tgggtacctg gcagatgggc cagccagtga ctccagctca gggccctgtc atggctcttc 1920
cagtgactct gtggtcaact gcacggacat cagcctacag ggggtccatg gcagcagttc 1980
tactttctgc agctccctaa gcagtgactt tgacccccta gtgtactgca gccctaaagg 2040
ggatccccag cgagtggaca tgcagcctag tgtgacctct cggcctcgtt ccttggactc 2100
ggtggtgccc acaggggaaa cccaggtttc cagccatgtc cactaccacc gccaccggca 2160
ccaccactac aaaaagcggt tccagtggca tggcaggaag cctggcccag aaaccggagt 2220
cccccagtcc aggcctccta ttcctcggac acagccccag ccagagccac cttctcctga 2280
tcagcaagtc accggatcca actcagcagc cccttcgggg cggctctcta acccacagtg 2340
ccccagggcc ctccctgagc cagcccctgg cccagttgac gcctccagca tctgccccag 2400
taccagcagt ctgttcaagt tgcacagaat ccacgcctct tctgccgcga cacctcacac 2460
gaggaaaagg acggggcggg tccctcctga gcccacccct gggccctcgg ccaccacgga 2520
tgcaacatgt gcacccagta cttgccagat ttttccccat tacaccccca gtgtgcgcag 2580
atccttggtc cccagaggca caccccttga actgtggacc tccaggcctg gaacacgagg 2640
ctgctaccag aaaaccccag gcccctgtta ctcaaattca acagccagtg tggtcgtgcc 2700
tgactcctcg accagcccct ggaaccacat ccacctgggg aggggccttc tgcaatggag 2760
ttctgacacc gcagagggca ggccatgccc ttatccgcac tgccaggtgc tgtcggccca 2820
gcctggctca gaggaggaac tcgaggagct gtgtgaacag gactgtgtga gatgttcagg 2880
cctagctcca accaagagtg tgctccagga tgtttttggg cccctacctg gcacagagtc 2940
ctgctccgtg gtgaaatgga atggaccaca gcaaacacca ttcttttggc cgtacttcct 3000
aggaagcact gggaagagga ctggatgatg gtgggagggt gagagggtgc cgtttcctgc 3060
tccagctcca gaccttgctc tgacgcaaaa catctgcaga tgccagcaac atccatgtcc 3120
agccaggaca accagctgct gcctgtggcg tgtgtgggct ggatcccttg aaggctgagt 3180
ttttgaaggg cagaaagcta gctatgggta gccaggtgtt tccaaaggtg ctgctccttc 3240
tccaacccct acttggtttc cctacacccc aatgcctcat gttcatacca gccaagtggg 3300
ttcagcagaa acgcatgaca cctttatcac ctcccttcct tgggtagagc tcgtgagaca 3360
ccagcgtttg gccccctcca cagtaaggct gctacatcag gggcaaccct ggctctatca 3420
ttttcctttt ttgcctaaag gaccagtagg cataggtgag ccctgagcac taaaaggagg 3480
gggtccctgg aagctttccc agctatagtg tgggagttct gttccctgga gggtggggta 3540
cagcagcctt tggttcctct gggggttgag aataagaaat agtggggtag ggaaaaactc 3600
ctctttgaag atttcctgtc tcagagtccc tgagtagtta gaaaggagga atttctgctg 3660
ggcctttatt ctggggcaag aggaaaggat gggaattaag ggtagaaaga ggcaaaaatt 3720
tccagttgag cgggggccaa caaaaagttt ttttttttgg aaaaagtttt tttcttagaa 3780
caaggatggc aaaatgggtg caccagcaat aggaaagagt caaacgtgtg aacccttggg 3840
gtttgggaca ggcccatgag gccccagctc ccctagtata agccatacag gtccaaggga 3900
tcctcacagt gagagtggac ttagagcacg aagtcgtggc gctgcgatct gagtgcgacc 3960
aagagtctga tagggcctag atgcagggta gacaatctca gcgccacagg gcagtcctga 4020
cccactcttt ggcccctcag cgcacttatc ccactttgga aatgtgaatt gtggtgggca 4080

aaagttgggg caagaggacc cccaactggg aaactttttc ccctccaggt tagttgggga 4140
actagcaccc tcaggtaacc caccactggc gtaatttata tctgaaccca gaccagacgc 4200
tttgaatcag gcactaaact ccagaaatat atttatttgc taatatattt atccacaaat 4260
gtggtctggt cttgtggttt tgttctgtcg tggagctngt ccagctngca ngngngtaga 4320
gcaagcngtc catgcgttcg ttgtcgtaca tctaagagaa gtaaattatt tatgttatca 4380
gaggctaggc tccgattcat gaaatggata gggtagagta gaggggcttg gccaattaag 4440
aactggtttg taagccccta aaagtgtggc ttaagtgaag atcagggaaa ggaagaaagc 4500
catgaactgg aatccttaac tgtgccttca gtctattatt attatactgt tcacttcaca 4560
cattatccat acttcaggtg gactcagacc tggggcaaat actctgtggc ctcgcttttt 4620
cagtccataa aatgggccta cttaatagtt gttagcagga ctatacatga gataatagag 4680
tgtagaaaga tatgttccaa aagtggaaaa gttttattca agtgatagaa gaacatccaa 4740
acctgtcaca agaagcccat ctgaaacaca gcatgggacc gccaacaaga agaaagcccg 4800
cccggaagca gctcaatcaa ggaggctggg ctggaatgac agcgcagcgg ggcctgaaac 4860
tatttatatc ccaaagctcc tctcagataa acacaaatga ctgcgttctg cctgcactcg 4920
ggctattgcg aggacagaga gctggtgctc cattggcgtg aagtctccag gggccagaaa 4980
ggggcctttg tcgcttcctc acaaggcaca agttcccctt ctgcttcccc gagaaaggtt 5040
tgggtagggg gtgggtggtt tagtgcctat agaacaaggc atttcgcttc ctagacggtg 5100
aaatgaaagg gaaaaaaagg acacctaatc tcctacaaat ggtctttagt aaaggaaccg 5160
tgtctaagcg ctaagaactg cgcaaagtat aaattatcag ccggaacgag caaacagacg 5220
gagttttaaa agataaatac gcattttttt ccgccgtagc tcccaggcca gcattcctgt 5280
gggaagcaag tggaaaccct atagcgctct cgcagttagg aaggaggggt ggggctgtcc 5340
ctggatttct tctcggtctc tgcagagaca ataccagagg gagagcagtg gattcactgc 5400
ccccaatgct tctaaaacgg ggagacaaaa caaaaaaaaa caaacgttcg ggttaccatc 5460
ggggaacagg accgacgccc agggccacca gcccagatca aacagcccgc gtctcggcgc 5520
tgcggctcag cccgacacac tcccgcgcaa gcgcagccgc ccccccgccc cgggggcccg 5580
ctgactaccc cacacagcct ccgccgcgcc ctcggcgggc tcaggtggct gcgacgcgct 5640
ccggcccagg tggcggccgg ccgcccagcc tccccgcctg ctggcgggag aaaccatctc 5700
ctctggcggg ggtaggggcg gagctggcgt ccgcccacac cggaagagga agtctaagcg 5760
ccggaagtgg tgggcattct gggtaacgag ctatttactt cctgcgggtg cacaggctgt 5820
ggtcgtctat ctccctgttg ttcttcccat cggcgaagat ggccctggag acggtgccga 5880
aggacctgcg gcatctgcgg gcctgtttgc tgtgttcgct ggtcaaggtg tcagtcgggg 5940
acctggttgt agggcccatg ggggaccaag gtcggggaaa gagggcggaa tggggctcgt 6000
aggatcgcgg acaggtcttg cagctgaggg caggggcggt cttacatgcc tttgaatcct 6060
cagctcttag acgttcggtg aacttacgtt ggagccgaaa gacactggga gtcagaggcg 6120
ggtggggatc cgctgctgag tgagtagtcg gaaaggatgc ctgaccctga gtagactcac 6180
agaactgttt cttttcctgc ttcaggaatc gtgcgggagc tgaaaagtcg aggagtggcc 6240
tcactgggtc agcatgacga tcaagcgaga ttcagattga gtgtgtttca tcaagttctc 6300
tagctgcctg ggctgcctcc cttccctcgg ccccgagtgc agaacgtgga ggtgaacggg 6360
atgaatccaa gctggttcgc agggcagtcc tcactgagca gtctctttcc aactctcacc 6420
accttttcca gctggtcctg ggatgtgagg aatcctgttg ggggcaggag gctggcagga 6480
ggaaatagat agctctttgc cccttgtttc cagacaagat aaggggagaa ttctactaga 6540
gccattccta gccaccctgc cttctctgca ttttgggagg tgtgccctcg agccagctga 6600
gaagatacca tggctgcctg ggggctgggc aggatttgga acacctcgtg 6650

<210> 14
<211> 1206
<212> DNA

<213> Homo sapiens

<400> 14
gcagtgccag gacctctccc ggaggcgggg cagagcagca gcttctcggc cctgtgccga 60
gcccaggcct gcacccctaa ggcaggcact gctccgtgat ccaggaacca cctctctcta 120
cagctgggag tgagcagtca gagagggaga cagccttgcc cggtgctacc cagcaagcta 180
gtcaccgagt gggcagaggg aggagcggcc ctcaccggat gtcaagcagc ctgggtcccc 240
agtccagctc tgcctgtccc tcgcaataac gcctcagtga cgaccatttg tgagccatct 300
ctctgtctca ggcacggtgc tacatgccaa cgaaacctgc tcccattgaa ccctggccag 360
ccagtgaaga aagggttggg cctgggaggt gccactttac agacaggggc accaaggggc 420
agggtggcag gaggcccacc ggacgttccc catgaagtag cagtcccagc atccacaccc 480
agcaggcacc acgctggccc gcagcctccc tgccagcacg cctggcttcc cggcctcgga 540
acttgatctg ctccctcttc cggacactgg ggctcctgcc aagtcctggg ctgggcagca 600
actgctgaac attctaagaa atccctccca gggttttctc aggagcccgg gtggggcagg 660
aagtccccag gggctgaggg gaccgtggcg gcaggtggca cccagagcag cactctcctg 720
gggcccaggc tgttgggcca gaggcaggac tgtgaggcct agtgtagggc ctcctgccag 780
tggccggcac ctacttgtgg ggctgggggt tcccccagca ggttgggctc cccacctgac 840
acactcacag accttgtgcc ttggagagcc agtgttcccg gggccacata gctatgccgc 900
ccaggggctg ggcctgtccc agctctggtc ccccggcccc aggtcctgga cgctggtccg 960
cgcagcagca ggcggcctcc ggaggacacg atgtgactgg ctgccgctac gtcgcactca 1020
gatgagtctg cgccggatcg acctgctgcc gagtcctgcc ggacaggcac aggcagggag 1080
tgaaaattat ctaccccttt ttatttctta ataactgaat gaaaataaac attggtggtt 1140
tgacaaataa ctacatattt tcaaacccag ccagtccagg ggatgcagtt tccaggtgcg 1200
ttatgc 1206

<210> 15
<211> 1443
<212> DNA
<213> Homo sapiens

<400> 15
gccttttatc actgacccaa agcgaaaagc accaggttta actctgttcc ccctgtgcta 60
ggtccccaca ggttttgtta tcctgtatcc ttccttactc ctagcagcta ctctgatcga 120
ttttctctca ccctcagagc agacttgtgg ccttgtttgg ggaagcactg gaattttgaa 180
cccccagcct atttgggtca attgtttggc aagagtgtcc gcttcatgat gctggtgatg 240
gcatgcacct cgtcacatgt gcacggctag gcttgtgcag gtggcctcta ttacccaaac 300
actgaaggga agcccctctg tgtccttgga gagatgccag gtgcttagtt tacatttttg 360
cctgcttgga gagctaacag cttgaagtaa accaatccat cagggactcc tgaggttttc 420
accagccagc accacccaat cgtgcgtgaa gactttctga ctccctggac attgccatgg 480
actcaacctg tcacttcagg acctgttttt gaactaacaa agctagactt ctgattctct 540
cttgcctgca cctacctgta cattccgaac acatggtaga gactctacaa aatgcttaat 600
atgtgatcta tggacggttc cccctgaaat tataaatgct gccatcttca tccttctggt 660
tttcccaagc tattacccct atccatttgt ctgtggtata caacgtcact atccaggcct 720
ccgtctcgga actgtgtgaa gctctttggt ctagggacca aaggcaggaa ttatttagtg 780
atcagacaat aagaaaacac tgaaagagat gatttgcctt tgatggatgt aaaaatacta 840
aaaatttatt ttcaatttat ggtaatgcta cttagccatt ttctctcaaa caccactgga 900
gaatttatat aacatgaagc atatacaaaa tgcatctagg gggtaatgag gcttctcttt 960

catcaacttc tgccttttag gatttgcccc aatattgtac ttggaggtaa atattaaaac 1020
tccattgagg actggtataa agttgtaaag tgaacaaaac ccagtagaaa gctattgata 1080
aagaatctat tttataaaat aagttttata caataaaatc tactctgtaa ttaccttttc 1140
aaagtatatt tctaaaatag cttatatgcc cttctgtacc aaatttctta aataagggat 1200
tatgttcaca ctttctcagt cctccttcca gctcttcaac ctactatccc aataagggtc 1260
ataagactga ggcagtttca acagctcctg ctaaggttaa agaaagatac ggggaagcat 1320
catgaaagga taggactctc cctatctaat gtatgtttat acatacctta tatatggagg 1380
ctaataagtt tcctttaagt atatcaataa ttaagatctg tactaagtga ccactataag 1440
tgt 1443

<210> 16
<211> 1957
<212> DNA
<213> Homo sapiens

<400> 16
gcggccgccg agctccgcgc ggggcaaacc tcccggcgcg gccatgcggg gaggtaagtg 60
atctgcctgt gcgcccaggg cgtgggaagg cgcccgccct ctcctctctc caggatgaaa 120
ggaaacgaag aatgccgcaa tgaaaaccgc tctgccctcc caaaaacaca tcttggccgt 180
gtgtccggtg ctcctgcagc tcgttgcacc cacggacgtg ggctctcact gtggagtgga 240
gtgggggcag aagcgtgccc tgccccacgg agagccccgg ctcgcctggg gctgctggca 300
gtgctcgggg agcgggacgg ggtggtggca cgactcggcg gtgaccccga gaacgccaca 360
cctccaccct ccactttcca aagaccggct tccccgggga gcccccacac taaacgccag 420
cgaactgcct ctccgtgaaa gtcttagcca gaaactttcc ccgctttgtc gccagtgcca 480
cagagagtcg tgtggctctg ggccggcgct gctggtccaa gaggcagcct ggcgtcttct 540
gcccctaccg tccccttctc aggccagttc tcacttgccc ctgagacgcc attcccggct 600
cggtgaaaaa ggcactatat ccatccctgc atcgtctcca agactcattc cctctaaacc 660
ttcaagttcc atggaaaatg ggagaccacc tgatcctgca gactgggccg tgatggatgt 720
cgtcaattat ttccgaaccg tgggatttga ggagcaagct agtgcttttc aggaacagga 780
aattgatgga aaatccctgc tattgatgac aagaaatgat gtgttgacag gacttcagtt 840
aaaattgggg cctgctctga aaatctacga atatcatgta aaacctctgc agacaaagca 900
tttaaagaac aactcttcat agtacagtca aattggggtc ttcgacctca aaaaaaatac 960
ataatgacat aattcagttt catgtaatga aactttgtaa acagaataca tacatgtgta 1020
tatgtaaaga atttcaatca aatgaaacgt tatcctattg gatagactag gcaattcatc 1080
agctcacctg aaatcagcca ggaggagcaa ggacaagatg cgcacagggt ggttttcctc 1140
atggattttg tcaaatagat gatctttgac acgattagac actcctcccc acaaaggctt 1200
tgaaatcata aggattttcc tcatctcttt atagctttcc caaaatcttt taaaaaaaga 1260
atttaattaa atgacagtct tttggttaca gacttaggat gagtaaaaac aagaaaattt 1320
ggggaggggg agaaagaaga aagggattgc tgtctccctt gaattcctct gttccttaga 1380
gcttgtgtta cttggacgga attgccaaca ccctttttta tagagggttc tccacttgac 1440
cttattaagg ttttattggg atatgctgca gtgtttgaaa tgaacatgca tcatggcccc 1500
ttcaggagca gaatcatagc tctgaaaaga gaagctccgt tgtgtactga ggatatccat 1560
ccatattcag ctagctttca aatggggtgt aatgatattt tctgcataga ttttctttta 1620
aattggttct ttgtttctga agaaagaatt ttttttaact tcatggtttt atttataata 1680
atttgtttct gaagaaattt gccgagagtt acaggtcaaa aagccttgtt actagtacag 1740
aatattttta tatatattcc ttcatgatgg tgtaattttt tttaattgtc ctatgctttg 1800
ttcggttcct gggttaagta cttgttttta agagcttgga aaaagtgggc ttgctacatc 1860

tctgttcaaa gagacatttg ttcaatctct gtgtgtcaac gccttgttga attggtgctt 1920
tgtggtagca ataaagcatt gcttcagttt ataaaaa 1957

<210> 17
<211> 2074
<212> DNA
<213> Homo sapiens

<400> 17
tgcagctatt ttaggttctc taacttcatc gtagtttata gggtaagtaa agggaagggg 60
aaagtgattg gtgtggttgt ctcccataag aactgatttt tttctactga agcatgtata 120
aagtttatat atgacttttt atatttgttt aataaaaatt ttacaggaac taaatttgat 180
tatcaatatg aagtttttct ttaatttcag atttcaacta ttgcagaaag tgaagattca 240
caggagtcag tggatagtgt aactgattcc caaaagcgaa gggaaattct ttcaaggagg 300
ccttcctaca gggagaagtc tgaagaggag acttcagcac ctgccatcac cactgtaacg 360
gtgccaactc caatttacca aactagcagt ggacagtata ttgccattac ccagggagga 420
gcaatacagc tggctaacaa tggtaccgat ggggtacagg gcctgcaaac attaaccatg 480
accaatgcag cagccactca gccgggtact accattctac agtatgcaca gaccactgat 540
ggacagcaga tcttagtgcc cagcaaccaa gttgttgttc aaggtactca aaaattgtaa 600
agcaggatgt cagtgaattt gaattctgaa cgtcagtttg aagatggtaa catgtttagt 660
atataaatct tttccactca aaccatacat tttaattgat attaataatt aatatgaact 720
aattttataa agaccttcaa atttttttaa gtaacattag gttccttatt aggagagcat 780
attattacgc tgtttttaga agcagtttga caaatagtga ttgtgtttgt ttttacaaat 840
ggtgaatcag ttagaaaaat aaaacttcag tttatttagc cattatcatt tacattaaaa 900
caatatgttt ttcaaataat ataattggca tcaagtgata cactttttca tacttttagt 960
tttgttttaa ttcaaaattt ataatagttg accataatgc tttatcttct ttttcatttt 1020
gctcatttta tgaaaaatca tggtcgtttt ttatgtctgt ggcaagagtc tacttgatat 1080
ttgtttaata tgaattttac caatatcaaa ggtatagtac tactgaggaa ctatactcta 1140
tctaggtaag atcatccaat gtctgtgccc catctgtacc ttttagaccg taagcgtgcc 1200
tctggagacg tacaatacta taccagtatt cgctactagc taccctacta gctactattg 1260
gcccctggag ttgttatggc atcctcccct agctacttcc tacacagcct gtctgaagat 1320
agcagctacg tataagtaga gaggtccgtc taatgaagat acagggaagc tagttctaga 1380
gtgtcgtaga aagaagtaaa gaatatgtga aatgtttaga aaacagagtg gctagtgcgt 1440
tgaaaatcaa taactagaca ttgattgagg agcttaaagc acttaaggac ctttactgcc 1500
acaaatcaga ttaatttggg atttaaattt tcacctgtta aggtggaaaa tggactggct 1560
tggccacaac ctgaaagaca aaataaacat tttattttct aaacatttct ttttttctat 1620
gcgcaaaact gcctgaaagc aactacagaa tttcattcat ttgtgctttt gcattaaact 1680
gtgaatgttc cagcacctgc ctccacttct cccctcaaga cattttcaac gccaggaatc 1740
atgaagagac ttctgctttt caaccccacc ctcctcaaga agtaataatt tgtttacttg 1800
taaattgatg ggagacatga ggaaaagaaa atctttttaa aaatgatttc aaggtttgtg 1860
ctgagctcct tgattgcctt agggacagaa ttaccccagc ctcttgagct gaagtaatgt 1920
gtgggccgca tgcataaagt aagtaaggtg caatgaagaa gtgttgattg ccaaattgac 1980
atgttgtcac attctcattg tgaattatgt aaagttgtta agagacatac cctctaaaaa 2040
agaactttag catggtattg aggacttaga aatg 2074

<210> 18

<211> 933
<212> DNA
<213> Homo sapiens

<400> 18
atggcggagg ctgtactgag ggtcgcccgg cggcagctga gccagcgcgg cgagtcttcg 60
agctcccatc ctcctgcggc agatgttcga gcctgtgagc tgcaccttca cgtacctgct 120
gggtgacaga gagtcccggg acgccgttct gatcgaccca gtcctggaaa cagcgcctcg 180
ggatgtccag ctgatcaagg agctggggct gcggctgctc tatgctgtga atacccactg 240
ccacgcggaa ccacattaca ggcttggggc tgctccgttc cctcctccct ggctgccagt 300
ctgtcatctc ccgccttagt ggggcccagg ctgacttaca cattgaggat gggagactcc 360
atccgcttcg ggcgcttcgg tacagcccca ctcctggctg ctttcacggg ctggtgtgga 420
gtatctgtgg cttttccagg cacatggtgc aagctctcgg tggatctaac actctgggtt 480
ctggagggcg atggccctct tctcacagct ccactagggg cagtgcccca gtgggaactc 540
tctgcgttgg agaccagggc cagccctggc cacaccccag gctgtgtcac cttcgtcctg 600
aatgaccaca gcatggcctt cactggagat gccctgttga tccgtgggtg tgggcggaca 660
gacttccagc aaggctgtgc caagaccttg taccactcgg tccatgaaaa gatcttcaca 720
cttccaggag actgtctgat ctaccctgct cacgattacc atgggttcac agtgtccacc 780
gtggaggagg agaggactct gaaccctcgg ctcaccctca gctgtgagga gtttgtcaaa 840
atcatgggca acctgaactt gcctaaacct cagcagatag actttgctgt tccagccaac 900
atgcgctgtg gggtgcagac acccactgcc tga 933

<210> 19
<211> 525
<212> DNA
<213> Homo sapiens

<400> 19
gccatgggtt ccccttcagc ctgtccatac agagtgtgca ttccctggca ggggctcctg 60
ctcacagcct cgcttttaac cttctggaac ctgccaaaca gtgcccagac caatattgat 120
ggtgtgccgt tcaatgtcgc agaagggaag gaggtccttc tagtagtcca taatgagtcc 180
cagaatcttt atggctacaa ctggtacaaa gggcaaaggg tgcatgccaa ctatcgaatt 240
ataggatatg taaaaaatat aagtcaagaa aatgccccag ggcccgcaca caacggtcga 300
gagacaatat accccaatgg aaccctgctg atccagaacg tcacccacaa tgacgcagga 360
atctataccc tacacgttat aaaagaaaat cttgtgaatg aagaagtaac cagacaattc 420
tacgtattct atgagtcagt acaagcaagt tcacctgacc tctcagctgg gaccgctgtc 480
agcatcatga ttggagtact ggctgggatg gctctgatat agcag 525

<210> 20
<211> 377
<212> DNA
<213> Homo sapiens

<220>
<221> unsure
<222> (28)

<220>
<221> unsure
<222> (74)

<220>
<221> unsure
<222> (92)

<220>
<221> unsure
<222> (126)

<220>
<221> unsure
<222> (135)

<220>
<221> unsure
<222> (113)

<400> 20
ctcaaccaac atctgacatc tttcccgngg agcaacttcc tgctccacgg gaaagaggcc 60
gaaggattta cccntggacc cataagtctg ancatcctgc tgaagtcccc tcnccattgc 120
tccttnaagc caaanctaca ctttgctggt tcctgtcccc tctgagaaag gggatagaaa 180
gctccttcct ctatgtcctc ccatcgagat ctgttctggg gatggagctt ccaacttcct 240
cttgcagcag gaaagaatgc tgctcaccct tctgtcttgc agagtgggat tgtgggaggg 300
attggcagcc ttcttctcca ccacctgtcc agcttcttcc tggtcagggc tgggaccccc 360
aggaatatta tgttgcc 377

<210> 21
<211> 709
<212> DNA
<213> Homo sapiens

<400> 21
tctgaatgtt ttggtgaata aatctgttct tcagcaaccc tacctgcttc tccaaactgc 60
ctaaagagat ccagtactga tgacgctgtt cttccatctt tactccctgg aaactaacca 120
cgttgtcttc gtttccttca ccacgcacca ggagctcaga gatcaaagcg gctttccatc 180
ttgttctccc agccccagga cactgactct gtacaggatg gggccgtcct cttgccctcc 240
ttctcatcct aatccccctt ctccagctga tcaacccggg gagtactcag tgttccttag 300
actccgttat ggataagaag atcaaggatg ttctcaacag tctagagtac agtccctctc 360
ctataagcaa gaagctctcg tgtgctagtg tcaaaagcca aggcagaccg tcctcactgc 420
cctgctgggg atggctgtca ctggctgtgc ttgtggctat ggctgtggtt cgtgggatgt 480
tcagctggaa accacctgcc actgccagtg cagtgtggtg gactggacca ctgcccgctg 540
ctgccacctg acctgacagg gaggaaggct gagaactcag ttctgtgacc atgacagtaa 600
tgaaaccagg gtcccaacca agaaatctaa ctcaaacgtc ccacttcatt tgttccattc 660

ctgattcttg ggtaataaag acaaactttg tacctctcaa aaaaaaaa 709

<210> 22
<211> 3195
<212> DNA
<213> Homo sapiens

<400> 22
gccaggaata actagagagg aacaatgggg ttattcagag gttttgtttt cctcttagtt 60
ctgtgcctgc tgcaccagtc aaatacttcc ttcattaagc tgaataataa tggctttgaa 120
gatattgtca ttgttataga tcctagtgtg ccagaagatg aaaaaataat tgaacaaata 180
gaggatatgg tgactacagc ttctacgtac ctgtttgaag ccacagaaaa aagatttttt 240
ttcaaaaatg tatctatatt aattcctgag aattggaagg aaaatcctca gtacaaaagg 300
ccaaaacatg aaaaccataa acatgctgat gttatagttg caccacctac actcccaggt 360
agagatgaac catacaccaa gcagttcaca gaatgtggag agaaaggcga atacattcac 420
ttcacccctg accttctact tggaaaaaaa acaaaatgaa tatggaccac caggcaaact 480
gtttgtccat gagtgggctc acctccggtg gggagtgttt gatgagtaca atgaagatca 540
gcctttctac cgtgctaagt caaaaaaaat cgaagcaaca aggtgttccg caggtatctc 600
tggtagaaat agagtttata agtgtcaagg aggcagctgt cttagtagag catgcagaat 660
tgattctaca acaaaactgt atggaaaaga ttgtcaattc tttcctgata aagtacaaac 720
agaaaaagca tccataatgt ttatgcaaag tattgattct gttgttgaat tttgtaacga 780
aaaaacccat aatcaagaag ctccaagcct acaaaacata aagtgcaatt ttagaagtac 840
atgggaggtg attagcaatt ctgaggattt taaaaacacc atacccatgg tgacaccacc 900
tcctccacct gtcttctcat tgctgaagat cagtcaaaga attgtgtgct tagttcttga 960
taagtctgga agcatggggg gtaaggaccg cctaaatcga atgaatcaag cagcaaaaca 1020
tttcctgctg cagactgttg aaaatggatc ctgggtgggg atggttcact ttgatagtac 1080
tgccactatt gtaaataagc taatccaaat aaaaagcagt gatgaaagaa acacactcat 1140
ggcaggatta cctacatatc ctctgggagg aacttccatc tgctctggaa ttaaatatgc 1200
atttcaggtg attggagagc tacattccca actcgatgga tccgaagtac tgctgctgac 1260
tgatggggag gataacactg caagttcttg tattgatgaa gtgaaacaaa gtggggccat 1320
tgttcatttt attgctttgg gaagagctgc tgatgaagca gtaatagaga tgagcaagat 1380
aacaggagga agtcattttt atgtttcaga tgaagctcag aacaatggcc tcattgatgc 1440
ttttggggct cttacatcag gaaatactga tctctcccag aagtcccttc agctcgaaag 1500
taagggatta acactgaata gtaatgcctg gatgaacgac actgtcataa ttgatagtac 1560
agtgggaaag gacacgttct ttctcatcac atggaacagt ctgcctccca gtatttctct 1620
ctgggatccc agtggaacaa taatggaaaa tttcacagtg gatgcaactt ccaaaatggc 1680
ctatctcagt attccaggaa ctgcaaaggt gggcacttgg gcatacaatc ttcaagccaa 1740
agcgaaccca gaaacattaa ctattacagt aacttctcga gcagcaaatt cttctgtgcc 1800
tccaatcaca gtgaatgcta aaatgaataa ggacgtaaac agtttcccca gcccaatgat 1860
tgtttacgca gaaattctac aaggatatgt acctgttctt ggagccaatg tgactgcttt 1920
cattgaatca cagaatggac atacagaagt tttggaactt ttggataatg gtgcaggcgc 1980
tgattctttc aagaatgatg gagtctactc caggtatttt acagcatata cagaaaatgg 2040
cagatatact taaaagttcg ggctcatgga ggagcaaaca ctgccaggct aaaattacgg 2100
cctccactga atagagccgc gtacatacca ggctgggtag tgaacgggga aattgaagca 2160
aacccgccaa gacctgaaat tgatgaggat actcagacca ccttggagga tttcagccga 2220
acagcatccg gaggtgcatt tgtggtatca caagtcccaa gccttccctt gcctgaccaa 2280
tacccaccaa gtcaaatcac agaccttgat gccacagttc atgaggataa gattattctt 2340

acatggacag caccaggaga taattttgat gttggaaaag ttcaacgtta tatcataaga 2400
ataagtgcaa gtattcttga tctaagagac agttttgatg atgctcttca agtaaatact 2460
actgatctgt caccaaagga ggccaactcc aaggaaagct ttgcatttaa accagaaaat 2520
atctcagaag aaaatgcaac ccacatattt attgccatta aaagtataga taaaagcaat 2580
ttgacatcaa aagtatccaa cattgcacaa gtaactttgt ttatccctca agcaaatcct 2640
gatgacattg atcctacacc tactcctact cctactccta ctcctgataa aagtcataat 2700
tctggagtta atatttctac gctggtattg tctgtgattg ggtctgttgt aattgttaac 2760
tttattttaa gtaccaccat ttgaacctta acgaagaaaa aatcttcaag tagacctaga 2820
agagagtttt aaaaaaacaa aacaatgtaa gtaaaggata tttctgaatc ttaaaattca 2880
tcccatgtgt gatcataaac tcataaaaat aattttaaga tgtcggaaaa ggatactttg 2940
attaaataaa aacactcatg gatatgtaaa aactgtcaag attaaaattt aatagtttca 3000
tttatttgtt attttatttg taagaaatag tgatgaacaa agatcctttt tcatactgat 3060
acctggttgt atattatttg atgcaacagt tttctgaaat gatatttcaa attgcatcaa 3120
gaaattaaaa tcatctatct gagtagtcaa aatacaagta aaggagagca aataaacaac 3180
atttggaaaa aaatg 3195

<210> 23
<211> 22
<212> DNA
<213> Artificial Sequence

<220>
<223> Description of Artificial Sequence: Synthetic

<400> 23
tggaaataga ttcaggggtc at 22

<210> 24
<211> 21
<212> DNA
<213> Artificial Sequence

<220>
<223> Description of Artificial Sequence: Synthetic

<400> 24
cgggtgtacc tcactgactt c 21

<210> 25
<211> 25
<212> DNA
<213> Artificial Sequence

<220>
<223> Description of Artificial Sequence: Synthetic

<400> 25
tgtcttccga gagaaccagg ctccg
25

## Description

### FIELD OF THE INVENTION

This invention relates, in part, to newly identified polynucleotides and polypeptides; variants and derivatives of the polynucleotides and polypeptides; processes for making the polynucleotides and the polypeptides, and their variants and derivatives; agonists and antagonists of the polypeptides; and uses of the polynucleotides, polypeptides, variants, derivatives, agonists and antagonists for detecting, diagnosing, monitoring, staging, prognosticating, imaging and treating cancers, particularly colon cancer. In particular, in these and in other regards, the invention relates to colon specific polynucleotides and polypeptides hereinafter referred to as colon specific genes or "CSGs".

### BACKGROUND OF THE INVENTION

Cancer of the colon is a highly treatable and often curable disease when localized to the bowel. It is one of the most frequently diagnosed malignancies in the United States as well as the second most common cause of cancer death. Surgery is the primary treatment and results in cure in approximately 50% of patients. However, recurrence following surgery is a major problem and often is the ultimate cause of death.

The prognosis of colon cancer is clearly related to the degree of penetration of the tumor through the bowel wall and the presence or absence of nodal involvement. These two characteristics form the basis for all staging systems developed for this disease. Treatment decisions are usually made in reference to the older Duke's or the Modified Astler-Coller (MAC) classification scheme for staging.

Bowel obstruction and bowel perforation are indicators of poor prognosis in patients with colon cancer. Elevated pretreatment serum levels of carcinoembryonic antigen (CEA) and of carbohydrate antigen 19-9 (CA 19-9) also have a negative prognostic significance.

Age greater than 70 years at presentation is not a contraindication to standard therapies. Acceptable morbidity and mortality, as well as long-term survival, are achieved in this patient population.

Because of the frequency of the disease (approximately 160,000 new cases of colon and rectal cancer per year), the identification of high-risk groups, the demonstrated slow growth of primary lesions, the better survival of early-stage lesions, and the relative simplicity and accuracy of screening tests, screening for colon cancer should be a part of routine care for all adults starting at age 50, especially those with first-degree relatives with colorectal cancer.

Procedures used for detecting, diagnosing, monitoring, staging, and prognosticating colon cancer are of critical importance to the outcome of the patient. For example, patients diagnosed with early colon cancer generally have a much greater five-year survival rate as compared to the survival rate for patients diagnosed with distant metastasized colon cancer. New diagnostic methods which are more sensitive and specific for detecting early colon cancer are clearly needed.

Colon cancer patients are closely monitored following initial therapy and during adjuvant therapy to determine response to therapy and to detect persistent or recurrent disease of metastasis. There is clearly a need for a colon cancer marker which is more sensitive and specific in detecting colon cancer, its recurrence, and progression.

Another important step in managing colon cancer is to determine the stage of the patient's disease. Stage determination has potential prognostic value and provides criteria for designing optimal therapy. Generally, pathological staging of colon cancer is preferable over clinical staging because the former gives a more accurate prognosis. However, clinical staging would be preferred were it at least as accurate as pathological staging because it does not depend on an invasive procedure to obtain tissue for pathological evaluation. Staging of colon cancer would be improved by detecting new markers in cells, tissues, or bodily fluids which could differentiate between different stages of invasion.

Accordingly, there is a great need for more sensitive and accurate methods for the staging of colon cancer in a human to determine whether or not such cancer has metastasized and for monitoring the progress of colon cancer in a human which has not metastasized for the onset of metastasis.

In the present invention, methods are provided for detecting, diagnosing, monitoring, staging, prognosticating, imaging and treating colon cancer via colon specific genes referred to herein as CSGs. For purposes of the present invention, CSG refers, among other things, to native protein expressed by the gene comprising a polynucleotide sequence of SEQ ID NO:1, 2, 3, 4, 5, 6, 7, 8, 9, 10, 11, 12, 13, 14, 15, 16, 17, 18, 19, 20, 21 or 22. By "CSG" it is also meant herein polynucleotides which, due to degeneracy in genetic coding, comprise variations in nucleotide sequence as compared to SEQ ID NO: 1, 2, 3, 4, 5, 6, 7, 8, 9, 10, 11, 12, 13, 14, 15, 16, 17, 18, 19, 20, 21 or 22 but which still encode the same protein. In the alternative, what is meant by CSG as used herein, means the native mRNA encoded by the gene comprising the polynucleotide sequence of SEQ ID NO: 1, 2, 3, 4, 5, 6, 7, 8, 9, 10, 11, 12, 13, 14, 15, 16, 17, 18, 19, 20, 21 or 22, levels of the gene comprising the polynucleotide sequence of SEQ ID NO: 1, 2, 3, 4, 5, 6, 7, 8, 9, 10, 11, 12, 13, 14, 15, 16, 17, 18, 19, 20, 21, or 22, or levels of a polynucleotide which is capable of hybridizing under stringent conditions to the antisense sequence of SEQ ID NO: 1, 2, 3, 4, 5, 6, 7, 8, 9, 10, 11, 12, 13, 14, 15, 16, 17, 18, 19, 20, 21 or 22.

Other objects, features, advantages and aspects of the present invention will become apparent to those of skill in the art from the following description. It should be understood, however, that the following description and the specific examples, while indicating preferred embodiments of the invention are given by way of illustration only. Various changes and modifications within the spirit and scope of the disclosed invention will become readily apparent to those skilled in the art from reading the following description and from reading the other parts of the present disclosure.

### SUMMARY OF THE INVENTION

Toward these ends, and others, it is an object of the present invention to provide CSGs comprising a polynucleotide of SEQ ID NO: 1, 2, 3, 4, 5, 6, 7, 8, 9, 10, 11, 12, 13, 14, 15, 16, 17, 18, 19, 20, 21 or 22, a protein expressed by a polynucleotide of SEQ ID NO: 1, 2, 3, 4, 5, 6, 7, 8, 9, 10, 11, 12, 13, 14, 15, 16, 17, 18, 19, 20, 21 or 22 or a variant thereof which expresses the protein; or a polynucleotide which is capable of hybridizing under stringent conditions to the antisense sequence of SEQ ID NO:1, 2, 3, 4, 5, 6, 7, 8, 9, 10, 11, 12, 13, 14, 15, 16, 17, 18, 19, 20, 21 or 22.

It is another object of the present invention to provide a method for diagnosing the presence of colon cancer by analyzing for changes in levels of CSG in cells, tissues or bodily fluids compared with levels of CSG in preferably the same cells, tissues, or bodily fluid type of a normal human control, wherein a change in levels of CSG in the patient versus the normal human control is associated with colon cancer.

Further provided is a method of diagnosing metastatic colon cancer in a patient having colon cancer which is not known to have metastasized by identifying a human patient suspected of having colon cancer that has metastasized; analyzing a sample of cells, tissues, or bodily fluid from such patient for CSG; comparing the CSG levels in such cells, tissues, or bodily fluid with levels of CSG in preferably the same cells, tissues, or bodily fluid type of a normal human control, wherein an increase in CSG levels in the patient versus the normal human control is associated with colon cancer which has metastasized.

Also provided by the invention is a method of staging colon cancer in a human which has such cancer by identifying a human patient having such cancer; analyzing a sample of cells, tissues, or bodily fluid from such patient for CSG; comparing CSG levels in such cells, tissues, or bodily fluid with levels of CSG in preferably the same cells, tissues, or bodily fluid type of a normal human control sample, wherein an increase in CSG levels in the patient versus the normal human control is associated with a cancer which is progressing and a decrease in the levels of CSG is associated with a cancer which is regressing or in remission.

Further provided is a method of monitoring colon cancer in a human having such cancer for the onset of metastasis. The method comprises identifying a human patient having such cancer that is not known to have metastasized; periodically analyzing a sample of cells, tissues, or bodily fluid from such patient for CSG; comparing the CSG levels in such cells, tissue, or bodily fluid with levels of CSG in preferably the same cells, tissues, or bodily fluid type of a normal human control sample, wherein an increase in CSG levels in the patient versus the normal human control is associated with a cancer which has metastasized.

Further provided is a method of monitoring the change in stage of colon cancer in a human having such cancer by looking at levels of CSG in a human having such cancer. The method comprises identifying a human patient having such cancer; periodically analyzing a sample of cells, tissues, or bodily fluid from such patient for CSG; comparing the CSG levels in such cells, tissue, or bodily fluid with levels of CSG in preferably the same cells, tissues, or bodily fluid type of a normal human control sample, wherein an increase in CSG levels in the patient versus the normal human control is associated with a cancer which is progressing and a decrease in the levels of CSG is associated with a cancer which is regressing or in remission.

Further provided are methods of designing new therapeutic agents targeted to a CSG for use in imaging and treating colon cancer. For example, in one embodiment, therapeutic agents such as antibodies targeted against CSG or fragments of such antibodies can be used to treat, detect or image localization of CSG in a patient for the purpose of detecting or diagnosing a disease or condition. In this embodiment, an increase in the amount of labeled antibody detected as compared to normal tissue would be indicative of tumor metastases or growth. Such antibodies can be polyclonal, monoclonal, or omniclonal or prepared by molecular biology techniques. The term "antibody", as used herein and throughout the instant specification is also meant to include aptamers and single-stranded oligonucleotides such as those derived from an in vitro evolution protocol referred to as SELEX and well known to those skilled in the art. Antibodies can be labeled with a variety of detectable and therapeutic labels including, but not limited to, radioisotopes and paramagnetic metals. Therapeutic agents such as small molecules and antibodies which decrease the concentration and/or activity of CSG can also be used in the treatment of diseases characterized by overexpression of CSG. Such agents can be readily identified in accordance with teachings herein.

Other objects, features, advantages and aspects of the present invention will become apparent to those of skill in the art from the following description. It should be understood, however, that the following description and the specific examples, while indicating preferred embodiments of the invention, are given by way of illustration only. Various changes and modifications within the spirit and scope of the disclosed invention will become readily apparent to those skilled in the art from reading the following description and from reading the other parts of the present disclosure.

### GLOSSARY

The following illustrative explanations are provided to facilitate understanding of certain terms used frequently herein, particularly in the examples. The explanations are provided as a convenience and are not limitative of the invention.

ISOLATED means altered "by the hand of man" from its natural state; i.e., that, if it occurs in nature, it has been changed or removed from its original environment, or both.

For example, a naturally occurring polynucleotide or a polypeptide naturally present in a living animal in its natural state is not "isolated," but the same polynucleotide or polypeptide separated from the coexisting materials of its natural state is "isolated", as the term is employed herein. For example, with respect to polynucleotides, the term isolated means that it is separated from the chromosome and cell in which it naturally occurs.

As part of or following isolation, such polynucleotides can be joined to other polynucleotides, such as DNAs, for mutagenesis, to form fusion proteins, and for propagation or expression in a host, for instance. The isolated polynucleotides, alone or joined to other polynucleotides such as vectors, can be introduced into host cells, in culture or in whole organisms. When introduced into host cells in culture or in whole organisms, such DNAs still would be isolated, as the term is used herein, because they would not be in their naturally occurring form or environment. Similarly, the polynucleotides and polypeptides may occur in a composition, such as media formulations, solutions for introduction of polynucleotides or polypeptides, for example, into cells, compositions or solutions for chemical or enzymatic reactions, for instance, which are not naturally occurring compositions, and, therein remain isolated polynucleotides or polypeptides within the meaning of that term as it is employed herein.

OLIGONUCLEOTIDE(S) refers to relatively short polynucleotides. Often the term refers to single-stranded deoxyribonucleotides, but it can refer as well to single-or double-stranded ribonucleotides, RNA:DNA hybrids and double-stranded DNAs, among others.

Oligonucleotides, such as single-stranded DNA probe oligonucleotides, often are synthesized by chemical methods, such as those implemented on automated oligonucleotide synthesizers. However, oligonucleotides can be made by a variety of other methods, including *in vitro* recombinant DNA-mediated techniques and by expression of DNAs in cells and organisms.

Initially, chemically synthesized DNAs typically are obtained without a 5' phosphate. The 5' ends of such oligonucleotides are not substrates for phosphodiester bond formation by ligation reactions that employ DNA ligases typically used to form recombinant DNA molecules. Where ligation of such oligonucleotides is desired, a phosphate can be added by standard techniques, such as those that employ a kinase and ATP.

The 3' end of a chemically synthesized oligonucleotide generally has a free hydroxyl group and, in the presence of a ligase such as T4 DNA ligase, readily will form a phosphodiester bond with a 5' phosphate of another polynucleotide, such as another oligonucleotide. As is well known, this reaction can be prevented selectively, where desired, by removing the 5' phosphates of the other polynucleotide(s) prior to ligation.

POLYNUCLEOTIDE(S) generally refers to any polyribonucleotide or polydeoxribonucleotide and is inclusive of unmodified RNA or DNA as well as modified RNA or DNA. Thus, for instance, polynucleotides as used herein refers to, among other things, single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, polynucleotide, as used herein, refers to triple-stranded regions comprising RNA or DNA or both RNA and DNA. The strands in such regions may be from the same molecule or from different molecules. The regions may include all of one or more of the molecules, but more typically involve only a region of some of the molecules. One of the molecules of a triple-helical region often is an oligonucleotide.

As used herein, the term polynucleotide is also inclusive of DNAs or RNAs as described above that contain one or more modified bases. Thus, DNAs or RNAs with backbones modified for stability or for other reasons are "polynucleotides" as that term is intended herein. Moreover, DNAs or RNAs comprising unusual bases, such as inosine, or modified bases, such as tritylated bases, to name just two examples, are polynucleotides as the term is used herein.

It will be appreciated that a great variety of modifications have been made to DNA and RNA that serve many useful purposes known to those of skill in the art. The term polynucleotide as it is employed herein embraces such chemically, enzymatically or metabolically modified forms of polynucleotides, as well as chemical forms of DNA and RNA characteristic of viruses and cells, including simple and complex cells, inter alia.

POLYPEPTIDES, as used herein, includes all polypeptides as described below. The basic structure of polypeptides is well known and has been described in innumerable textbooks and other publications in the art. In this context, the term is used herein to refer to any peptide or protein comprising two or more amino acids joined to each other in a linear chain by peptide bonds. As used herein, the term refers to both short chains, which also commonly are referred to in the art as peptides, oligopeptides and oligomers, for example, and to longer chains, which generally are referred to in the art as proteins, of which there are many types. It will be appreciated that polypeptides often contain amino acids other than the 20 amino acids commonly referred to as the 20 naturally occurring amino acids, and that many amino acids, including the terminal amino acids, may be modified in a given polypeptide, either by natural processes such as processing and other post-translational modifications, or by chemical modification techniques which are well known to the art. Even the common modifications that occur naturally in polypeptides are too numerous to list exhaustively here, but they are well described in basic texts and in more detailed monographs, as well as in a voluminous research literature, and they are well known to those of skill in the art.

Modifications which may be present in polypeptides of the present invention include, to name an illustrative few, acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination.

Such modifications are well known to those of skill and have been described in great detail in the scientific literature. Several particularly common modifications including, but not limited to, glycosylation, lipid attachment, sulfation, gamma-carboxylation of glutamic acid residues, hydroxylation and ADP-ribosylation are described in most basic texts, such as, for instance PROTEINS STRUCTURE AND MOLECULAR PROPERTIES, 2nd Ed., T. E. Creighton, W. H. Freeman and Company, New York (1993). Many detailed reviews are available on this subject, such as, for example, those provided by Wold, F., Posttranslational Protein Modifications: Perspectives and Prospects, pgs. 1-12 in POSTTRANSLATIONAL COVALENT MODIFICATION OF PROTEINS, B. C. Johnson, Ed., Academic Press, New York (1983); Seifter et al., Analysis for protein modifications and nonprotein cofactors, Meth. Enzymol. 182: 626-646 (1990) and Rattan et al., Protein Synthesis: Posttranslational Modifications and Aging, Ann. N.Y. Acad. Sci. 663: 48-62 (1992).

It will be appreciated that the polypeptides of the present invention are not always entirely linear. Instead, polypeptides may be branched as a result of ubiquitination, and they may be circular, with or without branching, generally as a result of posttranslation events including natural processing event and events brought about by human manipulation which do not occur naturally. Circular, branched and branched circular polypeptides may be synthesized by non-translation natural processes and by entirely synthetic methods, as well.

Modifications can occur anywhere in a polypeptide, including the peptide backbone, the amino acid side-chains and the amino or carboxyl termini. In fact, blockage of the amino and/or carboxyl group in a polypeptide by a covalent modification is common in naturally occurring and synthetic polypeptides and such modifications may be present in polypeptides of the present invention, as well. For instance, the amino terminal residue of polypeptides made in *E. coli*, prior to proteolytic processing, almost invariably will be N-formylmethionine.

The modifications that occur in a polypeptide often will be a function of how it is made. For polypeptides made by expressing a cloned gene in a host, for instance, the nature and extent of the modifications, in large part, will be determined by the host cell posttranslational modification capacity and the modification signals present in the polypeptide amino acid sequence. For instance, as is well known, glycosylation often does not occur in bacterial hosts such as *E. coli*. Accordingly, when glycosylation is desired, a polypeptide can be expressed in a glycosylating host, generally a eukaryotic cell. Insect cells often carry out the same posttranslational glycosylations as mammalian cells. Thus, insect cell expression systems have been developed to express efficiently mammalian proteins having native patterns of glycosylation, inter alia. Similar considerations apply to other modifications.

It will be appreciated that the same type of modification may be present in the same or varying degrees at several sites in a given polypeptide. Also, a given polypeptide may contain many types of modifications.

In general, as used herein, the term polypeptide encompasses all such modifications, particularly those that are present in polypeptides synthesized by expressing a polynucleotide in a host cell.

VARIANT(S) of polynucleotides or polypeptides, as the term is used herein, are polynucleotides or polypeptides that differ from a reference polynucleotide or polypeptide, respectively.

With respect to variant polynucleotides, differences are generally limited so that the nucleotide sequences of the reference and the variant are closely similar overall and, in many regions, identical. Thus, changes in the nucleotide sequence of the variant may be silent. That is, they may not alter the amino acids encoded by the polynucleotide. Where alterations are limited to silent changes of this type a variant will encode a polypeptide with the same amino acid sequence as the reference. Alternatively, changes in the nucleotide sequence of the variant may alter the amino acid sequence of a polypeptide encoded by the reference polynucleotide. Such nucleotide changes may result in amino acid substitutions, additions, deletions, fusions and truncations in the polypeptide encoded by the reference sequence.

With respect to variant polypeptides, differences are generally limited so that the sequences of the reference and the variant are closely similar overall and, in many region, identical. For example, a variant and reference polypeptide may differ in amino acid sequence by one or more substitutions, additions, deletions, fusions and truncations, which may be present in any combination.

RECEPTOR MOLECULE, as used herein, refers to molecules which bind or interact specifically with CSG polypeptides of the present invention and is inclusive not only of classic receptors, which are preferred, but also other molecules that specifically bind to or interact with polypeptides of the invention (which also may be referred to as "binding molecules" and "interaction molecules," respectively and as "CSG binding or interaction molecules". Binding between polypeptides of the invention and such molecules, including receptor or binding or interaction molecules may be exclusive to polypeptides of the invention, which is very highly preferred, or it may be highly specific for polypeptides of the invention, which is highly preferred, or it may be highly specific to a group of proteins that includes polypeptides of the invention, which is preferred, or it may be specific to several groups of proteins at least one of which includes polypeptides of the invention.

Receptors also may be non-naturally occurring, such as antibodies and antibody-derived reagents that bind to polypeptides of the invention.

### DETAILED DESCRIPTION OF THE INVENTION

The present invention relates to novel colon specific polypeptides and polynucleotides, referred to herein as CSGs, among other things, as described in greater detail below.

### Polynucleotides

In accordance with one aspect of the present invention, there are provided isolated CSG polynucleotides which encode CSG polypeptides.

Using the information provided herein, such as the polynucleotide sequences set out in SEQ ID NO:1, 2, 3, 4, 5, 6, 7, 8, 9, 10, 11, 12, 13, 14, 15, 16, 17, 18, 19, 20, 21, and 22, a polynucleotide of the present invention encoding a CSG may be obtained using standard cloning and screening procedures, such as those for cloning cDNAs using mRNA from cells of a human tumor as starting material.

Polynucleotides of the present invention may be in the form of RNA, such as mRNA, or in the form of DNA, including, for instance, cDNA and genomic DNA obtained by cloning or produced by chemical synthetic techniques or by a combination thereof. The DNA may be double-stranded or single-stranded. Single-stranded DNA may be the coding strand, also known as the sense strand, or it may be the non-coding strand, also referred to as the anti-sense strand.

The coding sequence which encodes the polypeptides may be identical to the coding sequence of the polynucleotides of SEQ ID NO:1, 2, 3, 4, 5, 6, 7, 8, 9, 10, 11, 12, 13, 14, 15, 16, 17, 18, 19, 20, 21 or 22. It also may be a polynucleotide with a different sequence, which, as a result of the redundancy (degeneracy) of the genetic code, encodes the same polypeptides as encoded by SEQ ID NO:1, 2, 3, 4, 5, 6, 7, 8, 9, 10, 11, 12, 13, 14, 15, 16, 17, 18, 19, 20, 21 or 22.

Polynucleotides of the present invention, such as SEQ ID NO: 1, 2, 3, 4, 5, 6, 7, 8, 9, 10, 11, 12, 13, 14, 15, 16, 17, 18, 19, 20, 21 or 22, which encode these polypeptides may comprise the coding sequence for the mature polypeptide by itself. Polynucleotides of the present invention may also comprise the coding sequence for the mature polypeptide and additional coding sequences such as those encoding a leader or secretory sequence such as a pre-, or pro- or preproprotein sequence. Polynucleotides of the present invention may also comprise the coding sequence of the mature polypeptide, with or without the aforementioned additional coding sequences, together with additional, non-coding sequences. Examples of additional non-coding sequences which may be incorporated into the polynucleotide of the present invention include, but are not limited to, introns and non-coding 5' and 3' sequences such as transcribed, non-translated sequences that play a role in transcription, mRNA processing including, for example, splicing and polyadenylation signals, ribosome binding and stability of mRNA, and additional coding sequence which codes for amino acids such as those which provide additional functionalities. Thus, for instance, the polypeptide may be fused to a marker sequence such as a peptide which facilitates purification of the fused polypeptide. In certain preferred embodiments of this aspect of the invention, the marker sequence is a hexa-histidine peptide, such as the tag provided in the pQE vector (Qiagen, Inc.), among others, many of which are commercially available. As described in Gentz et al. (Proc. Natl. Acad. Sci., USA 86: 821-824 (1989)), for instance, hexa-histidine provides for convenient purification of the fusion protein. The HA tag corresponds to an epitope derived of influenza hemagglutinin protein (Wilson et al., Cell 37: 767 (1984)).

In accordance with the foregoing, the term "polynucleotide encoding a polypeptide" as used herein encompasses polynucleotides which include a sequence encoding a polypeptide of the present invention, particularly SEQ ID NO:1, 2, 3, 4, 5, 6, 7, 8, 9, 10, 11, 12, 13, 14, 15, 16, 17, 18, 19, 20, 21 or 22. The term encompasses polynucleotides that include a single continuous region or discontinuous regions encoding the polypeptide (for example, interrupted by introns) together with additional regions, that also may contain coding and/or non-coding sequences.

The present invention further relates to variants of the herein above described polynucleotides which encode for fragments, analogs and derivatives of the CSG polypeptides. A variant of the polynucleotide may be a naturally occurring variant such as a naturally occurring allelic variant, or it may be a variant that is not known to occur naturally. Such non-naturally occurring variants of the polynucleotide may be made by mutagenesis techniques, including those applied to polynucleotides, cells or organisms.

Among variants in this regard are variants that differ from the aforementioned polynucleotides by nucleotide substitutions, deletions or additions. The substitutions, deletions or additions may involve one or more nucleotides. The variants may be altered in coding or non-coding regions or both. Alterations in the coding regions may produce conservative or non-conservative amino acid substitutions, deletions or additions.

Among the particularly preferred embodiments of the invention in this regard are polynucleotides encoding polypeptides having the same amino acid sequence encoded by a CSG polynucleotide comprising SEQ ID NO: 1, 2, 3, 4, 5, 6, 7, 8, 9, 10, 11, 12, 13, 14, 15, 16, 17, 18, 19, 20, 21, or 22; variants, analogs, derivatives and fragments thereof, and fragments of the variants, analogs and derivatives. Further particularly preferred in this regard are CSG polynucleotides encoding polypeptide variants, analogs, derivatives and fragments, and variants, analogs and derivatives of the fragments, in which several, a few, 5 to 10, 1 to 5, 1 to 3, 2, 1 or no amino acid residues are substituted, deleted or added, in any combination. Especially preferred among these are silent substitutions, additions and deletions, which do not alter the properties and activities of the CSG. Also especially preferred in this regard are conservative substitutions. Most highly preferred are polynucleotides encoding polypeptides having the amino acid sequences as polypeptides encoded by SEQ ID NO: 1, 2, 3, 4, 5, 6, 7, 8, 9, 10, 11, 12, 13, 14, 15, 16, 17, 18, 19, 20, 21 or 22, without substitutions.

Further preferred embodiments of the invention are CSG polynucleotides that are at least 70% identical to a polynucleotide of SEQ ID NO: 1, 2, 3, 4, 5, 6, 7, 8, 9, 10, 11, 12, 13, 14, 15, 16, 17, 18, 19, 20, 21 or 22, and polynucleotides which are complementary to such polynucleotides. More preferred are CSG polynucleotides that comprise a region that is at least 80% identical to a polynucleotide of SEQ ID NO: 1, 2, 3, 4, 5, 6, 7, 8, 9, 10, 11, 12, 13, 14, 15, 16, 17, 18, 19, 20, 21, or 22. In this regard, CSG polynucleotides at least 90% identical to the same are particularly preferred, and among these particularly preferred CSG polynucleotides, those with at least 95% are especially preferred. Furthermore, those with at least 97% are highly preferred among those with at least 95%, and among these those with at least 98% and at least 99% are particularly highly preferred, with at least 99% being the most preferred.

Particularly preferred embodiments in this respect, moreover, are polynucleotides which encode polypeptides which retain substantially the same biological function or activity as the mature polypeptides encoded by a polynucleotide of SEQ ID NO: 1, 2, 3, 4, 5, 6, 7, 8, 9, 10, 11, 12, 13, 14, 15, 16, 17, 18, 19, 20, 21 or 22.

The present invention further relates to polynucleotides that hybridize to the herein above-described CSG sequences. In this regard, the present invention especially relates to polynucleotides which hybridize under stringent conditions to the herein above-described polynucleotides. As herein used, the term "stringent conditions" means hybridization will occur only if there is at least 95% and preferably at least 97% identity between the sequences.

As discussed additionally herein regarding polynucleotide assays of the invention, for instance, polynucleotides of the invention as described herein, may be used as a hybridization probe for cDNA and genomic DNA to isolate full-length cDNAs and genomic clones encoding CSGs and to isolate cDNA and genomic clones of other genes that have a high sequence similarity to these CSGs. Such probes generally will comprise at least 15 bases. Preferably, such probes will have at least 30 bases and may have at least 50 bases.

For example, the coding region of CSG of the present invention may be isolated by screening using an oligonucleotide probe synthesized from the known DNA sequence. A labeled oligonucleotide having a sequence complementary to that of a gene of the present invention is used to screen a library of human cDNA, genomic DNA or mRNA to determine which members of the library the probe hybridizes with.

The polynucleotides and polypeptides of the present invention may be employed as research reagents and materials for discovery of treatments and diagnostics to human disease, as further discussed herein relating to polynucleotide assays, inter alia.

The polynucleotides may encode a polypeptide which is the mature protein plus additional amino or carboxyl-terminal amino acids, or amino acids interior to the mature polypeptide (when the mature form has more than one polypeptide chain, for instance). Such sequences may play a role in processing of a protein from precursor to a mature form, may facilitate/protein trafficking, may prolong or shorten protein half-life or may facilitate manipulation of a protein for assay or production, among other things. As generally is the case *in situ,* the additional amino acids may be processed away from the mature protein by cellular enzymes.

A precursor protein having the mature form of the polypeptide fused to one or more prosequences may be an inactive form of the polypeptide. When prosequences are removed, such inactive precursors generally are activated. Some or all of the prosequences may be removed before activation. Generally, such precursors are called proproteins.

In sum, a polynucleotide of the present invention may encode a mature protein, a mature protein plus a leader sequence (which may be referred to as a preprotein), a precursor of a mature protein having one or more prosequences which are not the leader sequences of a preprotein, or a preproprotein, which is a precursor to a proprotein, having a leader sequence and one or more prosequences, which generally are removed during processing steps that produce active and mature forms of the polypeptide.

### Polypeptides

The present invention further relates to CSG polypeptides, preferably polypeptides encoded by a polynucleotide of SEQ ID NO: 1, 2, 3, 4, 5, 6, 7, 8, 9, 10, 11, 12, 13, 14, 15, 16, 17, 18, 19, 20, 21, or 22. The invention also relates to fragments, analogs and derivatives of these polypeptides. The terms "fragment," "derivative" and "analog" when referring to the polypeptides of the present invention means a polypeptide which retains essentially the same biological function or activity as such polypeptides. Thus, an analog includes a proprotein which can be activated by cleavage of the proprotein portion to produce an active mature polypeptide.

The polypeptide of the present invention may be a recombinant polypeptide, a natural polypeptide or a synthetic polypeptide. In certain preferred embodiments it is a recombinant polypeptide.

The fragment, derivative or analog of a polypeptide of or the present invention may be (I) one in which one or more of the amino acid residues are substituted with a conserved or non-conserved amino acid residue (preferably a conserved amino acid residue) and such substituted amino acid residue may or may not be one encoded by the genetic code; (ii) one in which one or more of the amino acid residues includes a substituent group; (iii) one in which the mature polypeptide is fused with another compound, such as a compound to increase the half-life of the polypeptide (for example, polyethylene glycol); or (iv) one in which the additional amino acids are fused to the mature polypeptide, such as a leader or secretory sequence or a sequence which is employed for purification of the mature polypeptide or a proprotein sequence. Such fragments, derivatives and analogs are deemed to be within the scope of those skilled in the art from the teachings herein.

Among preferred variants are those that vary from a reference by conservative amino acid substitutions. Such substitutions are those that substitute a given amino acid in a polypeptide by another amino acid of like characteristics. Typically seen as conservative substitutions are the replacements, one for another, among the aliphatic amino acids Ala, Val, Leu and Ile; interchange of the hydroxyl residues Ser and Thr, exchange of the acidic residues Asp and Glu, substitution between the amide residues Asn and Gln, exchange of the basic residues Lys and Arg and replacements among the aromatic residues Phe, Tyr.

The polypeptides and polynucleotides of the present invention are preferably provided in an isolated form, and preferably are purified to homogeneity.

The polypeptides of the present invention include the polypeptide encoded by the polynucleotide of SEQ ID NO: 1, 2, 3, 4, 5, 6, 7, 8, 9, 10, 11, 12, 13, 14, 15, 16, 17, 18, 19, 20, 21 or 22 (in particular the mature polypeptide) as well as polypeptides which have at least 75% similarity (preferably at least 75% identity), more preferably at least 90% similarity (more preferably at least 90% identity), still more preferably at least 95% similarity (still more preferably at least 95% identity), to a polypeptide encoded by SEQ ID NO: 1, 2, 3, 4, 5, 6, 7, 8, 9, 10, 11, 12, 13, 14, 15, 16, 17, 18, 19, 20, 21, or 22. Also included are portions of such polypeptides generally containing at least 30 amino acids and more preferably at least 50 amino acids.

As known in the art "similarity" between two polypeptides is determined by comparing the amino acid sequence and its conserved amino acid substitutes of one polypeptide sequence with that of a second polypeptide.

Fragments or portions of the polypeptides of the present invention may be employed for producing the corresponding full-length polypeptide by peptide synthesis; therefore, the fragments may be employed as intermediates for producing the full-length polypeptides. Fragments or portions of the polynucleotides of the present invention may be used to synthesize full-length polynucleotides of the present invention.

### Fragments

Also among preferred embodiments of this aspect of the present invention are polypeptides comprising fragments of a polypeptide encoded by a polynucleotide of SEQ ID NO: 1, 2, 3, 4, 5, 6, 7, 8, 9, 10, 11, 12, 13, 14, 15, 16, 17, 18, 19, 20, 21 or 22. In this regard a fragment is a polypeptide having an amino acid sequence that entirely is the same as part but not all of the amino acid sequence of the aforementioned CSG polypeptides and variants or derivatives thereof.

Such fragments may be "free-standing," i.e., not part of or fused to other amino acids or polypeptides, or they may be contained within a larger polypeptide of which they form a part or region. When contained within a larger polypeptide, the presently discussed fragments most preferably form a single continuous region. However, several fragments may be comprised within a single larger polypeptide. For instance, certain preferred embodiments relate to a fragment of a CSG polypeptide of the present comprised within a precursor polypeptide designed for expression in a host and having heterologous pre- and pro-polypeptide regions fused to the amino terminus of the CSG fragment and an additional region fused to the carboxyl terminus of the fragment. Therefore, fragments in one aspect of the meaning intended herein, refers to the portion or portions of a fusion polypeptide or fusion protein derived from a CSG polypeptide.

As representative examples of polypeptide fragments of the invention, there may be mentioned those which have from about 15 to about 139 amino acids. In this context "about" includes the particularly recited range and ranges larger or smaller by several, a few, 5, 4, 3, 2 or 1 amino acid at either extreme or at both extremes. Highly preferred in this regard are the recited ranges plus or minus as many as 5 amino acids at either or at both extremes. Particularly highly preferred are the recited ranges plus or minus as many as 3 amino acids at either or at both the recited extremes. Especially preferred are ranges plus or minus 1 amino acid at either or at both extremes or the recited ranges with no additions or deletions. Most highly preferred of all in this regard are fragments from about 15 to about 45 amino acids.

Among especially preferred fragments of the invention are truncation mutants of the CSG polypeptides. Truncation mutants include CSG polypeptides having an amino acid sequence encoded by a polynucleotide of SEQ ID NO: 1, 2, 3, 4, 5, 6, 7, 8, 9, 10, 11, 12, 13, 14, 15, 16, 17, 18, 19, 20, 21 or 22, or variants or derivatives thereof, except for deletion of a continuous series of residues (that is, a continuous region, part or portion) that includes the amino terminus, or a continuous series of residues that includes the carboxyl terminus or, as in double truncation mutants, deletion of two continuous series of residues, one including the amino terminus and one including the carboxyl terminus. Fragments having the size ranges set out herein also are preferred embodiments of truncation fragments, which are especially preferred among fragments generally.

Also preferred in this aspect of the invention are fragments characterized by structural or functional attributes of the CSG polypeptides of the present invention. Preferred embodiments of the invention in this regard include fragments that comprise alpha-helix and alpha-helix forming regions ("alpha-regions"), beta-sheet and beta-sheet-forming regions ("beta-regions"), turn and turn-forming regions ("turn-regions"), coil and coil-forming regions ("coil-regions"), hydrophilic regions, hydrophobic regions, alpha amphipathic regions, beta amphipathic regions, flexible regions, surface-forming regions and high antigenic index regions of the CSG polypeptides of the present invention. Regions of the aforementioned types are identified routinely by analysis of the amino acid sequences encoded by the polynucleotides of SEQ ID NO: 1, 2, 3, 4, 5, 6, 7, 8, 9, 10, 11, 12, 13, 14, 15, 16, 17, 18, 19, 20, 21 or 22. Preferred regions include Garnier-Robson alpha-regions, beta-regions, turn-regions and coil-regions, Chou-Fasman alpha-regions, beta-regions and turn-regions, Kyte-Doolittle hydrophilic regions and hydrophilic regions, Eisenberg alpha and beta amphipathic regions, Karplus-Schulz flexible regions, Emini surface-forming regions and Jameson-Wolf high antigenic index regions. Among highly preferred fragments in this regard are those that comprise regions of CSGs that combine several structural features, such as several of the features set out above. In this regard, the regions defined by selected residues of a CSG polypeptide which all are characterized by amino acid compositions highly characteristic of turn-regions, hydrophilic regions, flexible-regions, surface-forming regions, and high antigenic index-regions, are especially highly preferred regions. Such regions may be comprised within a larger polypeptide or may be by themselves a preferred fragment of the present invention, as discussed above. It will be appreciated that the term "about" as used in this paragraph has the meaning set out above regarding fragments in general.

Further preferred regions are those that mediate activities of CSG polypeptides. Most highly preferred in this regard are fragments that have a chemical, biological or other activity of a CSG polypeptide, including those with a similar activity or an improved activity, or with a decreased undesirable activity. Highly preferred in this regard are fragments that contain regions that are homologs in sequence, or in position, or in both sequence and to active regions of related polypeptides, and which include colon specific-binding proteins. Among particularly preferred fragments in .these regards are truncation mutants, as discussed above.

It will be appreciated that the invention also relates to polynucleotides encoding the aforementioned fragments, polynucleotides that hybridize to polynucleotides encoding the fragments, particularly those that hybridize under stringent conditions, and polynucleotides such as PCR primers for amplifying polynucleotides that encode the fragments. In these regards, preferred polynucleotides are those that correspond to the preferred fragments, as discussed above.

### Fusion Proteins

In one embodiment of the present invention, the CSG polypeptides of the present invention are preferably fused to other proteins. These fusion proteins can be used for a variety of applications. For example, fusion of the present polypeptides to His-tag, HA-tag, protein A, IgG domains, and maltose binding protein facilitates purification. (See also EP A 394,827; Traunecker, et al., Nature 331: 84-86 (1988).) Similarly, fusion to IgG-1, IgG-3, and albumin increases the halflife time *in vivo.* Nuclear localization signals fused to the polypeptides of the present invention can target the protein to a specific subcellular localization, while covalent heterodimer or homodimers can increase or decrease the activity of a fusion protein. Fusion proteins can also create chimeric molecules having more than one function. Finally, fusion proteins can increase solubility and/or stability of the fused protein compared to the non-fused protein. All of these types of fusion proteins described above can be made in accordance with well known protocols.

For example, a CSG polypeptide can be fused to an IgG molecule via the following protocol. Briefly, the human Fc portion of the IgG molecule is PCR amplified using primers that span the 5' and 3' ends of the sequence. These primers also have convenient restriction enzyme sites that facilitate cloning into an expression vector, preferably a mammalian expression vector. For example, if pC4 (Accession No. 209646) is used, the human Fc portion can be ligated into the BamHI cloning site. In this protocol, the 3' BamHI site must be destroyed. Next, the vector containing the human Fc portion is re-restricted with BamHI thereby linearizing the vector, and a CSG polynucleotide of the present invention is ligated into this BamHI site. It is preferred that the polynucleotide is cloned without a stop codon, otherwise a fusion protein will not be produced.

If the naturally occurring signal sequence is used to produce the secreted protein, pC4 does not need a second signal peptide. Alternatively, if the naturally occurring signal sequence is not used, the vector can be modified to include a heterologous signal sequence. (See, e. g., WO 96/34891.)

### Diagnostic Assays

The present invention also relates to diagnostic assays and methods, both quantitative and qualitative for detecting, diagnosing, monitoring, staging and prognosticating cancers by comparing levels of CSG in a human patient with those of CSG in a normal human control. For purposes of the present invention, what is meant by CSG levels is, among other things, native protein expressed by a gene comprising the polynucleotide sequence of SEQ ID NO: 1, 2, 3, 4, 5, 6, 7, 8, 9, 10, 11, 12, 13, 14, 15, 16, 17, 18, 19, 20, 21 or 22. By "CSG" it is also meant herein polynucleotides which, due to degeneracy in genetic coding, comprise variations in nucleotide sequence as compared to SEQ ID NO: 1, 2, 3, 4, 5, 6, 7, 8, 9, 10, 11, 12, 13, 14, 15, 16, 17, 18, 19, 20, 21 or 22 but which still encode the same protein. The native protein being detected may be whole, a breakdown product, a complex of molecules or chemically modified. In the alternative, what is meant by CSG as used herein, means the native mRNA encoded by a polynucleotide sequence of SEQ ID NO: 1, 2, 3, 4, 5, 6, 7, 8, 9, 10, 11, 12, 13, 14, 15, 16, 17, 18, 19, 20, 21 or 22, levels of the gene comprising the polynucleotide sequence of SEQ ID NO: 1, 2, 3, 4, 5, 6, 7, 8, 9, 10, 11, 12, 13, 14, 15, 16, 17, 18, 19, 20, 21, or 22, or levels of a polynucleotide which is capable of hybridizing under stringent conditions to the antisense sequence of SEQ ID NO: 1, 2, 3, 4, 5, 6, 7, 8, 9, 10, 11, 12, 13, 14, 15, 16, 17, 18, 19, 20, 21, or 22. Such levels are preferably determined in at least one of cells, tissues and/or bodily fluids, including determination of normal and abnormal levels. Thus, for instance, a diagnostic assay in accordance with the invention for diagnosing overexpression of CSG protein compared to normal control bodily fluids, cells, or tissue samples may be used to diagnose the presence of colon cancer.

All the methods of the present invention may optionally include determining the levels of other cancer markers as well as CSG. Other cancer markers, in addition to CSG, useful in the present invention will depend on the cancer being tested and are known to those of skill in the art.

The present invention provides methods for diagnosing the presence of colon cancer by analyzing for changes in levels of CSG in cells, tissues or bodily fluids compared with levels of CSG in cells, tissues or bodily fluids of preferably the same type from a normal human control, wherein an increase in levels of CSG in the patient versus the normal human control is associated with the presence of colon cancer.

Without limiting the instant invention, typically, for a quantitative diagnostic assay a positive result indicating the patient being tested has cancer is one in which cells, tissues or bodily fluid levels of the cancer marker, such as CSG, are at least two times higher, and most preferably are at least five times higher, than in preferably the same cells, tissues or bodily fluid of a normal human control.

The present invention also provides a method of diagnosing metastatic colon cancer in a patient having colon cancer which has not yet metastasized for the onset of metastasis. In the method of the present invention, a human cancer patient suspected of having colon cancer which may have metastasized (but which was not previously known to have metastasized) is identified. This is accomplished by a variety of means known to those of skill in the art.

In the present invention, determining the presence of CSG levels in cells, tissues or bodily fluid, is particularly useful for discriminating between colon cancer which has not metastasized and colon cancer which has metastasized. Existing techniques have difficulty discriminating between colon cancer which has metastasized and colon cancer which has not metastasized and proper treatment selection is often dependent upon such knowledge.

In the present invention, the cancer marker levels measured in such cells, tissues or bodily fluid is CSG, and are compared with levels of CSG in preferably the same cells, tissue or bodily fluid type of a normal human control. That is, if the cancer marker being observed is just CSG in serum, this level is preferably compared with the level of CSG in serum of a normal human control. An increase in the CSG in the patient versus the normal human control is associated with colon cancer which has metastasized.

Without limiting the instant invention, typically, for a quantitative diagnostic assay a positive result indicating the cancer in the patient being tested or monitored has metastasized is one in which cells, tissues or bodily fluid levels of the cancer marker, such as CSG, are at least two times higher, and most preferably are at least five times higher, than in preferably the same cells, tissues or bodily fluid of a normal patient.

Normal human control as used herein includes a human patient without cancer and/or non cancerous samples from the patient; in the methods for diagnosing or monitoring for metastasis, normal human control may preferably also include samples from a human patient that is determined by reliable methods to have colon cancer which has not metastasized.

### Staging

The invention also provides a method of staging colon cancer in a human patient. The method comprises identifying a human patient having such cancer and analyzing cells, tissues or bodily fluid from such human patient for CSG. The CSG levels determined in the patient are then compared with levels of CSG in preferably the same cells, tissues or bodily fluid type of a normal human control, wherein an increase in CSG levels in the human patient versus the normal human control is associated with a cancer which is progressing and a decrease in the levels of CSG (but still increased over true normal levels) is associated with a cancer which is regressing or in remission.

### Monitoring

Further provided is a method of monitoring colon cancer in a human patient having such cancer for the onset of metastasis. The method comprises identifying a human patient having such cancer that is not known to have metastasized; periodically analyzing cells, tissues or bodily fluid from such human patient for CSG; and comparing the CSG levels determined in the human patient with levels of CSG in preferably the same cells, tissues or bodily fluid type of a normal human control, wherein an increase in CSG levels in the human patient versus the normal human control is associated with a cancer which has metastasized. In this method, normal human control samples may also include prior patient samples.

Further provided by this invention is a method of monitoring the change in stage of colon cancer in a human patient having such cancer. The method comprises identifying a human patient having such cancer; periodically analyzing cells, tissues or bodily fluid from such human patient for CSG; and comparing the CSG levels determined in the human patient with levels of CSG in preferably the same cells, tissues or bodily fluid type of a normal human control, wherein an increase in CSG levels in the human patient versus the normal human control is associated with a cancer which is progressing in stage and a decrease in the levels of CSG is associated with a cancer which is regressing in stage or in remission. In this method, normal human control samples may also include prior patient samples.

Monitoring a patient for onset of metastasis is periodic and preferably done on a quarterly basis. However, this may be done more or less frequently depending on the cancer, the particular patient, and the stage of the cancer.

### Prognostic Testing and Clinical Trial Monitoring

The methods described herein can further be utilized as prognostic assays to identify subjects having or at risk of developing a disease or disorder associated with increased levels of CSG. The present invention provides a method in which a test sample is obtained from a human patient and CSG is detected. The presence of higher CSG levels as compared to normal human controls is diagnostic for the human patient being at risk for developing cancer, particularly colon cancer.

The effectiveness of therapeutic agents to decrease expression or activity of the CSGs of the invention can also be monitored by analyzing levels of expression of the CSGs in a human patient in clinical trials or in in vitro screening assays such as in human cells. In this way, the gene expression pattern can serve as a marker, indicative of the physiological response of the human patient, or cells as the case may be, to the agent being tested.

### Detection of genetic lesions or mutations

The methods of the present invention can also be used to detect genetic lesions or mutations in CSG, thereby determining if a human with the genetic lesion is at risk for colon cancer or has colon cancer. Genetic lesions can be detected, for example, by ascertaining the existence of a deletion and/or addition and/or substitution of one or more nucleotides from the CSGs of this invention, a chromosomal rearrangement of CSG, aberrant modification of CSG (such as of the methylation pattern of the genomic DNA), the presence of a non-wild type splicing pattern of a mRNA transcript of CSG, allelic loss of CSG, and/or inappropriate post-translational modification of CSG protein. Methods to detect such lesions in the CSG of this invention are known to those of skill in the art.

For example, in one embodiment, alterations in a gene corresponding to a CSG polynucleotide of the present invention are determined via isolation of RNA from entire families or individual patients presenting with a phenotype of interest (such as a disease) is be isolated. cDNA is then generated from these RNA samples using protocols known in the art. See, e.g. Sambrook et al. (MOLECULAR CLONING: A LABORATORY MANUAL, 2nd Ed., Cold Spring Harbor Laboratory Press, Cold Spring Harbor, N.Y. (1989)), which is illustrative of the many laboratory manuals that detail these techniques. The cDNA is then used as a template for PCR, employing primers surrounding regions of interest in SEQ ID NO: 1, 2, 3, 4, 5, 6, 7, 8, 9, 10, 11, 12, 13, 14, 15, 16, 17, 18, 19, 20, 21 or 22. PCR conditions typically consist of 35 cycles at 95°C for 30 seconds; 60-120 seconds at 52-58°C; and 60-120 seconds at 70°C, using buffer solutions described in Sidransky, D., et al., Science 252: 706 (1991). PCR products are sequenced using primers labeled at their 5' end with T4 polynucleotide kinase, employing SequiTherm Polymerase (Epicentre Technologies). The intron-exon borders of selected exons are also determined and genomic PCR products analyzed to confirm the results. PCR products harboring suspected mutations are then cloned and sequenced to validate the results of the direct sequencing. PCR products are cloned into T-tailed vectors as described in Holton, T. A. and Graham, M. W., Nucleic Acids Research, 19 : 1156 (1991) and sequenced with T7 polymerase (United States Biochemical). Affected individuals are identified by mutations not present in unaffected individuals.

Genomic rearrangements can also be observed as a method of determining alterations in a gene corresponding to a polynucleotide. In this method, genomic clones are nick-translated with digoxigenin deoxy-uridine 5'triphosphate (Boehringer Manheim), and FISH is performed as described in Johnson, C. et al., Methods Cell Biol. 35: 73-99 (1991). Hybridization with a labeled probe is carried out using a vast excess of human DNA for specific hybridization to the corresponding genomic locus. Chromosomes are counterstained with 4,6-diamino-2-phenylidole and propidium iodide, producing a combination of C-and R-bands. Aligned images for precise mapping are obtained using a triple-band filter set (Chroma Technology, Brattleboro, VT) in combination with a cooled charge-coupled device camera (Photometrics, Tucson, AZ) and variable excitation wavelength filters (Johnson et al., Genet. Anal. Tech. Appl., 8: 75 (1991)). Image collection, analysis and chromosomal fractional length measurements are performed using the ISee Graphical Program System (Inovision Corporation, Durham, NC). Chromosome alterations of the genomic region hybridized by the probe are identified as insertions, deletions, and translocations. These alterations are used as a diagnostic marker for an associated disease. ***Assay Techniques***

Assay techniques that can be used to determine levels of gene expression (including protein levels), such as CSG of the present invention, in a sample derived from a patient are well known to those of skill in the art. Such assay methods include, without limitation, radioimmunoassays, reverse transcriptase PCR (RT-PCR) assays, immunohistochemistry assays, *in situ* hybridization assays, competitive-binding assays, Western Blot analyses, ELISA assays and proteomic approaches: two-dimensional gel electrophoresis (2D electrophoresis) and non-gel based approaches such as mass spectrometry or protein interaction profiling. Among these, ELISAs are frequently preferred to diagnose a gene's expressed protein in biological fluids.

An ELISA assay initially comprises preparing an antibody, if not readily available from a commercial source, specific to CSG, preferably a monoclonal antibody. In addition a reporter antibody generally is prepared which binds specifically to CSG. The reporter antibody is attached to a detectable reagent such as radioactive, fluorescent or enzymatic reagent, for example horseradish peroxidase enzyme or alkaline phosphatase.

To carry out the ELISA, antibody specific to CSG is incubated on a solid support, e.g. a polystyrene dish, that binds the antibody. Any free protein binding sites on the dish are then covered by incubating with a non-specific protein such as bovine serum albumin. Next, the sample to be analyzed is incubated in the dish, during which time CSG binds to the specific antibody attached to the polystyrene dish. Unbound sample is washed out with buffer. A reporter antibody specifically directed to CSG and linked to a detectable reagent such as horseradish peroxidase is placed in the dish resulting in binding of the reporter antibody to any monoclonal antibody bound to CSG. Unattached reporter antibody is then washed out. Reagents for peroxidase activity, including a colorimetric substrate are then added to the dish. Immobilized peroxidase, linked to CSG antibodies, produces a colored reaction product. The amount of color developed in a given time period is proportional to the amount of CSG protein present in the sample. Quantitative results typically are obtained by reference to a standard curve.

A competition assay can also be employed wherein antibodies specific to CSG are attached to a solid support and labeled CSG and a sample derived from the host are passed over the solid support. The amount of label detected which is attached to the solid support can be correlated to a quantity of CSG in the sample.

Using all or a portion of a nucleic acid sequence of CSG of the present invention as a hybridization probe, nucleic acid methods can also be used to detect CSG mRNA as a marker for colon cancer. Polymerase chain reaction (PCR) and other nucleic acid methods, such as ligase chain reaction (LCR) and nucleic acid sequence based amplification (NASBA), can be used to detect malignant cells for diagnosis and monitoring of various malignancies. For example, reverse-transcriptase PCR (RT-PCR) is a powerful technique which can be used to detect the presence of a specific mRNA population in a complex mixture of thousands of other mRNA species. In RT-PCR, an mRNA species is first reverse transcribed to complementary DNA (cDNA) with use of the enzyme reverse transcriptase; the cDNA is then amplified as in a standard PCR reaction. RT-PCR can thus reveal by amplification the presence of a single species of mRNA. Accordingly, if the mRNA is highly specific for the cell that produces it, RT-PCR can be used to identify the presence of a specific type of cell.

Hybridization to clones or oligonucleotides arrayed on a solid support (i.e. gridding) can be used to both detect the expression of and quantitate the level of expression of that gene. In this approach, a cDNA encoding the CSG gene is fixed to a substrate. The substrate may be of any suitable type including but not limited to glass, nitrocellulose, nylon or plastic. At least a portion of the DNA encoding the CSG gene is attached to the substrate and then incubated with the analyte, which may be RNA or a complementary DNA (cDNA) copy of the RNA, isolated from the tissue of interest. Hybridization between the substrate bound DNA and the analyte can be detected and quantitated by several means including but not limited to radioactive labeling or fluorescence labeling of the analyte or a secondary molecule designed to detect the hybrid. Quantitation of the level of gene expression can be done by comparison of the intensity of the signal from the analyte compared with that determined from known standards. The standards can be obtained by in vitro transcription of the target gene, quantitating the yield, and then using that material to generate a standard curve.

Of the proteomic approaches, 2D electrophoresis is a technique well known to those in the art. Isolation of individual proteins from a sample such as serum is accomplished using sequential separation of proteins by different characteristics usually on polyacrylamide gels. First, proteins are separated by size using an electric current. The current acts uniformly on all proteins, so smaller proteins move farther on the gel than larger proteins. The second dimension applies a current perpendicular to the first and separates proteins not on the basis of size but on the specific electric charge carried by each protein. Since no two proteins with different sequences are identical on the basis of both size and charge, the result of a 2D separation is a square gel in which each protein occupies a unique spot. Analysis of the spots with chemical or antibody probes, or subsequent protein microsequencing can reveal the relative abundance of a given protein and the identity of the proteins in the sample.

The above tests can be carried out on samples derived from a variety of cells, bodily fluids and/or tissue extracts such as homogenates or solubilized tissue obtained from a patient. Tissue extracts are obtained routinely from tissue biopsy and autopsy material. Bodily fluids useful in the present invention include blood, urine, saliva or any other bodily secretion or derivative thereof. By blood it is meant to include whole blood, plasma, serum or any derivative of blood.

### In Vivo Targeting of CSG/Colon Cancer Therapy

Identification of this CSG is also useful in the rational design of new therapeutics for imaging and treating cancers, and in particular colon cancer. For example, in one embodiment, antibodies which specifically bind to CSG can be raised and used in vivo in patients suspected of suffering from colon cancer. Antibodies which specifically bind CSG can be injected into a patient suspected of having colon cancer for diagnostic and/or therapeutic purposes. Thus, another aspect of the present invention provides for a method for preventing the onset and treatment of colon cancer in a human patient in need of such treatment by administering to the patient an effective amount of antibody. By "effective amount" it is meant the amount or concentration of antibody needed to bind to the target antigens expressed on the tumor to cause tumor shrinkage for surgical removal, or disappearance of the tumor. The binding of the antibody to the overexpressed CSG is believed to cause the death of the cancer cell expressing such CSG. The preparation and use of antibodies for *in vivo* diagnosis and treatment is well known in the art. For example, antibody-chelators labeled with Indium-111 have been described for use in the radioimmunoscintographic imaging of carcinoembryonic antigen expressing tumors (Sumerdon et al. Nucl. Med. Biol. 1990 17:247-254). In particular, these antibody-chelators have been used in detecting tumors in patients suspected of having recurrent colorectal cancer (Griffin et al. J. Clin. Onc. 1991 9:631-640). Antibodies with paramagnetic ions as labels.for use in magnetic resonance imaging have also been described (Lauffer, R.B. Magnetic Resonance in Medicine 1991 22:339-342). Antibodies directed against CSG can be used in a similar manner. Labeled antibodies which specifically bind CSG can be injected into patients suspected of having colon cancer for the purpose of diagnosing or staging of the disease status of the patient. The label used will be selected in accordance with the imaging modality to be used. For example, radioactive labels such as Indium-111, Technetium-99m or Iodine-131 can be used for planar scans or single photon emission computed tomography (SPECT). Positron emitting labels such as Fluorine-19 can be used in positron emission tomography. Paramagnetic ions such as Gadlinium (III) or Manganese (II) can be used in magnetic resonance imaging (MRI). Presence of the label, as compared to imaging of normal tissue, permits determination of the spread of the cancer. The amount of label within an organ or tissue also allows determination of the presence or absence of cancer in that organ or tissue.

Antibodies which can be used in in vivo methods include polyclonal, monoclonal and omniclonal antibodies and antibodies prepared via molecular biology techniques. Antibody fragments and aptamers and single-stranded oligonucleotides such as those derived from an *in vitro* evolution protocol referred to as SELEX and well known to those skilled in the art can also be used.

### Screening Assays

The present invention also provides methods for identifying modulators which bind to CSG protein or have a modulatory effect on the expression or activity of CSG protein. Modulators which decrease the expression or activity of CSG protein are believed to be useful in treating colon cancer. Such screening assays are known to those of skill in the art and include, without limitation, cell-based assays and cell free assays.

Small molecules predicted via computer imaging to specifically bind to regions of CSG can also be designed, synthesized and tested for use in the imaging and treatment of colon cancer. Further, libraries of molecules can be screened for potential anticancer agents by assessing the ability of the molecule to bind to the CSGs identified herein. Molecules identified in the library as being capable of binding to CSG are key candidates for further evaluation for use in the treatment of colon cancer. In a preferred embodiment, these molecules will downregulate expression and/or activity of CSG in cells.

### Adoptive Immunotherapy and Vaccines

Adoptive immunotherapy of cancer refers to a therapeutic approach in which immune cells with an antitumor reactivity are administered to a tumor-bearing host, with the aim that the cells mediate either directly or indirectly, the regression of an established tumor. Transfusion of lymphocytes, particularly T lymphocytes, falls into this category and investigators at the National Cancer Institute (NCI) have used autologous reinfusion of peripheral blood lymphocytes or tumor-infiltrating lymphocytes (TIL), T cell cultures from biopsies of subcutaneous lymph nodules, to treat several human cancers (Rosenberg, S. A., U.S. Patent No. 4,690,914, issued Sep. 1, 1987; Rosenberg, S. A., et al., 1988, N. England J. Med. 319:1676-1680).

The present invention relates to compositions and methods of adoptive immunotherapy for the prevention and/or treatment of primary and metastatic colon cancer in humans using macrophages sensitized to the antigenic CSG molecules, with or without non-covalent complexes of heat shock protein (hsp). Antigenicity or immunogenicity of the CSG is readily confirmed by the ability of the CSG protein or a fragment thereof to raise antibodies or educate naive effector cells, which in turn lyse target cells expressing the antigen (or epitope).

Cancer cells are, by definition, abnormal and contain proteins which should be recognized by the immune system as foreign since they are not present in normal tissues. However, the immune system often seems to ignore this abnormality and fails to attack tumors. The foreign CSG proteins that are produced by the cancer cells can be used to reveal their presence. The CSG is broken into short fragments, called tumor antigens, which are displayed on the surface of the cell. These tumor antigens are held or presented on the cell surface by molecules called MHC, of which there are two types: class I and II. Tumor antigens in association with MHC class I molecules are recognized by cytotoxic T cells while antigen-MHC class II complexes are recognized by a second subset of T cells called helper cells. These cells secrete cytokines which slow or stop tumor growth and help another type of white blood cell, B cells, to make antibodies against the tumor cells.

In adoptive immunotherapy, T cells or other antigen presenting cells (APCs) are stimulated outside the body (ex vivo), using the tumor specific CSG antigen. The stimulated cells are then reinfused into the patient where they attack the cancerous cells. Research has shown that using both cytotoxic and helper T cells is far more effective than using either subset alone. Additionally, the CSG antigen may be complexed with heat shock proteins to stimulate the APCs as described in U.S. Patent No. 5,985,270.

The APCs can be selected from among those antigen presenting cells known in the art including, but not limited to, macrophages, dendritic cells, B lymphocytes, and a combination thereof, and are preferably macrophages. In a preferred use, wherein cells are autologous to the individual, autologous immune cells such as lymphocytes, macrophages or other APCs are used to circumvent the issue of whom to select as the donor of the immune cells for adoptive transfer. Another problem circumvented by use of autologous immune cells is graft versus host disease which can be fatal if unsuccessfully treated.

In adoptive immunotherapy with gene therapy, DNA of the CSG can be introduced into effector cells similarly as in conventional gene therapy. This can enhance the cytotoxicity of the effector cells to tumor cells as they have been manipulated to produce the antigenic protein resulting in improvement of the adoptive immunotherapy.

CSG antigens of this invention are also useful as components of colon cancer vaccines. The vaccine comprises an immunogenically stimulatory amount of a CSG antigen. Immunogenically stimulatory amount refers to that amount of antigen that is able to invoke the desired immune response in the recipient for the amelioration, or treatment of colon cancer. Effective amounts may be determined empirically by standard procedures well known to those skilled in the art.

The CSG antigen may be provided in any one of a number of vaccine formulations which are designed to induce the desired type of immune response, e.g., antibody and/or cell mediated. Such formulations are known in the art and include, but are not limited to, formulations such as those described in U.S. Patent 5,585,103. Vaccine formulations of the present invention used to stimulate immune responses can also include pharmaceutically acceptable adjuvants.

### Vectors, host cells, expression

The present invention also relates to vectors which include polynucleotides of the present invention, host cells which are genetically engineered with vectors of the invention and the production of polypeptides of the invention by recombinant techniques.

Host cells can be genetically engineered to incorporate CSG polynucleotides and express CSG polypeptides of the present invention. For instance, CSG polynucleotides may be introduced into host cells using well known techniques of infection, transduction, transfection, transvection and transformation. The CSG polynucleotides may be introduced alone or with other polynucleotides. Such other polynucleotides may be introduced independently, co-introduced or introduced joined to the CSG polynucleotides of the invention.

For example, CSG polynucleotides of the invention may be transfected into host cells with another, separate, polynucleotide encoding a selectable marker, using standard techniques for co-transfection and selection in, for instance, mammalian cells. In this case, the polynucleotides generally will be stably incorporated into the host cell genome.

Alternatively, the CSG polynucleotide may be joined to a vector containing a selectable marker for propagation in a host. The vector construct may be introduced into host cells by the aforementioned techniques. Generally, a plasmid vector is introduced as DNA in a precipitate, such as a calcium phosphate precipitate, or in a complex with a charged lipid. Electroporation also may be used to introduce CSG polynucleotides into a host. If the vector is a virus, it may be packaged *in vitro* or introduced into a packaging cell and the packaged virus may be transduced into cells. A wide variety of well known techniques conducted routinely by those of skill in the art are suitable for making CSG polynucleotides and for introducing CSG polynucleotides into cells in accordance with this aspect of the invention. Such techniques are reviewed at length in reference texts such as Sambrook et al., previously cited herein.

Vectors which may be used in the present invention include, for example, plasmid vectors, single- or double-stranded phage vectors, and single- or double-stranded RNA or DNA viral vectors. Such vectors may be introduced into cells as polynucleotides, preferably DNA, by well known techniques for introducing DNA and RNA into cells. The vectors, in the case of phage and viral vectors, also may be and preferably are introduced into cells as packaged or encapsidated virus by well known techniques for infection and transduction. Viral vectors may be replication competent or replication defective. In the latter case viral propagation generally will occur only in complementing host cells.

Preferred vectors for expression of polynucleotides and polypeptides of the present invention include, but are not limited to, vectors comprising cis-acting control regions effective for expression in a host operatively linked to the polynucleotide to be expressed. Appropriate trans-acting factors either are supplied by the host, supplied by a complementing vector or supplied by the vector itself upon introduction into the host.

In certain preferred embodiments in this regard, the vectors provide for specific expression. Such specific expression may be inducible expression or expression only in certain types of cells or both inducible and cell-specific. Particularly preferred among inducible vectors are vectors that can be induced to express by environmental factors that are easy to manipulate, such as temperature and nutrient additives. A variety of vectors suitable to this aspect of the invention, including constitutive and inducible expression vectors for use in prokaryotic and eukaryotic hosts, are well known and employed routinely by those of skill in the art.

The engineered host cells can be cultured in conventional nutrient media which may be modified as appropriate for, *inter alia*, activating promoters, selecting transformants or amplifying genes. Culture conditions such as temperature, pH and the like, previously used with the host cell selected for expression, generally will be suitable for expression of CSG polypeptides of the present invention.

A great variety of expression vectors can be used to express CSG polypeptides of the invention. Such vectors include chromosomal, episomal and virus-derived vectors. Vectors may be derived from bacterial plasmids, from bacteriophage, from yeast episomes, from yeast chromosomal elements, from viruses such as baculoviruses, papova viruses, such as SV40, vaccinia viruses, adenoviruses, fowl pox viruses, pseudorabies viruses and retroviruses, and from combinations thereof such as those derived from plasmid and bacteriophage genetic elements, such as cosmids and phagemids. All may be used for expression in accordance with this aspect of the present invention. Generally, any vector suitable to maintain, propagate or express polynucleotides to express a polypeptide in a host may be used for expression in this regard.

The appropriate DNA sequence may be inserted into the vector by any of a variety of well-known and routine techniques. In general, a DNA sequence for expression is joined to an expression vector by cleaving the DNA sequence and the expression vector with one or more restriction endonucleases and then joining the restriction fragments together using T4 DNA ligase. Procedures for restriction and ligation that can be used to this end are well known and routine to those of skill. Suitable procedures in this regard, and for constructing expression vectors using alternative techniques, which also are well known and routine to those skill, are set forth in great detail in Sambrook et al. cited elsewhere herein.

The DNA sequence in the expression vector is operatively linked to appropriate expression control sequence(s), including, for instance, a promoter to direct mRNA transcription. Representative promoters include the phage lambda PL promoter, the *E. coli* lac, trp and tac promoters, the SV40 early and late promoters, and promoters of retroviral LTRs, to name just a few of the well-known promoters. It will be understood that numerous promoters not mentioned are also suitable for use in this aspect of the invention and are well known and readily may be employed by those of skill in the manner illustrated by the discussion and the examples herein.

In general, expression constructs will contain sites for transcription initiation and termination, and, in the transcribed region, a ribosome binding site for translation. The coding portion of the mature transcripts expressed by the constructs will include a translation initiating AUG at the beginning and a termination codon appropriately positioned at the end of the polypeptide to be translated.

In addition, the constructs may contain control regions that regulate as well as engender expression. Generally, in accordance with many commonly practiced procedures, such regions will operate by controlling transcription, such as repressor binding sites and enhancers, among others.

Vectors for propagation and expression generally will include selectable markers. Such markers also may be suitable for amplification or the vectors may contain additional markers for this purpose. In this regard, the expression vectors preferably contain one or more selectable marker genes to provide a phenotypic trait for selection of transformed host cells. Preferred markers include dihydrofolate reductase or neomycin resistance for eukaryotic cell culture, and tetracycline or ampicillin resistance genes for culturing in *E. coli* and other bacteria.

The vector containing the appropriate DNA sequence as described elsewhere herein, as well as an appropriate promoter, and other appropriate control sequences, may be introduced into an appropriate host using a variety of well known techniques suitable to expression therein of a desired polypeptide. Representative examples of appropriate hosts include bacterial cells, such as *E. coli, Streptomyces* and *Salmonella typhimurium* cells; fungal cells, such as yeast cells; insect cells such as *Drosophila* S2 and *Spodoptera* Sf9 cells; animal cells such as CHO, COS and Bowes melanoma cells; and plant cells. Hosts for a great variety of expression constructs are well known, and those of skill will be enabled by the present disclosure readily to select a host for expressing a CSG polypeptide in accordance with this aspect of the present invention.

More particularly, the present invention also includes recombinant constructs, such as expression constructs, comprising one or more of the sequences described above. The constructs comprise a vector, such as a plasmid or viral vector, into which such CSG sequence of the invention has been inserted. The sequence may be inserted in a forward or reverse orientation. In certain preferred embodiments in this regard, the construct further comprises regulatory sequences, including, for example, a promoter, operably linked to the sequence. Large numbers of suitable vectors and promoters are known to those of skill in the art, and there are many commercially available vectors suitable for use in the present invention.

The following vectors, which are commercially available, are provided by way of example. Among vectors preferred for use in bacteria are pQE70, pQE60 and pQE-9, available from Qiagen; pBS vectors, Phagescript vectors, Bluescript vectors, pNH8A, pNH16a, pNH18A, pNH46A, available from Stratagene; and ptrc99a, pKK223-3, pKK233-3, pDR540, pRIT5 available from Pharmacia. Among preferred eukaryotic vectors are PWLNEO, pSV2CAT, pOG44, pXT1 and pSG available from Stratagene; and pSVK3, pBPV, pMSG and pSVL available from Pharmacia. These vectors are listed solely by way of illustration of the many commercially available and well known vectors that are available to those of skill in the art for use in accordance with this aspect of the present invention. It will be appreciated by those of skill in the art upon reading this disclsoure that any other plasmid or vector suitable for introduction, maintenance, propagation and/or expression of a CSG polynucleotide or polypeptide of the invention in a host may be used in this aspect of the invention.

Promoter regions can be selected from any desired gene using vectors that contain a reporter transcription unit lacking a promoter region, such as a chloramphenicol acetyl transferase ("cat") transcription unit, downstream of a restriction site or sites for introducing a candidate promoter fragment; i.e., a fragment that may contain a promoter. As is well known, introduction into the vector of a promoter-containing fragment at the restriction site upstream of the cat gene engenders production of CAT activity detectable by standard CAT assays. Vectors suitable to this end are well known and readily available. Two such vectors are pKK232-8 and pCM7. Thus, promoters for expression of CSG polynucleotides of the present invention include, not only well known and readily available promoters, but also promoters that readily may be obtained by the foregoing technique, using a reporter gene.

Among known bacterial promoters suitable for expression of polynucleotides and polypeptides in accordance with the present invention are the *E. coli* laci and lacZ promoters, the T3 and T7 promoters, the gpt promoter, the lambda PR, PL promoters and the trp promoter. Among known eukaryotic promoters suitable in this regard are the CMV immediate early promoter, the HSV thymidine kinase promoter, the early and late SV40 promoters, the promoters of retroviral LTRs, such as those of the Rous sarcoma virus ("RSV"), and metallothionein promoters, such as the mouse metallothionein-I promoter.

Selection of appropriate vectors and promoters for expression in a host cell is a well known procedure and the requisite techniques for expression vector construction, introduction of the vector into the host and expression in the host are routine skills in the art.

The present invention also relates to host cells containing the above-described constructs. The host cell can be a higher eukaryotic cell, such as a mammalian cell, or a lower eukaryotic cell, such as a yeast cell. Alternatively, the host cell can be a prokaryotic cell, such as a bacterial cell.

Introduction of the construct into the host cell can be effected by calcium phosphate transfection, DEAE-dextran mediated transfection, cationic lipid-mediated transfection, electroporation, transduction, infection or other methods. Such methods are described in many standard laboratory manuals, such as Davis et al. BASIC METHODS IN MOLECULAR BIOLOGY, (1986).

Constructs in host cells can be used in a conventional manner to produce the gene product encoded by the recombinant sequence. Alternatively, CSG polypeptides of the invention can be synthetically produced by conventional peptide synthesizers.

Mature proteins can be expressed in mammalian cells, yeast, bacteria, or other cells under the control of appropriate promoters. Cell-free translation systems can also be employed to produce such proteins using RNAs derived from the DNA constructs of the present invention. Appropriate cloning and expression vectors for use with prokaryotic and eukaryotic hosts are described by Sambrook et al. cited elsewhere herein.

Generally, recombinant expression vectors will include origins of replication, a promoter derived from a highly-expressed gene to direct transcription of a downstream structural sequence, and a selectable marker to permit isolation of vector containing cells after exposure to the vector. Among suitable promoters are those derived from the genes that encode glycolytic enzymes such as 3-phosphoglycerate kinase ("PGK"), a-factor, acid phosphatase, and heat shock proteins, among others. Selectable markers include the ampicillin resistance gene of *E. coli* and the *trpl* gene of *S. cerevisiae.*

Transcription of DNA encoding the CSG polypeptides of the present invention by higher eukaryotes may be increased by inserting an enhancer sequence into the vector. Enhancers are cis-acting elements of DNA, usually about from 10 to 300 base pairs (bp) that act to increase transcriptional activity of a promoter in a given host cell-type. Examples of enhancers include the SV40 enhancer, which is located on the late side of the replication origin at bp 100 to 270, the cytomegalovirus early promoter enhancer, the polyoma enhancer on the late side of the replication origin, and adenovirus enhancers.

A polynucleotide of the present invention, encoding a heterologous structural sequence of a CSG polypeptide of the present invention, generally will be inserted into the vector using standard techniques so that it is operably linked to the promoter for expression. The polynucleotide will be positioned so that the transcription start site is located appropriately 5' to a ribosome binding site. The ribosome binding site will be 5' to the AUG that initiates translation of the polypeptide to be expressed. Generally, there will be no other open reading frames that begin with an initiation codon, usually AUG, lying between the ribosome binding site and the initiating AUG. Also, generally, there will be a translation stop codon at the end of the polypeptide and there will be a polyadenylation signal and a transcription termination signal appropriately disposed at the 3' end of the transcribed region.

Appropriate secretion signals may be incorporated into the expressed polypeptide for secretion of the translated protein into the lumen of the endoplasmic reticulum, into the periplasmic space or into the extracellular environment. The signals may be endogenous to the polypeptide or they may be heterologous signals.

The polypeptide may be expressed in a modified form, such as a fusion protein, and may include not only secretion signals but also additional heterologous functional regions. Thus, for instance, a region of additional amino acids, particularly charged amino acids, may be added to the N-terminus of the polypeptide to improve stability and persistence in the host cell during purification or during subsequent handling and storage. A region also may be added to the polypeptide to facilitate purification. Such regions may be removed prior to final preparation of the polypeptide. The addition of peptide moieties to polypeptides to engender secretion or excretion, to improve stability and to facilitate purification, among others, are familiar and routine techniques in the art.

Suitable prokaryotic hosts for propagation, maintenance or expression of CSG polynucleotides and polypeptides in accordance with the invention include *Escherichia coli, Bacillus* subtilis and *Salmonella typhimurium*. Various species of *Pseudomonas, Streptomyces,* and *Staphylococcus* are suitable hosts in this regard. Many other hosts also known to those of skill may also be employed in this regard.

As a representative, but non-limiting example, useful expression vectors for bacterial use can comprise a selectable marker and bacterial origin of replication derived from commercially available plasmids comprising genetic elements of the well known cloning vector pBR322. Such commercial vectors include, for example, pKK223-3 (Pharmacia Fine Chemicals, Uppsala, Sweden) and GEM1 (Promega Biotec, Madison, Wis., USA). These pBR322 "backbone" sections are combined with an appropriate promoter and the structural sequence to be expressed. Following transformation of a suitable host strain and growth of the host strain to an appropriate cell density, where the selected promoter is inducible it is induced by appropriate means (e.g., temperature shift or exposure to chemical inducer) and cells are cultured for an additional period. Cells typically then are harvested by centrifugation, disrupted by physical or chemical means, and the resulting crude extract retained for further purification. Microbial cells employed in expression of proteins can be disrupted by any convenient method, including freeze-thaw cycling, sonication, mechanical disruption, or use of cell lysing agents, such methods are well know to those skilled in the art.

Various mammalian cell culture systems can be employed for expression, as well. An exemplary mammalian expression systems is the COS-7 line of monkey kidney fibroblasts described in Gluzman et al., Cell 23: 175 (1981). Other mammalian cell lines capable of expressing a compatible vector include for example, the C127, 3T3, CHO, HeLa, human kidney 293 and BHK cell lines. Mammalian expression vectors comprise an origin of replication, a suitable promoter and enhancer, and any ribosome binding sites, polyadenylation sites, splice donor and acceptor sites, transcriptional termination sequences, and 5' flanking non-transcribed sequences that are necessary for expression. In certain preferred embodiments in this regard DNA sequences derived from the SV40 splice sites, and the SV40 polyadenylation sites are used for required non-transcribed genetic elements of these types.

CSG polypeptides can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography ("HPLC") is employed for purification. Well known techniques for refolding proteins may be employed to regenerate active conformation when the polypeptide is denatured during isolation and or purification.

CSG polypeptides of the present invention include naturally purified products, products of chemical synthetic procedures, and products produced by recombinant techniques from a prokaryotic or eukaryotic host, including, for example, bacterial, yeast, higher plant, insect and mammalian cells. Depending upon the host employed in a recombinant production procedure, the CSG polypeptides of the present invention may be glycosylated or may be non-glycosylated. In addition, CSG polypeptides of the invention may also include an initial modified methionine residue, in some cases as a result of host-mediated processes.

CSG polynucleotides and polypeptides may be used in accordance with the present invention for a variety of applications, particularly those that make use of the chemical and biological properties of the CSGs. Additional applications relate to diagnosis and to treatment of disorders of cells, tissues and organisms. These aspects of the invention are illustrated further by the following discussion.

### Polynucleotide assays

As discussed in some detail *supra,* this invention is also related to the use of CSG polynucleotides to detect complementary polynucleotides such as, for example, as a diagnostic reagent. Detection of a mutated form of CSG associated with a dysfunction will provide a diagnostic tool that can add to or define a diagnosis of a disease or susceptibility to a disease which results from under-expression, over-expression or altered expression of a CSG, such as, for example, a susceptibility to inherited colon cancer.

Individuals carrying mutations in a human CSG gene may be detected at the DNA level by a variety of techniques. Nucleic acids for diagnosis may be obtained from a patient's cells, such as from blood, urine, saliva, tissue biopsy and autopsy material. The genomic DNA may be used directly for detection or may be amplified enzymatically using PCR prior to analysis(Saiki et al., Nature, 324: 163-166 (1986)). RNA or cDNA may also be used in a similar manner. As an example, PCR primers complementary to a CSG polynucleotide of SEQ ID NO: 1, 2, 3, 4, 5, 6, 7, 8, 9, 10, 11, 12, 13, 14, 15, 16, 17, 18, 19, 20, 21 or 22 can be used to identify and analyze CSG expression and mutations. For example, deletions and insertions can be detected by a change in size of the amplified product in comparison to the normal genotype. Point mutations can be identified by hybridizing amplified DNA to radiolabeled CSG RNA or alternatively, radiolabeled CSG antisense DNA sequences. Perfectly matched sequences can be distinguished from mismatched duplexes by RNase A digestion or by differences in melting temperatures.

Sequence differences between a reference gene and genes having mutations also may be revealed by direct DNA sequencing. In addition, cloned DNA segments may be employed as probes to detect specific DNA segments. The sensitivity of such methods can be greatly enhanced by appropriate use of PCR or another amplification method. For example, a sequencing primer is used with double-stranded PCR product or a single-stranded template molecule generated by a modified PCR. The sequence determination is performed by conventional procedures with radiolabeled nucleotide or by automatic sequencing procedures with fluorescent-tags.

Genetic testing based on DNA sequence differences may be achieved by detection of alterations in electrophoretic mobility of DNA fragments in gels, with or without denaturing agents. Small sequence deletions and insertions can be visualized by high resolution gel electrophoresis. DNA fragments of different sequences may be distinguished on denaturing formamide gradient gels in which the mobilities of different DNA fragments are retarded in the gel at different positions according to their specific melting or partial melting temperatures (see, e.g., Myers et al., Science, 230: 1242 (1985)).

Sequence changes at specific locations also may be revealed by nuclease protection assays, such as RNase and S1 protection or the chemical cleavage method (e.g., Cotton et al., Proc. Natl. Acad. Sci., USA, 85: 4397-4401 (1985)).

Thus, the detection of a specific DNA sequence may be achieved by methods such as hybridization, RNase protection, chemical cleavage, direct DNA sequencing or the use of restriction enzymes, (e.g., restriction fragment length polymorphisms ("RFLP") and Southern blotting of genomic DNA. In addition to more conventional gel-electrophoresis and DNA sequencing, mutations also can be detected by *in situ* analysis.

### Chromosome assays

The CSG sequences of the present invention are also valuable for chromosome identification. There is a need for identifying particular sites on the chromosome and few chromosome marking reagents based on actual sequence data (repeat polymorphisms) are presently available for marking chromosomal location. Each CSG sequence of the present invention is specifically targeted to and can hybridize with a particular location on an individual human chromosome. Thus, the CSGs can be used in the mapping of DNAs to chromosomes, an important first step in correlating sequences with genes associated with disease.

In certain preferred embodiments in this regard, the cDNA herein disclosed is used to clone genomic DNA of a CSG of the present invention. This can be accomplished using a variety of well known techniques and libraries, which generally are available commercially. The genomic DNA is used for in situ chromosome mapping using well known techniques for this purpose.

In some cases, sequences can be mapped to chromosomes by preparing PCR primers (preferably 15-25 bp) from the cDNA. Computer analysis of the 3' untranslated region of the gene is used to rapidly select primers that do not span more than one exon in the genomic DNA, thus complicating the amplification process. These primers are then used for PCR screening of somatic cell hybrids containing individual human chromosomes. Only those hybrids containing the human gene corresponding to the primer will yield an amplified fragment.

PCR mapping of somatic cell hybrids is a rapid procedure for assigning a particular DNA to a particular chromosome. Using the present invention with the same oligonucleotide primers, sublocalization can be achieved with panels of fragments from specific chromosomes or pools of large genomic clones in an analogous manner. Other mapping strategies that can similarly be used to map to its chromosome include in situ hybridization, prescreening with labeled flow-sorted chromosomes and preselection by hybridization to construct chromosome specific-cDNA libraries.

Fluorescence in situ hybridization ("FISH") of a cDNA clone to a metaphase chromosomal spread can be used to provide a precise chromosomal location in one step. This technique can be used with cDNA as short as 50 or 60 bp. This technique is described by Verma et al. (HUMAN CHROMOSOMES: A MANUAL OF BASIC TECHNIQUES, Pergamon Press, New York (1988)).

Once a sequence has been mapped to a precise chromosomal location, the physical position of the sequence on the chromosome can be correlated with genetic map data. Such data are found, for example, in V. McKusick, MENDELIAN INHERITANCE IN MAN, available on line through Johns Hopkins University, Welch Medical Library. The relationship between genes and diseases that have been mapped to the same chromosomal region are then identified through linkage analysis (coinheritance of physically adjacent genes).

Next, it is necessary to determine the differences in the cDNA or genomic sequence between affected and unaffected individuals. If a mutation is observed in some or all of the affected individuals but not in any normal individuals, then the mutation is likely to be the causative agent of the disease.

With current resolution of physical mapping and genetic mapping techniques, a cDNA precisely localized to a chromosomal region associated with the disease could be one of between 50 and 500 potential causative genes. (This assumes 1 megabase mapping resolution and one gene per 20 kb).

### Polypeptide assays

As described in some detail *supra,* the present invention also relates to diagnostic assays such as quantitative and diagnostic assays for detecting levels of CSG polypeptide in cells and tissues, and biological fluids such as blood and urine, including determination of normal and abnormal levels. Thus, for instance, a diagnostic assay in accordance with the present invention for detecting over-expression or under-expression of a CSG polypeptide compared to normal control tissue samples may be used to detect the presence of neoplasia. Assay techniques that can be used to determine levels of a protein, such as a CSG polypeptide of the present invention, in a sample derived from a host are well-known to those of skill in the art. Such assay methods include radioimmunoassays, competitive-binding assays, Western Blot analysis and ELISA assays. Among these ELISAs frequently are preferred.

For example, antibody-sandwich ELISAs are used to detect polypeptides in a sample, preferably a biological sample. Wells of a microtiter plate are coated with specific antibodies, at a final concentration of 0.2 to 10 µg/ml. The antibodies are either monoclonal or polyclonal and are produced by methods as described herein. The wells are blocked so that non-specific binding of the polypeptide to the well is reduced. The coated wells are then incubated for > 2 hours at room temperature with a sample containing the CSG polypeptide. Preferably, serial dilutions of the sample should be used to validate results. The plates are then washed three times with deionized or distilled water to remove unbounded polypeptide. Next, 50 µl of specific antibodyalkaline phosphatase conjugate, at a concentration of 25-400 ng, is added and incubated for 2 hours at room temperature. The plates are again washed three times with deionized or distilled water to remove unbounded conjugate. 4-methylumbelliferyl phosphate (MUP) or p-nitrophenyl phosphate (NPP) substrate solution (75µl) is then added to each well and the plate is incubated 1 hour at room temperature. The reaction is measured by a microtiter plate reader. A standard curve is prepared using serial dilutions of a control sample, and polypeptide concentration is plotted on the X-axis (log scale) while fluorescence or absorbance is plotted on the Y-axis (linear scale). The concentration of the CSG polypeptide in the sample is interpolated using the standard curve.

### Antibodies

As discussed in some detail supra, CSG polypeptides, their fragments or other derivatives, or analogs thereof, or cells expressing them can be used as an immunogen to produce antibodies thereto. These antibodies can be polyclonal or monoclonal antibodies. The present invention also includes chimeric, single chain, and humanized antibodies, as well as Fab fragments, or the product of an Fab expression library. Various procedures known in the art may be used for the production of such antibodies and fragments.

A variety of methods for antibody production are set forth in Current Protocols, Chapter 2.

For example, cells expressing a CSG polypeptide of the present invention can be administered to an animal to induce the production of sera containing polyclonal antibodies. In a preferred method, a preparation of the secreted protein is prepared and purified to render it substantially free of natural contaminants. This preparation is then introduced into an animal in order to produce polyclonal antisera of greater specific activity. The antibody obtained will bind with the CSG polypeptide itself. In this manner, even a sequence encoding only a fragment of the CSG polypeptide can be used to generate antibodies binding the whole native polypeptide. Such antibodies can then be used to isolate the CSG polypeptide from tissue expressing that CSG polypeptide.

Alternatively, monoclonal antibodies can be prepared. Examples of techniques for production of monoclonal antibodies include, but are not limited to, the hybridoma technique (Kohler, G. and Milstein, C., Nature 256: 495-497 (1975), the trioma technique, the human B-cell hybridoma technique (Kozbor et al., Immunology Today 4: 72 (1983) and (Cole et al., pg. 77-96 in MONOCLONAL ANTIBODIES AND CANCER THERAPY, Alan R. Liss, Inc. (1985). The EBV-hybridoma technique is useful in production of human monoclonal antibodies.

Hybridoma technologies have also been described by Khler et al. (Eur. J. Immunol. 6: 511 (1976)) Khler et al. (Eur. J.Immunol. 6: 292 (1976)) and Hammerling et al. (in: Monoclonal Antibodies and T-Cell Hybridomas, Elsevier, N. Y., pp. 563-681 (1981)). In general, such procedures involve immunizing an animal (preferably a mouse) with CSG polypeptide or, more preferably, with a secreted CSG polypeptide-expressing cell. Such cells may be cultured in any suitable tissue culture medium; however, it is preferable to culture cells in Earle's modified Eagle's medium supplemented with 10% fetal bovine serum (inactivated at about 56°C), and supplemented with about 10 g/l of nonessential amino acids, about 1,000 U/ml of penicillin, and about 100 µg/ml of streptomycin. The splenocytes of such mice are extracted and fused with a suitable myeloma cell line. Any suitable myeloma cell line may be employed in accordance with the present invention; however, it is preferable to employ the parent myeloma cell line (SP20), available from the ATCC. After fusion, the resulting hybridoma cells are selectively maintained in HAT medium, and then cloned by limiting dilution as described by Wands et al. (Gastroenterology 80: 225-232 (1981).). The hybridoma cells obtained through such a selection are then assayed to identify clones which secrete antibodies capable of binding the polypeptide.

Alternatively, additional antibodies capable of binding to the polypeptide can be produced in a two-step procedure using anti-idiotypic antibodies. Such a method makes use of the fact that antibodies are themselves antigens, and therefore, it is possible to obtain an antibody which binds to a second antibody. In accordance with this method, protein specific antibodies are used to immunize an animal, preferably a mouse. The splenocytes of such an animal are then used to produce hybridoma cells, and the hybridoma cells are screened to identify clones which produce an antibody whose ability to bind to the protein-specific antibody can be blocked by the polypeptide. Such antibodies comprise anti-idiotypic antibodies to the protein specific antibody and can be used to immunize an animal to induce formation of further protein-specific antibodies.

Techniques described for the production of single chain antibodies (U.S. Patent 4,946,778) can also be adapted to produce single chain antibodies to immunogenic polypeptide products of this invention. Also, transgenic mice, as well as other nonhuman transgenic animals, may be used to express humanized antibodies to immunogenic polypeptide products of this invention.

It will be appreciated that Fab, F(ab')2 and other fragments of the antibodies of the present invention may also be used according to the methods disclosed herein. Such fragments are typically produced by proteolytic cleavage, using enzymes such as papain (to produce Fab fragments) or pepsin (to produce F(ab')2 fragments). Alternatively, secreted protein-binding fragments can be produced through the application of recombinant DNA technology or through synthetic chemistry.

For in vivo use of antibodies in humans, it may be preferable to use "humanized" chimeric monoclonal antibodies. Such antibodies can be produced using genetic constructs derived from hybridoma cells producing the monoclonal antibodies described above. Methods for producing chimeric antibodies are known in the art (See, for review, Morrison, Science 229: 1202 (1985); Oi et al., BioTechniques 4: 214 (1986); Cabilly et al., U. S. Patent 4,816,567; Taniguchi et al., EP 171496; Morrison et al., EP 173494; Neuberger et al., WO 8601533; Robinson et al., WO 8702671; Boulianne et al., Nature 312: 643 (1984); Neuberger et al., Nature 314: 268 (1985).)

The above-described antibodies may be employed to isolate or to identify clones expressing CSG polypeptides or purify CSG polypeptides of the present invention by attachment of the antibody to a solid support for isolation and/or purification by affinity chromatography. As discussed in more detail supra, antibodies specific against a CSG may also be used to image tumors, particularly cancer of the colon, in patients suffering from cancer. Such antibodies may also be used therapeutically to target tumors expressing a CSG.

### CSG binding molecules and assays

This invention also provides a method for identification of molecules, such as receptor molecules, that bind CSGs. Genes encoding proteins that bind CSGs, such as receptor proteins, can be identified by numerous methods known to those of skill in the art. Examples include, but are not limited to, ligand panning and FACS sorting. Such methods are described in many laboratory manuals such as, for instance, Coligan et al., Current Protocols in Immunology 1(2): Chapter 5 (1991).

Expression cloning may also be employed for this purpose. To this end, polyadenylated RNA is prepared from a cell responsive to a CSG of the present invention. A cDNA library is created from this RNA and the library is divided into pools. The pools are then transfected individually into cells that are not responsive to a CSG of the present invention. The transfected cells then are exposed to labeled CSG. CSG polypeptides can be labeled by a variety of well-known techniques including, but not limited to, standard methods of radio-iodination or inclusion of a recognition site for a site-specific protein kinase. Following exposure, the cells are fixed and binding of labeled CSG is determined. These procedures conveniently are carried out on glass slides. Pools containing labeled CSG are identified as containing cDNA that produced CSG-binding cells. Sub-pools are then prepared from these positives, transfected into host cells and screened as described above. Using an iterative sub-pooling and re-screening process, one or more single clones that encode the putative binding molecule, such as a receptor molecule, can be isolated.

Alternatively a labeled ligand can be photoaffinity linked to a cell extract, such as a membrane or a membrane extract, prepared from cells that express a molecule that it binds, such as a receptor molecule. Cross-linked material is resolved by polyacrylamide gel electrophoresis ("PAGE") and exposed to X-ray film. The labeled complex containing the ligand-receptor can be excised, resolved into peptide fragments, and subjected to protein microsequencing. The amino acid sequence obtained from microsequencing can be used to design unique or degenerate oligonucleotide probes to screen cDNA libraries to identify genes encoding the putative receptor molecule.

Polypeptides of the invention also can be used to assess CSG binding capacity of CSG binding molecules, such as receptor molecules, in cells or in cell-free preparations.

### Agonists and antagonists - assays and molecules

The invention also provides a method of screening compounds to identify those which enhance or block the action of a CSG on cells. By "compound", as used herein, it is meant to be inclusive of small organic molecules, peptides, polypeptides and antibodies as well as any other candidate molecules which have the potential to enhance or agonize or block or antagonize the action of CSG on cells. As used herein, an agonist is a compound which increases the natural biological functions of a CSG or which functions in a manner similar to a CSG, while an antagonist, as used herein, is a compound which decreases or eliminates such functions. Various known methods for screening for agonists and/or antagonists can be adapted for use in identifying CSG agonist or antagonists.

For example, a cellular compartment, such as a membrane or a preparation thereof, such as a membrane-preparation, may be prepared from a cell that expresses a molecule that binds a CSG, such as a molecule of a signaling or regulatory pathway modulated by CSG. The preparation is incubated with labeled CSG in the absence or the presence of a compound which may be a CSG agonist or antagonist. The ability of the compound to bind the binding molecule is reflected in decreased binding of the labeled ligand. Compounds which bind gratuitously, i.e., without inducing the effects of a CSG upon binding to the CSG binding molecule are most likely to be good antagonists. Compounds that bind well and elicit effects that are the same as or closely related to CSG are agonists. CSG-like effects of potential agonists and antagonists may by measured, for instance, by determining activity of a second messenger system following interaction of the candidate molecule with a cell or appropriate cell preparation, and comparing the effect with that of CSG or molecules that elicit the same effects as CSG. Second messenger systems that may be useful in this regard include, but are not limited to, AMP guanylate cyclase, ion channel or phosphoinositide hydrolysis second messenger systems.

Another example of an assay for CSG antagonists is a competitive assay that combines CSG and a potential antagonist with membrane-bound CSG receptor molecules or recombinant CSG receptor molecules under appropriate conditions for a competitive inhibition assay. CSG can be labeled, such as by radioactivity, such that the number of CSG molecules bound to a receptor molecule can be determined accurately to assess the effectiveness of the potential antagonist.

Potential antagonists include small organic molecules, peptides, polypeptides and antibodies that bind to a CSG polypeptide of the invention and thereby inhibit or extinguish its activity. Potential antagonists also may be small organic molecules, a peptide, a polypeptide such as a closely related protein or antibody that binds the same sites on a binding molecule, such as a receptor molecule, without inducing CSG-induced activities, thereby preventing the action of CSG by excluding CSG from binding.

Potential antagonists include small molecules which bind to and occupy the binding site of the CSG polypeptide thereby preventing binding to cellular binding molecules, such as receptor molecules, such that normal biological activity is prevented. Examples of small molecules include but are not limited to small organic molecules, peptides or peptide-like molecules.

Other potential antagonists include antisense molecules. Antisense technology can be used to control gene expression through antisense DNA or RNA or through triple-helix formation. Antisense techniques are discussed, for example, in Okano, J. Neurochem. 56: 560 (1991); OLIGODEOXYNUCLEOTIDES AS ANTISENSE INHIBITORS OF GENE EXPRESSION, CRC Press, Boca Raton, Fla. (1988). Triple helix formation is discussed in, for instance Lee et al., Nucleic Acids Research 6: 3073 (1979); Cooney et al., Science 241: 456 (1988); and Dervan et al., Science 251: 1360 (1991). The methods are based on binding of a polynucleotide to a complementary DNA or RNA. For example, the 5' coding portion of a polynucleotide that encodes a mature CSG polypeptide of the present invention may be used to design an antisense RNA oligonucleotide of from about 10 to 40 base pairs in length. A DNA oligonucleotide is designed to be complementary to a region of the gene involved in transcription thereby preventing transcription and the production of a CSG polypeptide. The antisense RNA oligonucleotide hybridizes to the mRNA *in vivo* and blocks translation of the mRNA molecule into a CSG polypeptide. The oligonucleotides described above can also be delivered to cells such that the antisense RNA or DNA may be expressed *in vivo* to inhibit production of a CSG.

### Compositions

The present invention also relates to compositions comprising a CSG polynucleotide or a CSG polypeptide or an agonist or antagonist thereof.

For example, a CSG polynucleotide, polypeptide or an agonist or antagonist thereof of the present invention may be employed in combination with a non-sterile or sterile carrier or carriers for use with cells, tissues or organisms, such as a pharmaceutical carrier suitable for administration to a subject. Such compositions comprise, for instance, a media additive or a therapeutically effective amount of a polypeptide of the invention and a pharmaceutically acceptable carrier or excipient. Such carriers may include, but are not limited to, saline, buffered saline, dextrose, water, glycerol, ethanol and combinations thereof. The formulation should suit the mode of administration.

Compositions of the present invention will be formulated and dosed in a fashion consistent with good medical practice, taking into account the clinical condition of the individual patient (especially the side effects of treatment with the polypeptide or other compound alone), the site of delivery, the method of administration, the scheduling of administration, and other factors known to practitioners. The "effective amount" for purposes herein is thus determined by such considerations.

As a general proposition, the total pharmaceutically effective amount of secreted polypeptide administered parenterally per dose will be in the range of about 1, µg/kg/day to 10 mg/kg/day of patient body weight, although, as noted above, this will be subject to therapeutic discretion. More preferably, this dose is at least 0.01 mg/kg/day, and most preferably for humans between about 0.01 and 1 mg/kg/day for the hormone. If given continuously, the polypeptide or other compound is typically administered at a dose rate of about 1 µg/kg/hour to about 50 mg/kg/hour, either by 1-4 injections per day or by continuous subcutaneous infusion, for example, using a mini-pump. An intravenous bag solution may also be employed. The length of treatment needed to observe changes and the interval following treatment for responses to occur appears to vary depending on the desired effect.

Pharmaceutical compositions containing the secreted protein of the invention are administered orally, rectally, parenterally, intracistemally, intravaginally, intraperitoneally, topically (as by powders, ointments, gels, drops or transdermal patch), bucally, or as an oral or nasal spray. "Pharmaceutically acceptable carrier" refers to a non-toxic solid, semisolid or liquid filler, diluent, encapsulating material or formulation auxiliary of any type. The term "parenteral" as used herein refers to modes of administration which include intravenous, intramuscular, intraperitoneal, intrasternal, subcutaneous and intraarticular injection and infusion.

The polypeptide or other compound is also suitably administered by sustained-release systems. Suitable examples of sustained-release compositions include semipermeable polymer matrices in the form of shaped articles, e. g., films, or microcapsules. Sustained-release matrices include polylactides (U.S. Patent 3,773,919 and EP 58481), copolymers of L-glutamic acid and gamma-ethyl-L-glutamate (Sidman, U. et al., Biopolymers 22: 547-556 (1983)), poly (2-hydroxyethyl methacrylate) (R. Langer et al., J. Biomed. Mater. Res. 15: 167-277 (1981), and R. Langer, Chem. Tech. 12: 98-105 (1982)), ethylene vinyl acetate (R. Langer et al.) and poly-D- (-)-3-hydroxybutyric acid (EP 133,988). Sustained-release compositions also include liposomally entrapped polypeptides. Liposomes containing the polypeptide or other compound are prepared by well known methods (Epstein et al., Proc. Natl. Acad. Sci. USA 82: 3688-3692 (1985); Hwang et al., Proc. Natl. Acad. Sci. USA 77: 4030-4034 (1980); EP 52322; EP 36676; EP 88046; EP 143949; EP 142641; Japanese Pat. Appl. 83-118008; U.S. Patent 4,485,045 and 4,544,545; and EP 102324). Ordinarily, the liposomes are of the small (about 200-800 Angstroms) unilamellar type in which the lipid content is greater than about 30 mol. percent cholesterol, the selected proportion being adjusted for the optimal therapy.

For parenteral administration, in one embodiment, the polypeptide or other compound is formulated generally by mixing it at the desired degree of purity, in a unit dosage injectable form (solution, suspension, or emulsion), with a pharmaceutically acceptable carrier, i.e., one that.is non-toxic to recipients at the dosages and concentrations employed and is compatible with other ingredients of the formulation.

For example, the formulation preferably does not include oxidizing agents and other compounds that are known to be deleterious to the polypeptide or other compound.

Generally, the formulations are prepared by contacting the polypeptide or other compound uniformly and intimately with liquid carriers or finely divided solid carriers or both. Then, if necessary, the product is shaped into the desired formulation. Preferably the carrier is a parenteral carrier, more preferably a solution that is isotonic with the blood of the recipient. Examples of such carrier vehicles include water, saline, Ringer's solution, and dextrose solution. Nonaqueous vehicles such as fixed oils and ethyl oleate are also useful herein, as well as liposomes.

The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) polypeptides, e. g., polyarginine or tripeptides; proteins, such as serum albumin, gelatin, or immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, mannose, or dextrins; chelating agents such as EDTA; sugar alcohols such as mannitol or sorbitol; counterions such as sodium; and/or nonionic surfactants such as polysorbates, poloxamers, or PEG.

The polypeptide or other compound is typically formulated in such vehicles at a concentration of about 0.1 mg/ml to 100 mg/ml, preferably 1-10 mg/ml, at a pH of about 3 to 8. It will be understood that the use of certain of the foregoing excipients, carriers, or stabilizers will result in the formation of polypeptide salts or salts of the other compounds.

Any polypeptide to be used for therapeutic administration should be sterile. Sterility is readily accomplished by filtration through sterile filtration membranes (e. g., 0.2 micron membranes). Therapeutic polypeptide compositions generally are placed into a container having a sterile access port, for example, an intravenous solution bag or vial having a stopper pierceable by a hypodermic injection needle.

Polypeptides ordinarily will be stored in unit or multidose containers, for example, sealed ampules or vials, as an aqueous solution or as a lyophilized formulation for reconstitution. As an example of a lyophilized formulation, 10-ml vials are filled with 5 ml of sterile-filtered 1 % (w/v) aqueous polypeptide solution, and the resulting mixture is lyophilized. The infusion solution is prepared by reconstituting the lyophilized polypeptide using bacteriostatic Water-for-Injection.

### Kits

The invention further relates to pharmaceutical packs and kits comprising one or more containers filled with one or more of the ingredients of the aforementioned compositions of the invention. Associated with such container(s) can be a notice in the form prescribed by a governmental agency regulating the manufacture, use or sale of pharmaceuticals or biological products, reflecting approval by the agency of the manufacture, use or sale of the product for human administration.

### Administration

CSG polypeptides or polynucleotides or other compounds, preferably agonists or antagonists thereof of the present invention may be employed alone or in conjunction with other compounds, such as therapeutic compounds.

The pharmaceutical compositions may be administered in any effective, convenient manner including, for instance, administration by topical, oral, anal, vaginal, intravenous, intraperitoneal, intramuscular, subcutaneous, intranasal or intradermal routes among others.

The pharmaceutical compositions generally are administered in an amount effective for treatment or prophylaxis of a specific indication or indications. In general, the compositions are administered in an amount of at least about 10 µg/kg body weight. However, it will be appreciated that optimum dosage will be determined by standard methods for each treatment modality and indication, taking into account the indication, its severity, route of administration, complicating conditions and the like.

It will be appreciated that conditions caused by a decrease in the standard or normal expression level of a CSG polypeptide in an individual can be treated by administering the CSG polypeptide of the present invention, preferably in the secreted form, or an agonist thereof. Thus, the invention also provides a method of treatment of an individual in need of an increased level of a CSG polypeptide comprising administering to such an individual a pharmaceutical composition comprising an amount of the CSG polypeptide or an agonist thereof to increase the activity level of the CSG polypeptide in such an individual. For example, a patient with decreased levels of a CSG polypeptide may receive a daily dose 0.1-100 µg/kg of a CSG polypeptide or agonist thereof for six consecutive days. Preferably, if a CSG polypeptide is administered it is in the secreted form.

Compositions of the present invention can also be administered to treating increased levels of a CSG polypeptide. For example, antisense technology can be used to inhibit production of a CSG polypeptide of the present invention. This technology is one example of a method of decreasing levels of a polypeptide, preferably a secreted form, due to a variety of etiologies, such as cancer. A patient diagnosed with abnormally increased levels of a polypeptide can be administered intravenously antisense polynucleotides at 0.5, 1.0, 1.5, 2.0 and 3.0 mg/kg day for 21 days. This treatment is preferably repeated after a 7-day rest period if the treatment was well tolerated. Compositions comprising an antagonist of a CSG polypeptide can also be administered to decrease levels of CSG in a patient.

### Gene therapy

The CSG polynucleotides, polypeptides, agonists and antagonists that are polypeptides may be employed in accordance with the present invention by expression of such polypeptides in vivo, in treatment modalities often referred to as "gene therapy."

Thus, for example, cells from a patient may be engineered with a polynucleotide, such as a DNA or RNA, encoding a polypeptide ex vivo, and the engineered cells then can be provided to a patient to be treated with the polypeptide. For example, cells may be engineered ex vivo by the use of a retroviral plasmid vector containing RNA encoding a polypeptide of the present invention. Such methods are well-known in the art and their use in the present invention will be apparent from the teachings herein.

Similarly, cells may be engineered in vivo for expression of a polypeptide in vivo by procedures known in the art. For example, a polynucleotide of the invention may be engineered for expression in a replication defective retroviral vector, as discussed supra. The retroviral expression construct then may be isolated and introduced into a packaging cell transduced with a retroviral plasmid vector containing RNA encoding a polypeptide of the present invention such that the packaging cell now produces infectious viral particles containing the gene of interest. These producer cells may be administered to a patient for engineering cells in vivo and expression of the polypeptide *in vivo.* These and other methods for administering a polypeptide of the present invention would be apparent to those skilled in the art upon reading the instant application.

Retroviruses from which the retroviral plasmid vectors herein above mentioned may be derived include, but are not limited to, Moloney Murine Leukemia Virus, spleen necrosis virus, retroviruses such as Rous Sarcoma Virus, Harvey Sarcoma Virus, avian leukosis virus, gibbon ape leukemia virus, human immunodeficiency virus, adenovirus, Myeloproliferative Sarcoma Virus, and mammary tumor virus. In one embodiment, the retroviral plasmid vector is derived from Moloney Murine Leukemia Virus.

Such vectors will include one or more promoters for expressing the polypeptide. The selection of a suitable promoter will be apparent to those skilled in the art from the teachings contained herein. However, examples of suitable promoters which may be employed include, but are not limited to, the retroviral LTR, the SV40 promoter, the human cytomegalovirus (CMV) promoter described in Miller et al., Biotechniques 7: 980-990 (1989), and eukaryotic cellular promoters such as the histone, RNA polymerase III, and beta-actin promoters. Other viral promoters which may be employed include, but are not limited to, adenovirus promoters, thymidine kinase (TK) promoters, and B19 parvovirus promoters. Additional promoters which may be used include respiratory syncytial virus (RSV) promoter, inducible promoters such as the MMT promoter, the metallothionein promoter, heat shock promoters, the albumin promoter, the ApoAI promoter, human globin promoters, viral thymidine kinase promoters such as the Herpes Simplex thymidine kinase promoter, retroviral LTRs, the beta-actin promoter, and human growth hormone promoters. The promoter also may be the native promoter which controls the gene encoding the polypeptide.

The nucleic acid sequence encoding the polypeptide of the present invention will be placed under the control of a suitable promoter.

In one embodiment, the retroviral plasmid vector is employed to transduce packaging cell lines to form producer cell lines. Examples of packaging cells which may be transfected include, but are not limited to, the PE501, PA317, Y-2, Y-AM, PA12, T19-14X, VT-19-17-H2, YCRE, YCRIP, GP+E-86, GP+envAml2, and DAN cell lines as described in Miller, A., Human Gene Therapy 1: 5-14 (1990). The vector may be transduced into the packaging cells through any means known in the art. Such means include, but are not limited to, electroporation, the use of liposomes, and CaPO₄ precipitation. Alternatively, the retroviral plasmid vector may be encapsulated into a liposome, or coupled to a lipid, and then administered to a host. The producer cell line will generate infectious retroviral vector particles which are inclusive of the nucleic acid sequence(s) encoding the polypeptides. Such retroviral vector particles then may be employed to transduce eukaryotic cells, either in vitro or in vivo. The transduced eukaryotic cells will express the nucleic acid sequence(s) encoding the polypeptide. Eukaryotic cells which may be transduced include, but are not limited to, embryonic stem cells, embryonic carcinoma cells, as well as hematopoietic stem cells, hepatocytes, fibroblasts, myoblasts, keratinocytes, endothelial cells, and bronchial epithelial cells.

An exemplary method of gene therapy involves transplantation of fibroblasts which are capable of expressing a CSG polypeptide or an agonist or antagonist thereof onto a patient. Generally fibroblasts are obtained from a subject by skin biopsy. The resulting tissue is placed in tissue-culture medium and separated into small pieces. Small chunks of the tissue are placed on a wet surface of a tissue culture flask, approximately ten pieces are placed in each flask. The flask is turned upside down, closed tight and left at room temperature over night. After 24 hours at room temperature, the flask is inverted and the chunks of tissue remain fixed to the bottom of the flask and fresh media (e. g., Ham's F12 media, with 10% FBS, penicillin and streptomycin) is added. The flasks are then incubated at 37°C for approximately one week. At this time, fresh media is added and subsequently changed every several days. After an additional two weeks in culture, a monolayer of fibroblasts emerge. The monolayer is trypsinized and scaled into larger flasks. pMV-7 (Kirschmeier, P. T. et al., DNA, 7: 219-25 (1988)), flanked by the long terminal repeats of the Moloney murine sarcoma virus, is digested with EcoRI and HindIII and subsequently treated with calf intestinal phosphatase. The linear vector is fractionated on agarose gel and purified, using glass beads. The cDNA encoding a CSG polypeptide of the present invention or an agonist or antagonist thereof can be amplified using PCR primers which correspond to their 5' and 3' end sequences respectively. Preferably, the 5' primer contains an EcoRI site and the 3' primer includes a HindIII site. Equal quantities of the Moloney murine sarcoma virus linear backbone and the amplified EcoRI and HindIII fragment are added together in the presence of T4 DNA ligase. The resulting mixture is maintained under conditions appropriate for ligation of the two fragments. The ligation mixture is then used to transform bacteria HB 101, which are then plated onto agar containing kanamycin for the purpose of confirming that the vector has the gene of interest properly inserted. Amphotropic pA317 or GP+aml2 packaging cells are grown in tissue culture to confluent density in Dulbecco's Modified Eagles Medium (DMEM) with 10% calf serum (CS), penicillin and streptomycin. The MSV vector containing the gene is then added to the media and the packaging cells transduced with the vector. The packaging cells now produce infectious viral particles containing the gene (the packaging cells are now referred to as producer cells). Fresh media is added to the transduced producer cells, and subsequently, the media is harvested from a 10 cm plate of confluent producer cells. The spent media, containing the infectious viral particles, is filtered through a millipore filter to remove detached producer cells and this media is then used to infect fibroblast cells. Media is removed from a sub-confluent plate of fibroblasts and quickly replaced with the media from the producer cells. This media is removed and replaced with fresh media. If the titer of virus is high, then virtually all fibroblasts will be infected and no selection is required. If the titer is very low, then it is necessary to use a retroviral vector that has a selectable marker, such as neo or his. Once the fibroblasts have been efficiently infected, the fibroblasts are analyzed to determine whether protein is produced. The engineered fibroblasts are then transplanted onto the host, either alone or after having been grown to confluence on cytodex 3 microcarrier beads.

Alternatively, in vivo gene therapy methods can be used to treat CSG related disorders, diseases and conditions. Gene therapy methods relate to the introduction of naked nucleic acid (DNA, RNA, and antisense DNA or RNA) sequences into an animal to increase or decrease the expression of the polypeptide.

For example, a CSG polynucleotide of the present invention or a nucleic acid sequence encoding an agonist or antagonist thereto may be operatively linked to a promoter or any other genetic elements necessary for the expression of the polypeptide by the target tissue. Such gene therapy and delivery techniques and methods are known in the art, see, for example, W0 90/11092, W0 98/11779; U.S. Patents 5,693,622, 5,705,151, and 5,580,859; Tabata H. et al. (1997) Cardiovasc. Res. 35 (3): 470-479, Chao J et al. (1997) Pharmacol. Res. 35 (6): 517-522, Wolff J. A. (1997) Neuromuscul. Disord. 7 (5): 314-318, Schwartz B. et al. (1996) Gene Ther. 3 (5): 405-411, Tsurumi Y. et al. (1996) Circulation 94 (12): 3281-3290 (incorporated herein by reference). The polynucleotide constructs may be delivered by any method that delivers injectable materials to the cells of an animal, such as, injection into the interstitial space of tissues (heart, muscle, skin, lung, liver, intestine and the like). The polynucleotide constructs can be delivered in a pharmaceutically acceptable liquid or aqueous carrier.

The term "naked" polynucleotide, DNA or RNA, refers to sequences that are free from any delivery vehicle that acts to assist, promote, or facilitate entry into the cell, including viral sequences, viral particles, liposome formulations, lipofectin or precipitating agents and the like. However, polynucleotides may also be delivered in liposome formulations (such as those taught in Felgner P. L. et al. (1995) Ann. NY Acad. Sci. 772: 126-139 and Abdallah B. et al. (1995) Biol. Cell 85 (1): 1-7) which can be prepared by methods well known to those skilled in the art.

The polynucleotide vector constructs used in the gene therapy method are preferably constructs that will not integrate into the host genome nor will they contain sequences that allow for replication. Any strong promoter known to those skilled in the art can be used for driving the expression of DNA. Unlike other gene therapies techniques, one major advantage of introducing naked nucleic acid sequences into target cells is the transitory nature of the polynucleotide synthesis in the cells. Studies have shown that non-replicating DNA sequences can be introduced into cells to provide production of the desired polypeptide for periods of up to six months.

The polynucleotide construct can be delivered to the interstitial space of tissues within the an animal, including of muscle, skin, brain, lung, liver, spleen, bone marrow, thymus, heart, lymph, blood, bone, cartilage, pancreas, kidney, gall bladder, stomach, intestine, testis, ovary, uterus, rectum, nervous system, eye, gland, and connective tissue. Interstitial space of the tissues comprises the intercellular fluid, mucopolysaccharide matrix among the reticular fibers of organ tissues, elastic fibers in the walls of vessels or chambers, collagen fibers of fibrous tissues, or that same matrix within connective tissue ensheathing muscle cells or in the lacunae of bone. It is similarly the space occupied by the plasma of the circulation and the lymph fluid of the lymphatic channels. Delivery to the interstitial space of muscle tissue is preferred. The polynucleotide construct may be conveniently delivered by injection into the tissues comprising these cells. They are preferably delivered to and expressed in persistent, non-dividing cells which are differentiated, although delivery and expression may be achieved in non-differentiated or less completely differentiated cells, such as, for example, stem cells of blood or skin fibroblasts. *In vivo* muscle cells are particularly competent in their ability to take up and express polynucleotides.

For the naked polynucleotide injection, an effective dosage amount of DNA or RNA will be in the range of from about 0.05 µg/kg body weight to about 50 mg/kg body weight. Preferably the dosage will be from about 0.005 mg/kg to about 20 mg/kg and more preferably from about 0.05 mg/kg to about 5 mg/kg. Of course, as the artisan of ordinary skill will appreciate, this dosage will vary according to the tissue site of injection. The appropriate and effective dosage of nucleic acid sequence can readily be determined by those of ordinary skill in the art and may depend on the condition being treated and the route of administration. The preferred route of administration is by the parenteral route of injection into the interstitial space of tissues. However, other parenteral routes may also be used, such as, inhalation of an aerosol formulation particularly for delivery to lungs or bronchial tissues, throat or mucous membranes of the nose. In addition, naked polynucleotide constructs can be delivered to arteries during angioplasty by the catheter used in the procedure.

The dose response effects of injected polynucleotide in muscle *in vivo* is determined as follows. Suitable template DNA for production of mRNA coding for polypeptide of the present invention is prepared in accordance with a standard recombinant DNA methodology. The template DNA, which may be either circular or linear, is either used as naked DNA or complexed with liposomes. The quadriceps muscles of mice are then injected with various amounts of the template DNA.

Five to six week old female and male Balb/C mice are anesthetized by intraperitoneal injection with 0.3 ml of 2.5% Avertin. A 1.5 cm incision is made on the anterior thigh, and the quadriceps muscle is directly visualized. The template DNA is injected in 0.1 ml of carrier in a 1 cc syringe through a 27 gauge needle over one minute, approximately 0.5 cm from the distal insertion site of the muscle into the knee and about 0.2 cm deep. A suture is placed over the injection site for future localization, and the skin is closed with stainless steel clips.

After an appropriate incubation time (e. g., 7 days) muscle extracts are prepared by excising the entire quadriceps. Every fifth 15 µm cross-section of the individual quadriceps muscles is histochemically stained for protein expression. A time course for protein expression may be done in a similar fashion except that quadriceps from different mice are harvested at different times. Persistence of DNA in muscle following injection may be determined by Southern blot analysis after preparing total cellular DNA and HIRT supernatants from injected and control mice.

The results of the above experimentation in mice can be use to extrapolate proper dosages and other treatment parameters in humans and other animals using naked DNA.

### Nonhuman Transgenic Animals

The CSG polypeptides of the invention can also be expressed in nonhuman transgenic animals. Nonhuman animals of any species, including, but not limited to, mice, rats, rabbits, hamsters, guinea pigs, pigs, micro-pigs, goats, sheep, cows and non-human primates, e. g., baboons, monkeys, and chimpanzees, may be used to generate transgenic animals. Any technique known in the art may be used to introduce the transgene (I. e., polynucleotides of the invention) into animals to produce the founder lines of transgenic animals. Such techniques include, but are not limited to, pronuclear microinjection (Paterson et al., Appl. Microbiol. Biotechnol. 40: 691-698 (1994); Carver et al., Biotechnology (NY) 11: 1263-1270 (1993); Wright et al., Biotechnology (NY) 9: 830-834 (1991); and Hoppe et al., U.S. Patent 4,873,191); retrovirus mediated gene transfer into germ lines (Van der Putten et al., Proc. Natl. Acad. Sci., USA 82: 6148-6152 (1985)), blastocysts or embryos; gene targeting in embryonic stem cells (Thompson et al., Cell 56: 313-321 (1989)); electroporation of cells or embryos (Lo, 1983, Mol. Cell. Biol. 3: 1803-1814 (1983)); introduction of the polynucleotides of the invention using a gene gun (see, e. g., Ulmer et al., Science 259: 1745 (1993); introducing nucleic acid constructs into embryonic pluripotent stem cells and transferring the stem cells back into the blastocyst; and sperm mediated gene transfer (Lavitrano et al., Cell 57: 717-723 (1989)). For a review of such techniques, see Gordon,"Transgenic Animals," Intl. Rev. Cytol. 115: 171-229 (1989), which is incorporated by reference herein in its entirety.

Any technique known in the art may be used to produce transgenic clones containing polynucleotides of the invention, for example, nuclear transfer into enucleated oocytes of nuclei from cultured embryonic, fetal, or adult cells induced to quiescence (Campell et al., Nature 380: 64-66 (1996); Wilmut et al., Nature 385: 810813 (1997)).

The present invention provides for transgenic animals that carry the transgene in all their cells, as well as animals which carry the transgene in some, but not all their cells, i.e., mosaic or chimeric animals. The transgene may be integrated as a single transgene or as multiple copies such as in concatamers, e. g., head-to-head tandems or head-to-tail tandems. The transgene may also be selectively introduced into and activated in a particular cell type by following, for example, the teaching of Lasko et al. (Lasko et al., Proc. Natl. Acad. Sci. USA 89: 6232-6236 (1992)). The regulatory sequences required for such a cell-type specific activation will depend upon the particular cell type of interest, and will be apparent to those of skill in the art. When it is desired that the polynucleotide transgene be integrated into the chromosomal site of the endogenous gene, gene targeting is preferred. Briefly, when such a technique is to be utilized, vectors containing some nucleotide sequences homologous to the endogenous gene are designed for the purpose of integrating, via homologous recombination with chromosomal sequences, into and disrupting the function of the nucleotide sequence of the endogenous gene. The transgene may also be selectively introduced into a particular cell type, thus inactivating the endogenous gene in only that cell type, by following, for example, the teaching of Gu et al. (Science 265: 103-106 (1994)). The regulatory sequences required for such a cell-type specific inactivation will depend upon the particular cell type of interest, and will be apparent to those of skill in the art.

Once transgenic animals have been generated, the expression of the recombinant gene may be assayed utilizing standard techniques. Initial screening may be accomplished by Southern blot analysis or PCR techniques to analyze animal tissues to verify that integration of the transgene has taken place. The level of mRNA expression of the transgene in the tissues of the transgenic animals may also be assessed using techniques which include, but are not limited to, Northern blot analysis of tissue samples obtained from the animal, in situ hybridization analysis, and reverse transcriptase-PCR (rt-PCR). Samples of transgenic gene-expressing tissue may also be evaluated immunocytochemically or immunohistochemically using antibodies specific for the transgene product.

Once the founder animals are produced, they may be bred, inbred, outbred, or crossbred to produce colonies of the particular animal. Examples of such breeding strategies include, but are not limited to: outbreeding of founder animals with more than one integration site in order to establish separate lines; inbreeding of separate lines in order to produce compound transgenics that express the transgene at higher levels because of the effects of additive expression of each transgene; crossing of heterozygous transgenic animals to produce animals homozygous for a given integration site in order to both augment expression and eliminate the need for screening of animals by DNA analysis; crossing of separate homozygous lines to produce compound heterozygous or homozygous lines; and breeding to place the transgene on a distinct background that is appropriate for an experimental model of interest.

Transgenic animals of the invention have uses which include, but are not limited to, animal model systems useful in elaborating the biological function of CSG polypeptides of the present invention, studying conditions and/or disorders associated with aberrant expression of CSGs, and in screening for compounds effective in ameliorating such CSG associated conditions and/or disorders.

### Knock-Out Animals

Endogenous gene expression can also be reduced by inactivating or"knocking out" the gene and/or its promoter using targeted homologous recombination (e. g., see Smithies et al., Nature 317: 230-234 (1985); Thomas & Capecchi, Cell 51: 503512 (1987); Thompson et al., Cell 5: 313-321 (1989); each of which is incorporated by reference herein in its entirety). For example, a mutant, non-functional CSG polynucleotide of the invention (or a completely unrelated DNA sequence) flanked by DNA homologous to the endogenous CSG polynucleotide sequence (either the coding regions or regulatory regions of the gene) can be used, with or without a selectable marker and/or a negative selectable marker, to transfect cells that express polypeptides of the invention in vivo. In another embodiment, techniques known in the art are used to generate knockouts in cells that contain, but do not express the gene of interest. Insertion of the DNA construct, via targeted homologous recombination, results in inactivation of the targeted gene. Such approaches are particularly suited in research and agricultural fields where modifications to embryonic stem cells can be used to generate animal offspring with an inactive targeted gene (e. g., see Thomas & Capecchi 1987 and Thompson 1989, supra). This approach can also be routinely adapted for use in humans provided the recombinant DNA constructs are directly administered or targeted to the required site in vivo using appropriate viral vectors that will be apparent to those of skill in the art.

In further embodiments of the invention, cells that are genetically engineered to express the CSG polypeptides of the invention, or alternatively, that are genetically engineered not to express the CSG polypeptides of the invention (e. g., knockouts) are administered to a patient in vivo. Such cells may be obtained from the patient or a MHC compatible donor and can include, but are not limited to, fibroblasts, bone marrow cells, blood cells (e. g., lymphocytes), adipocytes, muscle cells, and endothelial cells. The cells are genetically engineered *in vitro* using recombinant DNA techniques to introduce the coding sequence of polypeptides of the invention into the cells, or alternatively, to disrupt the coding sequence and/or endogenous regulatory sequence associated with the polypeptides of the invention, e. g., by transduction (using viral vectors, and preferably vectors that integrate the transgene into the cell genome) or transfection procedures, including, but not limited to, the use of plasmids, cosmids, YACs, naked DNA, electroporation, liposomes, etc.

The coding sequence of the CSG polypeptides of the invention can be placed under the control of a strong constitutive or inducible promoter or promoter/enhancer to achieve expression, and preferably secretion, of the CSG polypeptides of the invention. The engineered cells which express and preferably secrete the CSG polypeptides of the invention can be introduced into the patient systemically, e.g., in the circulation, or intraperitoneally.

Alternatively, the cells can be incorporated into a matrix and implanted in the body, e.g., genetically engineered fibroblasts can be implanted as part of a skin graft or genetically engineered endothelial cells can be implanted as part of a lymphatic or vascular graft (see, for example, U.S. Patent 5,399,349 and U.S. Patent 5,460,959 each of which is incorporated by reference herein in its entirety).

When the cells to be administered are non-autologous or non-MHC compatible cells, they can be administered using well known techniques which prevent the development of a host immune response against the introduced cells. For example, the cells may be introduced in an encapsulated form which, while allowing for an exchange of components with the immediate extracellular environment, does not allow the introduced cells to be recognized by the host immune system.

Transgenic and"knock-out" animals of the invention have uses which include, but are not limited to, animal model systems useful in elaborating the biological function of CSG polypeptides of the present invention, studying conditions and/or disorders associated with aberrant CSG expression, and in screening for compounds effective in ameliorating such CSG associated conditions and/or disorders.

### EXAMPLE

The present invention is further described by the following example. The example is provided solely to illustrate the invention by reference to specific embodiments. This exemplification, while illustrating certain aspects of the invention, does not portray the limitations or circumscribe the scope of the disclosed invention.

All examples outlined here were carried out using standard techniques, which are well known and routine to those of skill in the art, except where otherwise described in detail. Routine molecular biology techniques of the following example can be carried out as described in standard laboratory manuals, such as Sambrook et al., MOLECULAR CLONING: A LABORATORY MANUAL, 2nd Ed.; Cold Spring Harbor Laboratory Press, Cold Spring Harbor, N.Y. (1989).

### Identification of CSGs

Identification of CSGs (Colon Specific Gene) was carried out by a systematic analysis of data in the LIFESEQ Gold database available from Incyte Pharmaceuticals, Palo Alto, CA using the data mining Cancer Leads Automatic Search Package referred to herein as CLASP.

CLASP performs the following steps. First, highly expressed organ specific genes are selected based on the abundance level of the corresponding EST in the targeted organ versus all the other organs. Next, the expression level of each highly expressed organ specific gene is analyzed in normal tissue, tumor tissue, and tissue libraries associated with tumor or disease. Candidates are selected based upon demonstration of components of ESTs as well as expression exclusively or more frequently in tumor tissue or tumor libraries.

Thus, CLASP allows the identification of highly expressed organ and cancer specific genes. A final manual in depth evaluation is then performed to finalize the gene selection.

Using the CLASP method, the following Incyte sequences were identified as CSGs.

| **SEQ ID NO:** | **LSGold Gene ID** |
|---|---|
| 1 | 237623 |
| 2 | 234891 |
| 3 | 262167 |
| 4 | 246508 |
| 5 | 203279 |
| 6 | 983538 |
| 7 | 206344 |
| 8 | 222237 |
| 9 | 118593 |
| 10 | 337950 |
| 11 | 982786 |
| 12 | 398963 |
| 13 | 203640 |
| 14 | 88875 |
| 15 | 230552 |
| 16 | 407124 |
| 17 | 62662 |
| 18 | 230495 |
| 19 | 470880 |
| 20 | 898601 |
| 21 | 29586 |
| 22 | 370788 |

### Relative Quantitation of Gene Expression

Real-Time quantitative PCR with fluorescent Taqman probes is a quantitation detection system utilizing the 5'- 3' nuclease activity of Taq DNA polymerase. The method uses an internal fluorescent oligonucleotide probe (Taqman) labeled with a 5' reporter dye and a downstream, 3' quencher dye. During PCR, the 5'-3' nuclease activity of Taq DNA polymerase releases the reporter, whose fluorescence can then be detected by the laser detector of the Model 7700 Sequence Detection System (PE Applied Biosystems, Foster City, CA, USA).

Amplification of an endogenous control is used to standardize the amount of sample RNA added to the reaction and normalize for Reverse Transcriptase (RT) efficiency. Either cyclophilin, glyceraldehyde-3-phosphate dehydrogenase (GAPDH) or 18S ribosomal RNA (rRNA) was used as this endogenous control. To calculate relative quantitation between all the samples studied, the target RNA levels for one sample was used as the basis for comparative results (calibrator). Quantitation relative to the "calibrator" can be obtained using the standard curve method or the comparative method (User Bulletin #2: ABI PRISM 7700 Sequence Detection System).

The tissue distribution and the level of the target gene were determined for each sample of normal and cancer tissue. Total RNA was extracted from normal tissues, cancer tissues and from cancers and the corresponding matched adjacent tissues. Subsequently, first strand cDNA was prepared with reverse transcriptase and the polymerase chain reaction was done using primers and Taqman probe specific to each target gene. The results were analyzed using the ABI PRISM 7700 Sequence Detector. The absolute numbers are relative levels of expression of the target gene in a particular tissue compared to the calibrator tissue.

The following primers were used for real-time quantitative PCR:
forward primer: TGGAAATAGATTCAGGGGTCAT (SEQ ID NO:23)
reverse primer: CGGGTGTACCTCACTGACTTC (SEQ ID NO:24)
Q-PCR probe:

TGTCTTCCGAGAGAACCAGGCTCCG (SEQ ID NO:25)

The absolute numbers depicted in Table 1 are relative levels of expression of Gene ID 203279 (also referred to herein as Cln129 or SEQ ID NO:5) in 24 normal different tissues. All the values were compared to normal liver (calibrator). These RNA samples are commercially available pools, originated by pooling samples of a particular tissue from different individuals.

**Table 1: Relative Levels of CSG Cln129 Expression in Pooled Samples**

| **TISSUE** | **NORMAL** |
|---|---|
| Adrenal Gland | 0 |
| Bladder | 0 |
| Brain | 0 |
| Cervix | 0 |
| Colon | 0.7 |
| Endometrium | 0.4 |
| Esophagus | 0 |
| Heart | 0 |
| Kidney | 3.7 |
| Liver | 1 |
| Lung | 0 |
| Mammary Gland | 0.2 |
| Muscle | 0 |
| Ovary | 0 |
| Pancreas | 0 |
| Prostate | 0 |
| Rectum | 23 |
| Small Intestine | 1.5 |
| Spleen | 0 |
| Stomach | 0.8 |
| Testis | 0.1 |
| Thymus | 0.4 |
| Trachea | 0 |
| Uterus | 0 |

The relative levels of expression in Table 1 show that Cln129 mRNA expression is detected at high levels in the pool of normal rectum (23), and at a lower levels in kidney (3.7). In contrast, Cln129 is expressed at very low levels in the other 22 normal tissue pools analyzed. Further, the level of expression in rectum is 6 fold higher compared to the expression in kidney. These results demonstrate that Cln129 mRNA expression is highly specific for rectum tissue.

The absolute numbers in Table 1 were obtained analyzing pools of samples of a particular tissue from different individuals. They can not be compared to the absolute numbers originated from RNA obtained from tissue samples of a single individual in Table 2.

The absolute numbers depicted in Table 2 are relative levels of expression of Cln129 in 21 pairs of matching samples. All the values are compared to normal liver (calibrator). A matching pair is formed by mRNA from the cancer sample for a particular tissue and mRNA from the normal adjacent sample for that same tissue from the same individual.

**Table 2: Relative Levels of CSG Cln129 Expression in Individual Samples**

| **Sample ID** | **Tissue** | **CANCER** | **NORMAL** |
|---|---|---|---|
| ClnAS98 | Colon ascending (C)1 | 383 | 24 |
| ClnCM67 | Colon cecum (B)2 | 15 | 8 |
| ClnCXGA | Colon rectum (A)3 | 85 | 118 |
| ClnMT38 | Colon splenic flexture (D)4 | 33 | 18 |
| ClnRC24 | Colon rectum (D)5 | 77 | 29 |
| ClnRC67 | Colon rectum (B)6 | 0.9 | 15 |
| ClnRS45 | Colon rectosigmoid (C)7 | 161 | 25 |
| ClnSG27 | Colon sigmoid (C)8 | 48 | 13 |
| ClnSG33 | Colon sigmoid (C)9 | 190 | 100 |
| ClnSG36 | Colon sigmoid (B)10 | 186 | 93 |
| ClnRC89 | Colon rectum (D)11 | 0 | 28 |
| Bld32XK | Bladder 1 | 0 | 0 |
| CvxKS52 | Cervix 1 | 0 | 0 |
| Endo8XA | Endometrium 1 | 0 | 0.7 |
| Kid106XD | Kidney 1 | 0 | 6.7 |
| Liv15XA | Liver 1 | 1.7 | 3.2 |
| Lng47XQ | Lung 1 | 3.4 | 0 |
| Mam59X | Mammary Gland 1 | 1.3 | 0 |
| Pro34B | Prostate 1 | 0 | 0 |
| SmInt | Small Intestine 1 | 5.4 | 1.7 |
| Utr85XU | Uterus 1 | 0.9 | 0 |

| | | | |
|---|---|---|---|
| 0= Negative | | | |

Among 42 samples in Table 2 representing 11 different tissues significant expression is seen only in colon, kidney, and small intestine tissues. These results confirm the tissue specificity results obtained with normal samples shown in Table 1. Table 1 and Table 2 represent a combined total of 66 samples in 24 human tissue types. Only one small intestine sample, one lung sample, one liver sample, and one kidney sample showed expression of Cln129, out of a total of forty-two samples representing 22 different tissue types different than colon and rectum.

Comparisons of the level of mRNA expression in colon cancer samples and the normal adjacent tissue from the same individuals are shown in Table 2. Cln129 is expressed at higher levels in 8 of 11 (73%) cancer samples (colon 1, 2, 4, 5, 7, 8, 9, 10) compared to normal adjacent tissue.

Altogether, the high level of tissue specificity, plus the mRNA upregulation in 73% of the colon cancer matching samples tested indicate Cln129 to be a diagnostic marker for colon cancer.

It will be clear that the invention may be practiced otherwise than as particularly described in the foregoing description and examples. Numerous modifications and variations of the present invention are possible in light of the above teachings and, therefore, are within the scope of the appended claims.
The entire disclosure of each document cited (including patents, patent applications, journal articles, abstracts, laboratory manuals, books, or other disclosures) in the Background of the Invention, Detailed Description, and Examples is hereby incorporated herein by reference. Further, the hard copy of the sequence listing submitted herewith and the corresponding computer readable form are both incorporated herein by reference in their entireties.

## Claims

1. A colon specific gene (CSG) comprising:
(a) a polynucleotide of SEQ ID NO: 13 or a variant thereof;
(b) a protein expressed by a polynucleotide of SEQ ID NO: 13 or a variant thereof; or
(c) a polynucleotide which is capable of hybridizing under stringent conditions to the antisense sequence of SEQ ID NO: 13.

2. A diagnostic method indicative of the presence of colon cancer in a patient, the method comprising:
(a) determining levels of a CSG of claim 1 in cells, tissues or bodily fluids obtained from the patient; and
(b) comparing the determined levels of CSG with levels of CSG in cells, tissues or bodily fluids obtained from a normal human control, wherein a change in determined levels of CSG in cells, tissues or bodily fluids obtained from the patient versus normal human control is associated with the presence of colon cancer.

3. A diagnostic method indicative of the presence metastases of colon cancer in a patient having colon cancer that is not known to have metastasized, the method comprising:
(a) determining levels of a CSG of claim 1 in a sample of cells, tissues, or bodily fluid obtained from the patient; and
(b) comparing the determined CSG levels with levels of CSG in cells, tissues, or bodily fluid obtained a normal human control, wherein an increase in determined CSG levels in cells, tissues or bodily fluids obtained from the patient versus the normal human control is associated with a cancer which has metastasized.

4. A method of staging colon cancer in a patient having colon cancer, the method comprising:
(a) determining levels of a CSG of claim 1 in a sample of cells, tissue, or bodily fluids obtained from the patient; and
(b) comparing determined CSG levels with levels of CSG in cells, tissues, or bodily fluids obtained from a normal human control, wherein an increase in determined CSG levels in cells, tissues or bodily fluids obtained from the patient versus the normal human control is associated with a cancer which is progressing and a decrease in the determined CSG levels is associated with a cancer which is regressing or in remission.

5. A method of monitoring colon cancer in a patient having colon cancer that is not known to have metastasized for the onset of metastasis, the method comprising:
(a) periodically determining levels of a CSG of claim 1 in samples of cells, tissues, or bodily fluids obtained from the patient; and
(b) comparing the periodically determined CSG levels with levels of CSG in cells, tissues, or bodily fluids obtained from a normal human control, wherein an increase in any one of the periodically determined CSG levels in cells, tissues or bodily fluids obtained from the patient versus the normal human control is associated with a cancer which has metastasized.

6. A method of monitoring a change in stage of colon cancer in a patient having colon cancer, the method comprising:
(a) periodically determining levels of a CSG of claim 1 in cells, tissues, or bodily fluids obtained from the patient; and
(b) comparing the periodically determined CSG levels with levels of CSG in cells, tissues, or bodily fluid obtained from a normal human control, wherein an increase in any one of the periodically determined CSG levels in cells, tissues or bodily fluids obtained from the patient versus the normal human control is associated with a cancer which is progressing in stage and a decrease is associated with a cancer which is regressing in stage or in remission.

7. A method of identifying potential therapeutic agents for use in imaging and treating colon cancer, the method comprising screening compounds for an ability to bind to or decrease expression of a CSG of claim 1 relative to the CSG in the absence of the compound wherein the ability of the compound to bind to the CSG or decrease expression of the CSG is indicative of the compound being useful in imaging and treating colon cancer.

8. An antibody which specifically binds a polypeptide encoded by a CSG of claim 1.

9. A composition for imaging colon cancer in a patient, the composition comprising an antibody of claim 8.

10. A composition according to claim 9 wherein the antibody is labeled with paramagnetic ions or a radioisotope.

11. A composition for treating colon cancer in a patient, the composition comprising a compound which downregulates expression or activity of a CSG of claim 1.

12. A composition for inducing an immune response against a target cell expressing a CSG of claim 1, the composition comprising an immunogenically stimulatory amount of a CSG polypeptide so that an immune response is mounted against the target cell.

13. A composition according to claim 12 wherein the CSG polypeptide is encoded by a polynucleotide of SEQ ID 13.

14. A vaccine for treating colon cancer, the vaccine comprising a CSG of claim 1.
